# EUROPEAN PATENT SPECIFICATION

(11) **EP 4 274 572 B1**
(45) Date of publication and mention of the grant of the patent: **18.02.2026**
(21) Application number: 21917385.3
(22) Date of filing: 19.11.2021
(51) Int. Cl.: C07D 471/10, A61K 31/438, A61P 19/02, A61P 7/02, A61P 35/00, C07D 498/10, A61K 45/06

(54) **NOVEL INHIBITORS OF AUTOTAXIN**
NEUE AUTOTAXIN-HEMMER
NOUVEAUX INHIBITEURS DE L'AUTOTAXINE

(30) Priority: 05.01.2021 IN 202121000352
(43) Date of publication of application: 15.11.2023
(60) Divisional application: 25184407.2
(73) Proprietor: Zydus Lifesciences Limited, Ahmedabad 382481 (IN)
(72) Inventor: SHARMA, Rajiv, Gandhinagar Highway, Ahmedabad 382481 Gujarat (IN); BAHEKAR, Rajesh, Gandhinagar Highway, Ahmedabad 382481 Gujarat (IN); PRAJAPATI, Vijay, Gandhinagar Highway, Ahmedabad 382481 Gujarat (IN); JOSHI, Darshan, Gandhinagar Highway, Ahmedabad 382481 Gujarat (IN); DAVE, Kamal, Gandhinagar Highway, Ahmedabad 382481 Gujarat (IN)
(74) Representative: Gill Jennings & Every LLP
(86) International application number: PCT/IB2021/060738
(87) International publication number: WO 2022/149010

(56) References cited:
- WO-A1-2006/006490
- WO-A1-2015/154023
- WO-A1-2017/072722
- WO-A1-2017/152062
- US-A1- 2013 331 369
- US-A1- 2017 029 425

## Description

### FIELD OF THE INVENTION

The present invention describe novel compounds as Autotaxin (ATX) inhibitors for the treatment and prophylaxis of conditions or a disorder caused by Autotaxin (ATX) activation or increased concentration of lysophosphatidic acid (LPA). Invention also describes pharmaceutically acceptable salts, enantiomers, diastereomers, tautomeric forms and pharmaceutical composition of novel Autotaxin inhibitors.

### BACKGROUND OF THE INVENTION

ATX enzyme is important for converting Lysophosphatidylcholine (LPC) into LPA, as a bioactive signaling molecule. ATX is a secreted enzyme of the Ectonucleotide phosphatase family, also known as Ectonucleotide Pyrophosphatase/ Phosphodiesterase 2 (ENPP-2 or NPP2). ATX plays important role in driving pathological conditions, including fibrosis, arthritic inflammation, neurodegeneration, neuropathic pain and cancer. LPA is a bioactive lipid that affects migration, proliferation and survival of various cell types. LPA mediates variety of cellular and biological actions through LPA receptors (LPAR). The LPA shows broad tissue expression and it can couple to at least six distinct G proteins, known as LPAR1-6, which in turn, feed into multiple effector systems (Yung, Y.C. et al., J. Lipid Res. 2014, 55, 1192 and Kihara, Y. et al., Exp. Cell Res. 2015, 333, 171). Since the LPA level in plasma is highly related to the activity of ATX, it is believed that ATX is an important supply source of extracellular LPA.

Inhibition of ATX has been shown to reduce LPA levels in pathological settings. Reduction of LPA may provide therapeutic benefits in diseases with unmet need, including cancer, lymphocyte homing chronic inflammation, neuropathic pain, fibrotic diseases such as Idiopathic Pulmonary Fibrosis (IPF), thrombosis and cholestatic pruritus which caused and / or propagated by increased LPA levels and / or activation of ATX.

IPF is characterized as a progressive scarring of lung tissue which leads to worsening lung function and is ultimately fatal within 3-5 years from the onset of symptoms. There were no treatment options for IPF until 2014, when the FDA approved Nintedanib (Ofev) and Pirfenidone (Esbriet) (King, T, E. et al., Lancet 2011, 378, 1949; Richeldi, L. at el., N. Engl. J. Med. 2014, 370, 2071; Roth, G. J. et al., J. Med. Chem. 2009, 52, 4466; J. Med. Chem. 2015, 58, 1053; Hilberg, F. et al., Drugs Future 2010, 35, 5; and King, T. E. et al., N. Engl. J. Med. 2014, 370, 2083). Despite this advancement, there remains a need for additional medicines to treat IPF. Patients with IPF have elevated levels of LPA in their bronchoalveolar lavage fluid (BALF) and exhaled breath condensate. LPAR1 has been identified to be the predominant LPA receptor (Tager, A. M. et al., Nat. Med. 2008, 14, 45 and Montesi, S. B. et al., BMC Pulm. Med. 2014). In the lung fibroblasts of an IPF patient, LPAR1 was found to be responsible for enhanced fibroblast cell migration and vascular leak. It is therefore envisaged that LPAR1 antagonists will be a potential drug target for the treatment of IPF. In recent years, several LPAR1 antagonists have been reported, and some of these compounds are currently being evaluated for the treatment of IPF (Budd, D. C et al.., Future Med. Chem. 2013, 5, 1935; Qian, Y. et al., J. Med. Chem. 2012, 55, 7920 and Terakado, M. et al., ACS Med. Chem. Lett. 2016, 7, 913).

Fibrosis can develop in the liver, kidney, lung, dermis, vasculature, gut and other sites. Fibrosis develops due to action of pathways including growth factors, cytokines, integrin and lipids. ATX, LPA and LPAR pathways have been implicated in fibrotic disease. Increased levels of ATX, LPA and LPARs observed in various rodent models of fibrosis and in patient fluids and biopsy tissues. LPA can induce proliferative, survival and chemotactic responses in cells known to be critical in fibrotic disease, including: fibroblasts, smooth muscle cells, macrophages, epithelial and endothelial cells and leukocytes. Inhibitors of LPARs indicate that antagonism of receptors within this pathway blocked or reversed fibrosis in the lung, liver, kidney and skin in rodents. Accordingly in fibrotic diseases, it is desirable to lower LPA levels. This can be accomplished through inhibition of enzymes involved in LPA biosynthesis, such as ATX.

Various publications refer to compounds that are capable of inhibiting ATX, including: WO2019228403, WO2019108943, WO2019029620, WO2019223721, WO2019158107, WO201815312, WO2017152062, WO2017050791, WO2017050792, WO2017050747, WO2017050732, WO2015144605, WO2015042052, WO2015154023, WO2015175171, WO2014139882, WO2014139978, WO2014048865, WO2014202458, WO2013186159. Thus there is an unmet need for ATX inhibitors for use in the treatment and / or prophylaxis of physiological and / or pathophysiological conditions such as cancer, chronic inflammation, neuropathic pain, fibrotic diseases, thrombosis which are caused, medicated and / or propagated by increased LPA levels and / or the activation of ATX.

### SUMMARY OF THE INVENTION

The present invention describes novel compounds as autotaxin (ATX) inhibitors for treatment and prophylaxis of conditions or a disorder caused by ATX activation or increased concentration of LAP and also a pharmaceutical composition containing the same.

### EMBODIMENT(S) OF THE INVENTION

An embodiment of the present invention provides novel compounds represented by the compounds of Table 1 and of claim 1.

Also disclosed are pharmaceutical compositions containing novel compounds of the general formula (I), their tautomeric forms, their enantiomers, their diastereomers, their stereoisomers, their pharmaceutically acceptable salts, or their mixtures in combination with suitable carriers, solvents, diluents and other media normally employed in preparing such compositions.

Also disclosed is the use of these compounds as ATX inhibitors.

Further disclosed is a process for preparing the novel compounds of the present invention.

### DESCRIPTION OF THE INVENTION

Accordingly, the present disclosure relates to the compounds of the general formula **(I)** represents below and their pharmaceutically acceptable salts, enantiomers and their diastereomers; 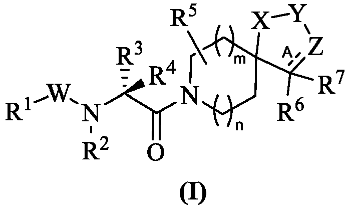 wherein,
R¹ is selected from (C₁-C₆)alkyl, (C₃-C₇)cycloalkyl, heterocyclyl, heteroaryl, aryl, arylalkyl, heterocycloalkyl, heteroarylalkyl, aryloxy, wherein each of these groups, wherever applicable, is further substituted with substituent(s) independently selected from -H, halo, -(C₁-C₆)alkyl, (C₁-C₆)alkoxy, (C₁-C₆)acyloxy, -(CH₂)ₚCF₃, -CN, -O(CH₂)ₚCF₃, -OCHF₂, -(CH₂)ₚOR^{a}, - (CR^{a}R^{b})ₚNR^{a}R^{b}, -COOR^{a}, -C(O)NR^{a}R^{b} , -NR^{c}C(O)NR^{a}R^{b} -S(O)₂NR^{a}R^{b}, - NR^{c}S(O)₂NR^{a}R^{b}, aryl, (C₃-C₇)cycloalkyl, or heterocyclyl;
W is selcected from -C(O)-, -C(O)-NR^{d} -, -S(O)₂-or -S(O)₂NR^{d}-;
R² is selected from H, (C₁-C₆)alkyl ;
R³ and R⁴ are each independendently selected from -H, (C₁-C₆)alkyl, (C₃-C₇)cycloalkyl, heterocyclyl, heteroaryl, aryl, arylalkyl, heterocycloalkyl, heteroarylalkyl, wherein each of these groups, wherever applicable, is further substituted with substituent(s) independently selected from -H, halo, -(C₁-C₆)alkyl, (C₁-C₆)alkoxy, (C₁-C₆)acyloxy, -(CH₂)_{q}CF₃, -CN, -O(CH₂)_{q}CF₃, - OCHF₂, -(CH₂)_{q}OR^{e}, -(CR^{e}R^{f})_{q}NR^{e}R^{f}, -COOR^{e}, -C(O)NR^{e}R^{f} , -NR^{g}C(O)NR^{e}R^{f} -S(O)₂NR^{e}R^{f}, -NR^{g}S(O)₂NR^{e}R^{f}, aryl, (C₃-C₇)cycloalkyl, or heterocyclyl;
R⁵ is H,;
X is independently selected from one or more of -(CR^{h}Rⁱ)ᵣ-, -(CR^{h}Rⁱ)ᵣ-C(O)-, - C(O)- or O ;
Y and Z are each independently selected from one or more of -NRⁱ, -(CR^{h}Rⁱ)ₛ-, - O-, or -C(O)-;
R⁶ and R⁷ are each independently selected from -H, (C₁-C₆)alkyl, (C₃-C₇)cycloalkyl, heterocyclyl, heteroaryl, aryl, arylalkyl, heterocycloalkyl, heteroarylalkyl, wherein each of these groups, wherever applicable, is further substituted with substituent(s) independently selected from -H, halo, -(C₁-C₆)alkyl, (C₁-C₆)alkoxy, (C₁-C₆)acyloxy, -(CH₂)ₜCF₃, -CN, -O(CH₂)ₜCF₃, - OCHF₂, -(CH₂)ₜOR^{j}, -(CR^{j}R^{k})ₜNR^{j}R^{k}, -COOR^{j}, -C(O)NR^{j}R^{k}, -NR^{l}C(O)NR^{j}R^{k} - S(O)₂NR^{j}R^{k}, -NR^{l}S(O)₂NR^{j}R^{k}, aryl, (C₃-C₇)cycloalkyl, or heterocyclyl;

The bond identified as 'A' is a single bond or double bond;
m and n are independently selected from 0, 1, or 2;
p, q, r, s and t are independently selected from 0, 1, 2 or 3;
R^{a}, R^{b}, R^{c}, R^{d}, R^{e}, R^{f}, R^{g}, R^{h}, Rⁱ, R^{j}, R^{k}, and R^{l} are independently selected from H, (C₁-C₆)alkyl, , (C₃-C₇)cycloalkyl, -(CR^{j}R^{k})ₜNR^{j}R^{k}, -(CR^{j}R^{k})ₜCOOR^{j}, - (CR^{j}R^{k})ₜ(C₃-C₇)cycloalkyl haloalkyl, aryl, heteroaryl or arylalkyl;

In a preferred embodiment R¹ is selected from heteroaryl and aryl further substituted with substituent(s) independently selected from -H, halo, (C₁-C₆)alkyl, (C₁-C₆)alkoxy, -(CH₂)ₚCF₃, -O(CH₂)ₚCF₃ wherein p is 0;
W is -C(O)-; R² is H; R³ is selected from -H, (C₁-C₆)alkyl, (C₃-C₇)cycloalkyl, and aryl; R⁴ and R⁶ and R⁷ are independently selected form -H, (C₁-C₆)alkyl, aryl and heteroaryl wherein aryl and heteroaryl may further substituted with substituent(s) independently selected from -H, , halo, -(C₁-C₆)alkyl, (C₁-C₆)alkoxy, -COOR^{j} wherein R^{j} is -H;
Rⁱ and R^{h} are independently selected from -H, (C₁-C₆) alkyl, -(CR^{j}R^{k})ₜ(C₃-C₇)cycloalkyl, -(CR^{j}R^{k})ₜCOOR^{j} wherein R^{j} and R^{k} are -H and -(CR^{j}R^{k})ₜCOOR^{j} and t is selected from 0, 1 and 2.

The radicals described above may be selected from:
"Alkyl", as well as other groups having the prefix "alk", such as alkoxy and alkanoyl, means carbon chain which may either be linear or branched, and combinations thereof, unless the carbon chain is defined otherwise. Examples of alkyl group include but not limited to methyl, ethyl, propyl, isopropyl, butyl, sec-butyl, tert.-butyl, pentyl, hexyl etc.

"Cycloalkyl" is the subset of alkyl and means saturated carbocyclic ring having a specified number of carbon atoms, preferably 3-6 carbon atoms. Examples of cycloalkyl include cyclopropyl, cyclobutyl, cyclopentyl, cyclohexyl, cycloheptyl etc. A cycloalkyl group generally is monocyclic unless otherwise stated. Cycloalkyl groups are saturated unless and otherwise stated.

"Aryl" means a mono- or polycyclic aromatic ring system containing carbon ring atoms. The preferred aryls are monocyclic or bicyclic 6-10 membered aromatic ring systems. Phenyl and naphthyl are preferred aryls.

Suitable groups and substituents on the groups may be selected from those described anywhere in the specification.

The term "substituted," as used herein, means that any one or more hydrogens on the designated atom is replaced with a selection from the indicated group, provided that the designated atom's normal valency is not exceeded, and that the substitution results in a stable compound. The term "substituted," as used herein, means that any one or more hydrogens on the designated atom is replaced with a selection from the indicated group, provided that the designated atom's normal valency is not exceeded, and that the substitution results in a stable compound.

"Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of the basic residues. Such conventional non-toxic salts include, but are not limited to, those derived from inorganic and organic acids selected from 1, 2-ethanedisulfonic, 2-acetoxybenzoic, 2-hydroxyethanesulfonic, acetic, ascorbic, benzenesulfonic, benzoic, bicarbonic, carbonic, citric, eidetic, ethane disulfonic, ethane sulfonic, fumaric, glucoheptonic, gluconic, glutamic, glycolic, glycollyarsanilic, hexylresorcinic, hydrabamic, hydrobromic, hydrochloric, hydroiodide, hydroxymaleic, hydroxynaphthoic, isethionic, lactic, lactobionic, lauryl sulfonic, maleic, malic, mandelic, methanesulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, and toluenesulfonic.

The term 'optional' or 'optionally' means that the subsequent described event or circumstance may or may not occur, and the description includes instances where the event or circumstance occur and instances in which it does not. For example, 'optionally substituted alkyl' means either 'alkyl' or 'substituted alkyl'. Further an optionally substituted group means unsubstituted.

Unless otherwise stated in the specification, structures depicted herein are also meant to include compounds which differ only in the presence of one or more isotopically enriched atoms.

In the following examples molecules with a single chiral center, unless otherwise noted, exist as a racemic mixture. Those molecules with two or more chiral centers, unless otherwise noted, exist as a racemic mixture of diastereomers. Single enantiomers/diastereomers may be obtained by methods known to those skilled in the art. The compounds of the invention are shown in Table 1 below:

**Table: 1**

| **Compd. No.** | **Structures** | **IUPAC Name** |
|---|---|---|
| **1** | 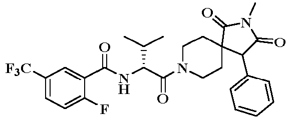 | 2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzamide |
| **2** | 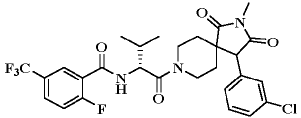 | N-((2R)-1-(4-(3-chlorophenyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **3** | 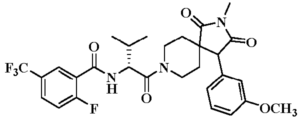 | 2-fluoro-N-((2R)-1-(4-(3-methoxyphenyl)-2-methyl-1,3-dioxo-2,8-diazaspiro[4.5]decan-8-yl)-3-methyl-1-oxobutan-2-yl)-5-(trifluoromethyl)benzamide |
| **4** | 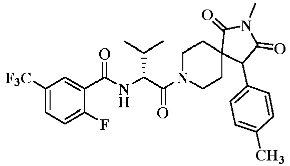 | 2-fluoro-N-((2R)-3-methyl-1-(2-methyl-1,3-dioxo-4-(p-tolyl)-2,8-diazaspiro[4.5]decan-8-yl)-1-oxobutan-2-yl)-5-(trifluoromethyl)benzamide |
| **5** | 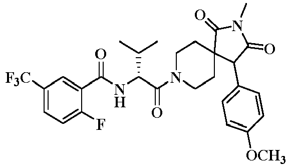 | 2-fluoro-N-((2R)-1-(4-(4-methoxyphenyl)-2-methyl-1,3-dioxo-2,8-diazaspiro[4.5]decan-8-yl)-3-methyl-1-oxobutan-2-yl)-5-(trifluoromethyl)benzamide |
| **6** | 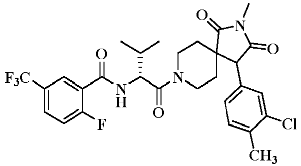 | N-((2R)-1-(4-(3-chloro-4-methylphenyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **7** | 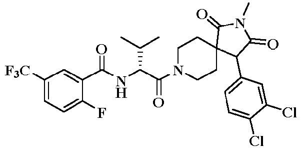 | N-((2R)-1-(4-(3,4-dichlorophenyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **8** | 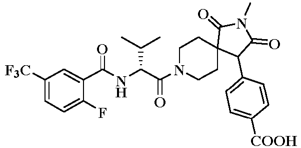 | 4-(8-((2-fluoro-5-(trifluoromethyl)benzoyl)-D-valyl)-2-methyl-1,3-dioxo-2,8-diazaspiro[4.5]decan-4-yl)benzoic acid |
| **9** | 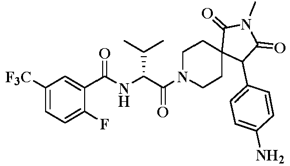 | N-((2R)-1-(4-(4-aminophenyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **10** | 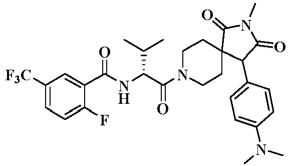 | N-((2R)-1-(4-(4-(dimethylamino)phenyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **11** | 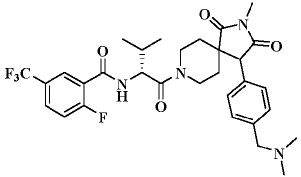 | N-((2R)-1-(4-(4-((dimethylamino)methyl)phenyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **12** | 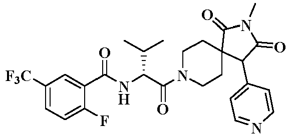 | 2-fluoro-N-((2R)-3-methyl-1-(2-methyl-1,3-dioxo-4-(pyridin-4-yl)-2,8-diazaspiro[4.5]decan-8-yl)-1-oxobutan-2-yl)-5-(trifluoromethyl)benzamide |
| **13** | 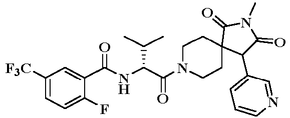 | 2-fluoro-N-((2R)-3-methyl-1-(2-methyl-1,3-dioxo-4-(pyridin-3-yl)-2,8-diazaspiro[4.5]decan-8-yl)-1-oxobutan-2-yl)-5-(trifluoromethyl)benzamide |
| **14** | 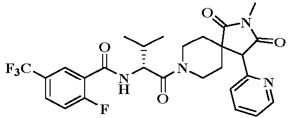 | 2-fluoro-N-((2R)-3-methyl-1-(2-methyl-1,3-dioxo-4-(pyridin-2-yl)-2,8-diazaspiro[4.5]decan-8-yl)-1-oxobutan-2-yl)-5-(trifluoromethyl)benzamide |
| **15** | 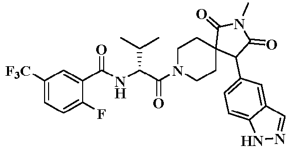 | N-((2R)-1-(4-(1H-indazol-5-yl)-2-methyl-1,3-dioxo-2,8-diazaspiro[4.5]decan-8-yl)-3-methyl-1-oxobutan-2-yl)-2-fluoro-5-(trifluoromethyl)benzamide |
| **16** | 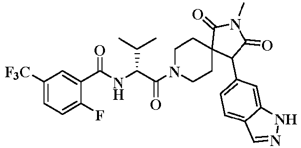 | N-((2R)-1-(4-(1H-indazol-6-yl)-2-methyl-1,3-dioxo-2,8-diazaspiro[4.5]decan-8-yl)-3-methyl-1-oxobutan-2-yl)-2-fluoro-5-(trifluoromethyl)benzamide |
| **17** | 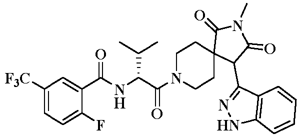 | N-((2R)-1-(4-(1H-indazol-3-yl)-2-methyl-1,3-dioxo-2,8-diazaspiro[4.5]decan-8-yl)-3-methyl-1-oxobutan-2-yl)-2-fluoro-5-(trifluoromethyl)benzamide |
| 18 | 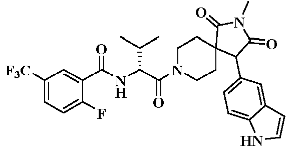 | N-((2R)-1-(4-(1H-indol-5-yl)-2-methyl-1,3-dioxo-2,8-diazaspiro[4.5]decan-8-yl)-3-methyl-1-oxobutan-2-yl)-2-fluoro-5-(trifluoromethyl)benzamide |
| 19 | 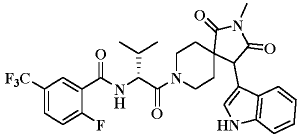 | N-((2R)-1-(4-(1H-indol-3-yl)-2-methyl-1,3-dioxo-2,8-diazaspiro[4.5]decan-8-yl)-3-methyl-1-oxobutan-2-yl)-2-fluoro-5-(trifluoromethyl)benzamide |
| 20 | 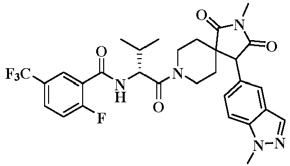 | 2-fluoro-N-((2R)-3-methyl-1-(2-methyl-4-(1-methyl-1H-indazol-5-yl)-1,3-dioxo-2,8-diazaspiro[4.5]decan-8-yl)-1-oxobutan-2-yl)-5-(trifluoromethyl)benzamide |
| 21 | 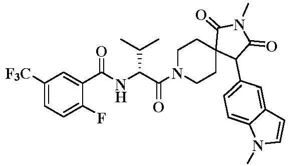 | 2-fluoro-N-((2R)-3-methyl-1-(2-methyl-4-(1-methyl-1H-indol-5-yl)-1,3-dioxo-2,8-diazaspiro[4.5]decan-8-yl)-1-oxobutan-2-yl)-5-(trifluoromethyl)benzamide |
| 22 | 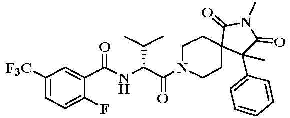 | N-((2R)-1-(2,4-dimethyl-1,3-dioxo-4-phenyl-2,8-diazaspiro[4.5]decan-8-yl)-3-methyl-1-oxobutan-2-yl)-2-fluoro-5-(trifluoromethyl)benzamide |
| 23 | 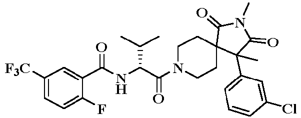 | N-((2R)-1-(4-(3-chlorophenyl)-2,4-dimethyl-1,3-dioxo-2,8-diazaspiro[4.5]decan-8-yl)-3-methyl-1-oxobutan-2-yl)-2-fluoro-5-(trifluoromethyl)benzamide |
| 24 | 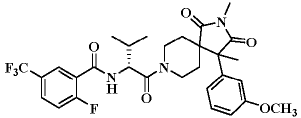 | 2-fluoro-N-((2R)-1-(4-(3-methoxyphenyl)-2,4-dimethyl-1,3-dioxo-2,8-diazaspiro[4.5]decan-8-yl)-3-methyl-1-oxobutan-2-yl)-5-(trifluoromethyl)benzamide |
| 25 | 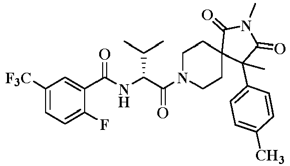 | N-((2R)-1-(2,4-dimethyl-1,3-dioxo-4-(p-tolyl)-2,8-diazaspiro[4.5]decan-8-yl)-3-methyl-1-oxobutan-2-yl)-2-fluoro-5-(trifluoromethyl)benzamide |
| 26 | 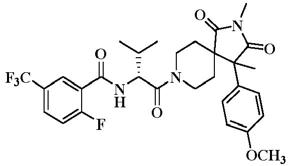 | 2-fluoro-N-((2R)-1-(4-(4-methoxyphenyl)-2,4-dimethyl-1,3-dioxo-2,8-diazaspiro[4.5]decan-8-yl)-3-methyl-1-oxobutan-2-yl)-5-(trifluoromethyl)benzamide |
| 27 | 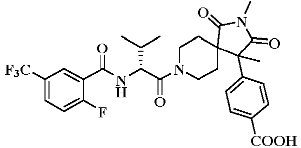 | 4-(8-((2-fluoro-5-(trifluoromethyl)benzoyl)-D-valyl)-2,4-dimethyl-1,3-dioxo-2,8-diazaspiro[4.5]decan-4-yl)benzoic acid |
| 28 | 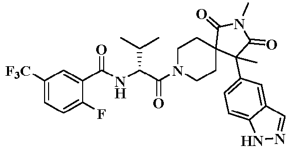 | N-((2R)-1-(4-(1H-indazol-5-yl)-2,4-dimethyl-1,3-dioxo-2,8-diazaspiro[4.5]decan-8-yl)-3-methyl-1-oxobutan-2-yl)-2-fluoro-5-(trifluoromethyl)benzamide |
| 29 | 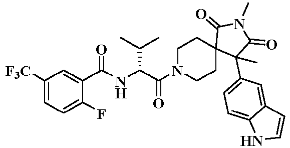 | N-((2R)-1-(4-(1H-indol-5-yl)-2,4-dimethyl-1,3-dioxo-2,8-diazaspiro[4.5]decan-8-yl)-3-methyl-1-oxobutan-2-yl)-2-fluoro-5-(trifluoromethyl)benzamide |
| 30 | 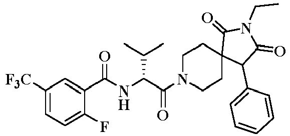 | N-((2R)-1-(2-ethyl-1,3-dioxo-4-phenyl-2,8-diazaspiro[4.5]decan-8-yl)-3-methyl-1-oxobutan-2-yl)-2-fluoro-5-(trifluoromethyl)benzamide |
| 31 | 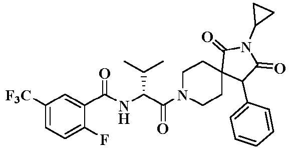 | N-((2R)-1-(2-cyclopropyl-1,3-dioxo-4-phenyl-2,8-diazaspiro[4.5]decan-8-yl)-3-methyl-1-oxobutan-2-yl)-2-fluoro-5-(trifluoromethyl)benzamide |
| 32 | 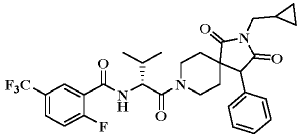 | N-((2R)-1-(2-(cyclopropylmethyl)-1,3-dioxo-4-phenyl-2,8-diazaspiro[4.5]decan-8-yl)-3-methyl-1-oxobutan-2-yl)-2-fluoro-5-(trifluoromethyl)benzamide |
| 33 | 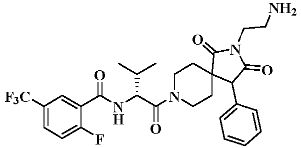 | N-((2R)-1-(2-(2-aminoethyl)-1,3-dioxo-4-phenyl-2,8-diazaspiro[4.5]decan-8-yl)-3-methyl-1-oxobutan-2-yl)-2-fluoro-5-(trifluoromethyl)benzamide |
| 34 | 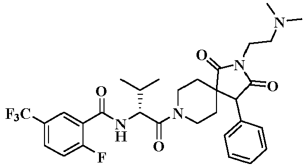 | N-((2R)-1-(2-(2-(dimethylamino)ethyl)-1,3-dioxo-4-phenyl-2,8-diazaspiro[4.5]decan-8-yl)-3-methyl-1-oxobutan-2-yl)-2-fluoro-5-(trifluoromethyl)benzamide |
| 35 | 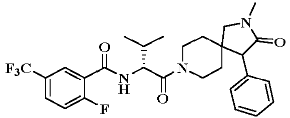 | 2-fluoro-N-((2R)-3-methyl-1-(2-methyl-3-oxo-4-phenyl-2,8-diazaspiro[4.5]decan-8-yl)-1-oxobutan-2-yl)-5-(trifluoromethyl)benzamide |
| 36 | 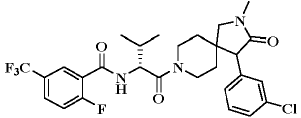 | N-((2R)-1-(4-(3-chlorophenyl)-2-methyl-3-oxo-2,8-diazaspiro[4.5]decan-8-yl)-3-methyl-1-oxobutan-2-yl)-2-fluoro-5-(trifluoromethyl)benzamide |
| 37 | 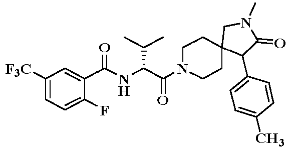 | 2-fluoro-N-((2R)-3-methyl-1-(2-methyl-3-oxo-4-(p-tolyl)-2,8-diazaspiro[4.5]decan-8-yl)-1-oxobutan-2-yl)-5-(trifluoromethyl)benzamide |
| 38 | 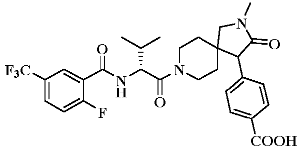 | 4-(8-((2-fluoro-5-(trifluoromethyl)benzoyl)-D-valyl)-2-methyl-3-oxo-2,8-diazaspiro[4.5]decan-4-yl)benzoic acid |
| 39 | 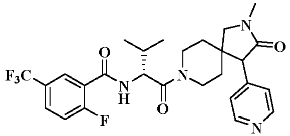 | 2-fluoro-N-((2R)-3-methyl-1-(2-methyl-3-oxo-4-(pyridin-4-yl)-2,8-diazaspiro[4.5]decan-8-yl)-1-oxobutan-2-yl)-5-(trifluoromethyl)benzamide |
| 40 | 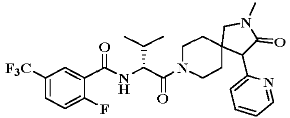 | 2-fluoro-N-((2R)-3-methyl-1-(2-methyl-3-oxo-4-(pyridin-2-yl)-2,8-diazaspiro[4.5]decan-8-yl)-1-oxobutan-2-yl)-5-(trifluoromethyl)benzamide |
| 41 | 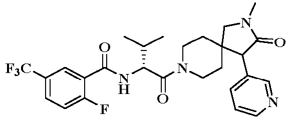 | 2-fluoro-N-((2R)-3-methyl-1-(2-methyl-3-oxo-4-(pyridin-3-yl)-2,8-diazaspiro[4.5]decan-8-yl)-1-oxobutan-2-yl)-5-(trifluoromethyl)benzamide |
| 42 | 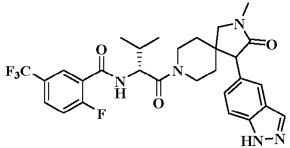 | N-((2R)-1-(4-(1H-indazol-5-yl)-2-methyl-3-oxo-2,8-diazaspiro[4.5]decan-8-yl)-3-methyl-1-oxobutan-2-yl)-2-fluoro-5-(trifluoromethyl)benzamide |
| 43 | 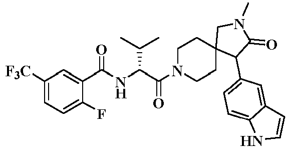 | N-((2R)-1-(4-(1H-indol-5-yl)-2-methyl-3-oxo-2,8-diazaspiro[4.5]decan-8-yl)-3-methyl-1-oxobutan-2-yl)-2-fluoro-5-(trifluoromethyl)benzamide |
| 44 | 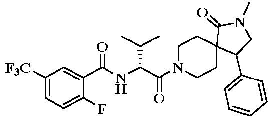 | 2-fluoro-N-((2R)-3-methyl-1-(2-methyl-1-oxo-4-phenyl-2,8-diazaspiro[4.5]decan-8-yl)-1-oxobutan-2-yl)-5-(trifluoromethyl)benzamide |
| 45 | 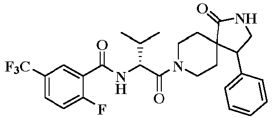 | 2-fluoro-N-((2R)-3-methyl-1-oxo-1-(1-oxo-4-phenyl-2,8-diazaspiro[4.5]decan-8-yl)butan-2-yl)-5-(trifluoromethyl)benzamide |
| 46 | 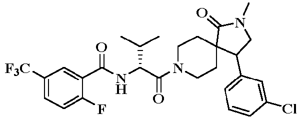 | N-((2R)-1-(4-(3-chlorophenyl)-2-methyl-1-oxo-2,8-diazaspiro[4.5]decan-8-yl)-3-methyl-1-oxobutan-2-yl)-2-fluoro-5-(trifluoromethyl)benzamide |
| 47 | 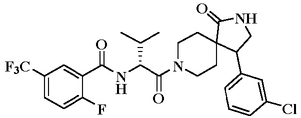 | N-((2R)-1-(4-(3-chlorophenyl)-1-oxo-2,8-diazaspiro[4.5]decan-8-yl)-3-methyl-1-oxobutan-2-yl)-2-fluoro-5-(trifluoromethyl)benzamide |
| 48 | 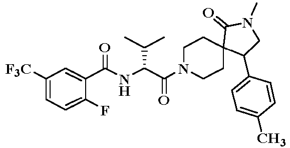 | 2-fluoro-N-((2R)-3-methyl-1-(2-methyl-1-oxo-4-(p-tolyl)-2,8-diazaspiro[4.5]decan-8-yl)-1-oxobutan-2-yl)-5-(trifluoromethyl)benzamide |
| 49 | 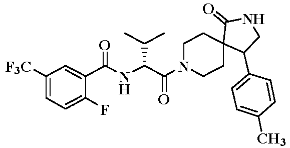 | 2-fluoro-N-((2R)-3-methyl-1-oxo-1-(1-oxo-4-(p-tolyl)-2,8-diazaspiro[4.5]decan-8-yl)butan-2-yl)-5-(trifluoromethyl)benzamide |
| 50 | 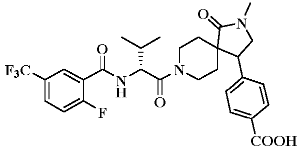 | 4-(8-((2-fluoro-5-(trifluoromethyl)benzoyl)-D-valyl)-2-methyl-1-oxo-2,8-diazaspiro[4.5]decan-4-yl)benzoic acid |
| 51 | 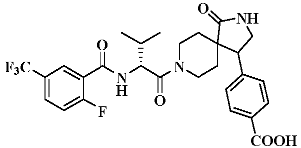 | 4-(8-((2-fluoro-5-(trifluoromethyl)benzoyl)-D-valyl)-1-oxo-2,8-diazaspiro[4.5]decan-4-yl)benzoic acid |
| 52 | 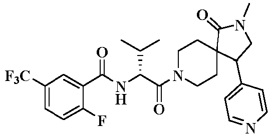 | 2-fluoro-N-((2R)-3-methyl-1-(2-methyl-1-oxo-4-(pyridin-4-yl)-2,8-diazaspiro[4.5]decan-8-yl)-1-oxobutan-2-yl)-5-(trifluoromethyl)benzamide |
| 53 | 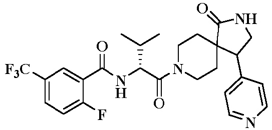 | 2-fluoro-N-((2R)-3-methyl-1-oxo-1-(1-oxo-4-(pyridin-4-yl)-2,8-diazaspiro[4.5]decan-8-yl)butan-2-yl)-5-(trifluoromethyl)benzamide |
| **54** | 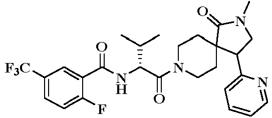 | 2-fluoro-N-((2R)-3-methyl-1-(2-methyl-1-oxo-4-(pyridin-2-yl)-2,8-diazaspiro[4.5]decan-8-yl)-1-oxobutan-2-yl)-5-(trifluoromethyl)benzamide |
| **55** | 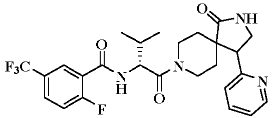 | 2-fluoro-N-((2R)-3-methyl-1-oxo-1-(1-oxo-4-(pyridin-2-yl)-2,8-diazaspiro[4.5]decan-8-yl)butan-2-yl)-5-(trifluoromethyl)benzamide |
| **56** | 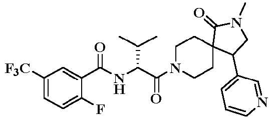 | 2-fluoro-N-((2R)-3-methyl-1-(2-methyl-1-oxo-4-(pyridin-3-yl)-2,8-diazaspiro[4.5]decan-8-yl)-1-oxobutan-2-yl)-5-(trifluoromethyl)benzamide |
| **57** | 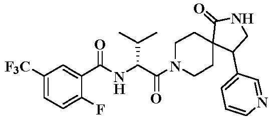 | 2-fluoro-N-((2R)-3-methyl-1-oxo-1-(1-oxo-4-(pyridin-3-yl)-2,8-diazaspiro[4.5]decan-8-yl)butan-2-yl)-5-(trifluoromethyl)benzamide |
| **58** | 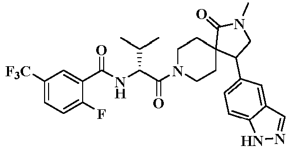 | N-((2R)-1-(4-(1H-indazol-5-yl)-2-methyl-1-oxo-2,8-diazaspiro[4.5]decan-8-yl)-3-methyl-1-oxobutan-2-yl)-2-fluoro-5-(trifluoromethyl)benzamide |
| **59** | 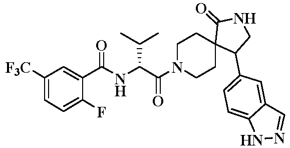 | N-((2R)-1-(4-(1H-indazol-5-yl)-1-oxo-2,8-diazaspiro[4.5]decan-8-yl)-3-methyl-1-oxobutan-2-yl)-2-fluoro-5-(trifluoromethyl)benzamide |
| **60** | 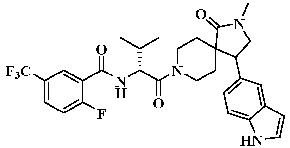 | N-((2R)-1-(4-(1H-indol-5-yl)-2-methyl-1-oxo-2,8-diazaspiro[4.5]decan-8-yl)-3-methyl-1-oxobutan-2-yl)-2-fluoro-5-(trifluoromethyl)benzamide |
| **61** | 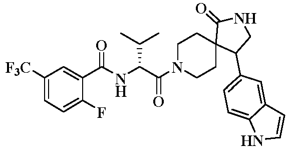 | N-((2R)-1-(4-(1H-indol-5-yl)-1-oxo-2,8-diazaspiro[4.5] decan-8-yl)-3-methyl-1-oxobutan-2-yl)-2-fluoro-5-(trifluoromethyl)benzamide |
| **62** | 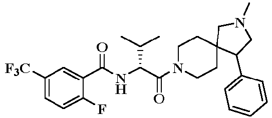 | 2-fluoro-N-((2R)-3-methyl-1-(2-methyl-4-phenyl-2,8-diazaspiro[4.5]decan-8-yl)-1-oxobutan-2-yl)-5-(trifluoromethyl)benzamide |
| **63** | 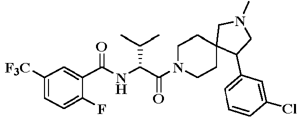 | N-((2R)-1-(4-(3-chlorophenyl)-2-methyl-2,8-diazaspiro[4.5]decan-8-yl)-3-methyl-1-oxobutan-2-yl)-2-fluoro-5-(trifluoromethyl)benzamide |
| **64** | 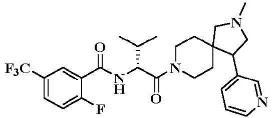 | 2-fluoro-N-((2R)-3-methyl-1-(2-methyl-4-(pyridin-3-yl)-2,8-diazaspiro[4.5]decan-8-yl)-1-oxobutan-2-yl)-5-(trifluoromethyl)benzamide |
| **65** | 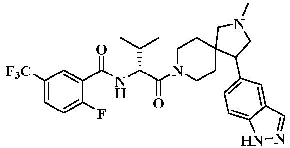 | N-((2R)-1-(4-(1H-indazol-5-yl)-2-methyl-2,8-diazaspiro[4.5]decan-8-yl)-3-methyl-1-oxobutan-2-yl)-2-fluoro-5-(trifluoromethyl)benzamide |
| **66** | 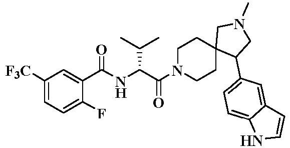 | N-((2R)-1-(4-(1H-indol-5-yl)-2-methyl-2,8-diazaspiro[4.5]decan-8-yl)-3-methyl-1-oxobutan-2-yl)-2-fluoro-5-(trifluoromethyl)benzamide |
| **67** | 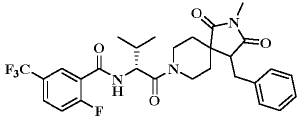 | N-((2R)-1-(4-benzyl-2-methyl-1,3-dioxo-2,8-diazaspiro[4.5]decan-8-yl)-3-methyl-1-oxobutan-2-yl)-2-fluoro-5-(trifluoromethyl)benzamide |
| **68** | 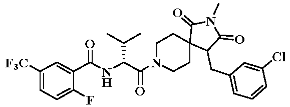 | N-((2R)-1-(4-(3-chlorobenzyl)-2-methyl-1,3-dioxo-2,8-diazaspiro[4.5]decan-8-yl)-3-methyl-1-oxobutan-2-yl)-2-fluoro-5-(trifluoromethyl)benzamide |
| **69** | 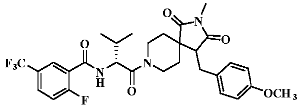 | 2-fluoro-N-((2R)-1-(4-(4-methoxybenzyl)-2-methyl-1,3-dioxo-2,8-diazaspiro[4.5]decan-8-yl)-3-methyl-1-oxobutan-2-yl)-5-(trifluoromethyl)benzamide |
| **70** | 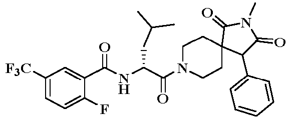 | 2-fluoro-N-((2R)-4-methyl-1-(2-methyl-1,3-dioxo-4-phenyl-2,8-diazaspiro[4.5]decan-8-yl)-1-oxopentan-2-yl)-5-(trifluoromethyl)benzamide |
| **71** | 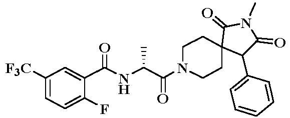 | 2-fluoro-N-((2R)-1-(2-methyl-1,3-dioxo-4-phenyl-2,8-diazaspiro[4.5]decan-8-yl)-1-oxopropan-2-yl)-5-(trifluoromethyl)benzamide |
| **72** | 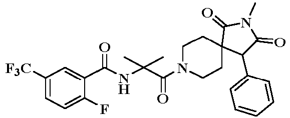 | 2-fluoro-N-(2-methyl-1-(2-methyl-1,3-dioxo-4-phenyl-2,8-diazaspiro[4.5]decan-8-yl)-1-oxopropan-2-yl)-5-(trifluoromethyl)benzamide |
| **73** | 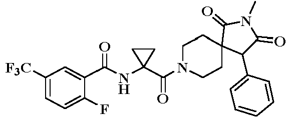 | 2-fluoro-N-(1-(2-methyl-1,3-dioxo-4-phenyl-2,8-diazaspiro[4.5]decane-8-carbonyl)cyclopropyl)-5-(trifluoromethyl)benzamide |
| **74** | 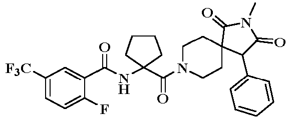 | 2-fluoro-N-(1-(2-methyl-1,3-dioxo-4-phenyl-2,8-diazaspiro [4.5]decane-8-carbonyl)cyclopentyl)-5-(trifluoromethyl)benzamide |
| **75** | 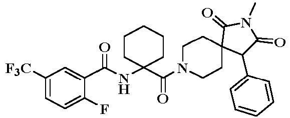 | 2-fluoro-N-(1-(2-methyl-1,3-dioxo-4-phenyl-2,8-diazaspiro [4.5]decane-8-carbonyl)cyclohexyl)-5-(trifluoromethyl)benzamide |
| **76** | 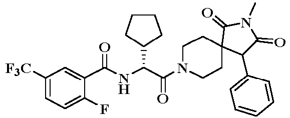 | N-((1R)-1-cyclopentyl-2-(2-methyl-1,3-dioxo-4-phenyl-2,8-diazaspiro[4.5]decan-8-yl)-2-oxoethyl)-2-fluoro-5-(trifluoromethyl)benzamide |
| **77** | 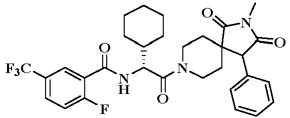 | N-((1R)-1-cyclohexyl-2-(2-methyl-1,3-dioxo-4-phenyl-2,8-diazaspiro[4.5]decan-8-yl)-2-oxoethyl)-2-fluoro-5-(trifluoromethyl)benzamide |
| **78** | 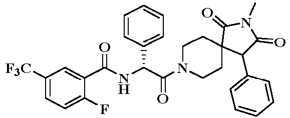 | 2-fluoro-N-((1R)-2-(2-methyl-1,3-dioxo-4-phenyl-2,8-diazaspiro[4.5]decan-8-yl)-2-oxo-1-phenylethyl)-5-(trifluoromethyl)benzamide |
| **79** | 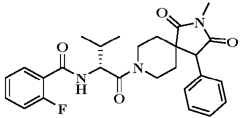 | 2-fluoro-N-((2R)-3-methyl-1-(2-methyl-1,3-dioxo-4-phenyl-2,8-diazaspiro[4.5]decan-8-yl)-1-oxobutan-2-yl)benzamide |
| **80** | 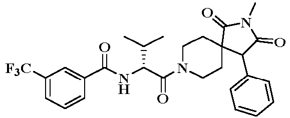 | N-((2R)-3-methyl-1-(2-methyl-1,3-dioxo-4-phenyl-2,8-diazaspiro[4.5]decan-8-yl)-1-oxobutan-2-yl)-3-(trifluoromethyl)benzamide |
| **81** | 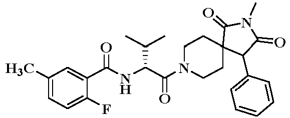 | 2-fluoro-5-methyl-N-((2R)-3-methyl-1-(2-methyl-1,3-dioxo-4-phenyl-2,8-diazaspiro[4.5]decan-8-yl)-1-oxobutan-2-yl)benzamide |
| **82** | 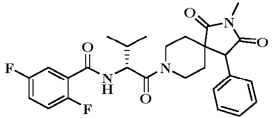 | 2,5-difluoro-N-((2R)-3-methyl-1-(2-methyl-1,3-dioxo-4-phenyl-2,8-diazaspiro[4.5]decan-8-yl)-1-oxobutan-2-yl)benzamide |
| **83** | 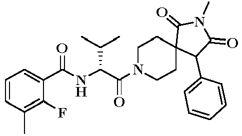 | 2-fluoro-3-methyl-N-((2R)-3-methyl-1-(2-methyl-1,3-dioxo-4-phenyl-2,8-diazaspiro[4.5]decan-8-yl)-1-oxobutan-2-yl)benzamide |
| **84** | 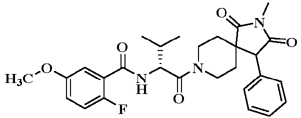 | 2-fluoro-5-methoxy-N-((2R)-3-methyl-1-(2-methyl-1,3-dioxo-4-phenyl-2,8-diazaspiro[4.5]decan-8-yl)-1-oxobutan-2-yl)benzamide |
| **85** | 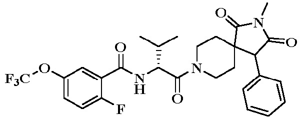 | 2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethoxy)benzamide |
| **86** | 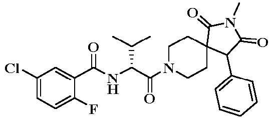 | 5-chloro-2-fluoro-N-((2R)-3-methyl-1-(2-methyl-1,3-dioxo-4-phenyl-2,8-diazaspiro[4.5]decan-8-yl)-1-oxobutan-2-yl)benzamide |
| **87** | 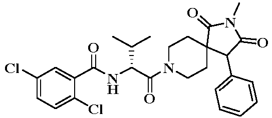 | 2,5-dichloro-N-((2R)-3-methyl-1-(2-methyl-1,3-dioxo-4-phenyl-2,8-diazaspiro[4.5]decan-8-yl)-1-oxobutan-2-yl)benzamide |
| **88** | 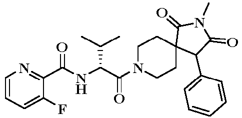 | 3-fluoro-N-((2R)-3-methyl-1-(2-methyl-1,3-dioxo-4-phenyl-2,8-diazaspiro[4.5]decan-8-yl)-1-oxobutan-2-yl)picolinamide |
| **89** | 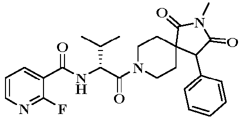 | 2-fluoro-N-((2R)-3-methyl-1-(2-methyl-1,3-dioxo-4-phenyl-2,8-diazaspiro[4.5]decan-8-yl)-1-oxobutan-2-yl)nicotinamide |
| **90** | 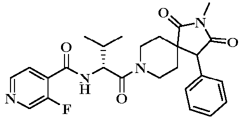 | 3-fluoro-N-((2R)-3-methyl-1-(2-methyl-1,3-dioxo-4-phenyl-2,8-diazaspiro[4.5]decan-8-yl)-1-oxobutan-2-yl)isonicotinamide |
| **91** | 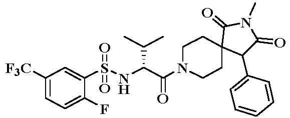 | 2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzenesulfonamide |
| **92** | 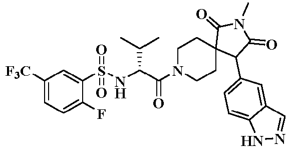 | N-((2R)-1-(4-(1H-indazol-5-yl)-2-methyl-1,3-dioxo-2,8-diazaspiro[4.5]decan-8-yl)-3-methyl-1-oxobutan-2-yl)-2-fluoro-5-(trifluoromethyl)benzenesulfonamide |
| **93** | 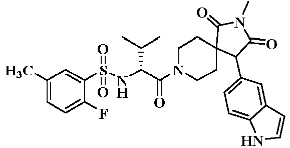 | N-((2R)-1-(4-(1H-indol-5-yl)-2-methyl-1,3-dioxo-2,8-diazaspiro[4.5]decan-8-yl)-3-methyl-1-oxobutan-2-yl)-2-fluoro-5-methylbenzenesulfonamide |
| **94** | 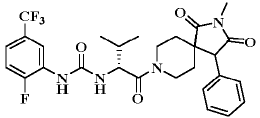 | 1-(2-fluoro-5-(trifluoromethyl)phenyl)-3-((2R)-3-methyl-1-(2-methyl-1,3-dioxo-4-phenyl-2,8-diazaspiro[4.5]decan-8-yl)-1-oxobutan-2-yl)urea |
| **95** | 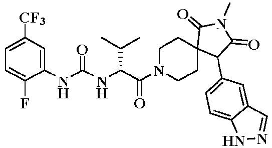 | 1-((2R)-1-(4-(1H-indazol-5-yl)-2-methyl-1,3-dioxo-2,8-diazaspiro[4.5]decan-8-yl)-3-methyl-1-oxobutan-2-yl)-3-(2-fluoro-5-(trifluoromethyl)phenyl)urea |
| **96** | 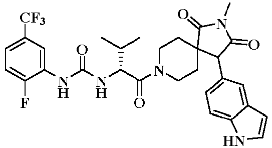 | 1-((2R)-1-(4-(1H-indol-5-yl)-2-methyl-1,3-dioxo-2,8-diazaspiro[4.5]decan-8-yl)-3-methyl-1-oxobutan-2-yl)-3-(2-fluoro-5-(trifluoromethyl)phenyl)urea |
| **97** | 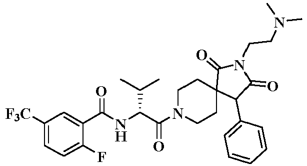 | N-((2R)-1-(2-(2-(dimethylamino)ethyl)-1,3-dioxo-4-phenyl-2,8-diazaspiro[4.5]decan-8-yl)-3-methyl-1-oxobutan-2-yl)-2-fluoro-5-(trifluoromethyl)benzamide |
| **98** | 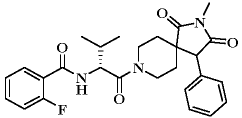 | 2-fluoro-N-((2R)-3-methyl-1-(2-methyl-1,3-dioxo-4-phenyl-2,8-diazaspiro[4.5]decan-8-yl)-1-oxobutan-2-yl)benzamide |
| **99** | 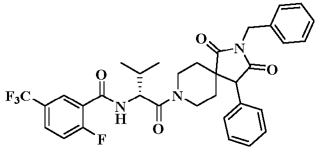 | N-((2R)-1-(2-benzyl-1,3-dioxo-4-phenyl-2,8-diazaspiro [4.5]decan-8-yl)-3-methyl-1-oxobutan-2-yl)-2-fluoro-5-(trifluoromethyl)benzamide |
| **100** | 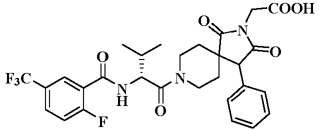 | 2-(8-((2-fluoro-5-(trifluoromethyl)benzoyl)-D-valyl)-1,3-dioxo-4-phenyl-2,8-diazaspiro[4.5]decan-2-yl)acetic acid |
| **101** | 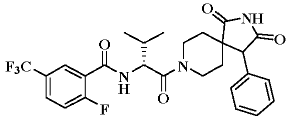 | N-((2R)-1-(1,3-dioxo-4-phenyl-2,8-diazaspiro[4.5]decan-8-yl)-3-methyl-1-oxobutan-2-yl)-2-fluoro-5-(trifluoromethyl)benzamide |
| **102** | 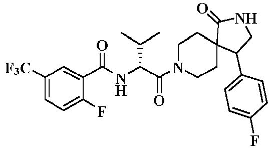 | 2-fluoro-N-((2R)-1-(4-(4-fluorophenyl)-1-oxo-2,8-diazaspiro[4.5]decan-8-yl)-3-methyl-1-oxobutan-2-yl)-5-(trifluoromethyl)benzamide |
| **103** | 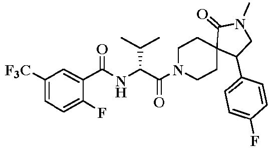 | 2-fluoro-N-((2R)-1-(4-(4-fluorophenyl)-2-methyl-1-oxo-2,8-diazaspiro[4.5]decan-8-yl)-3-methyl-1-oxobutan-2-yl)-5-(trifluoromethyl)benzamide |
| **104** | 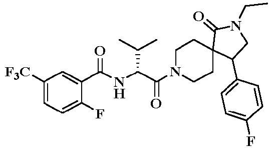 | N-((2R)-1-(2-ethyl-4-(4-fluorophenyl)-1-oxo-2,8-diazaspiro[4.5]decan-8-yl)-3-methyl-1-oxobutan-2-yl)-2-fluoro-5-(trifluoromethyl)benzamide |
| **105** | 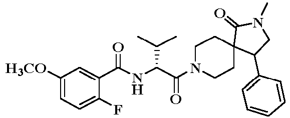 | 2-fluoro-5-methoxy-N-((2R)-3-methyl-1-(2-methyl-1-oxo-4-phenyl-2,8-diazaspiro[4.5]decan-8-yl)-1-oxobutan-2-yl)benzamide |
| **106** | 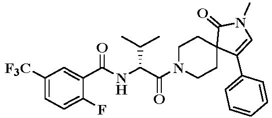 | (R)-2-fluoro-N-(3-methyl-1-(2-methyl-1-oxo-4-phenyl-2,8-diazaspiro[4.5]dec-3-en-8-yl)-1-oxobutan-2-yl)-5-(trifluoromethyl)benzamide |
| **107** | 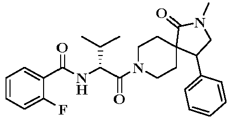 | 2-fluoro-N-((2R)-3-methyl-1-(2-methyl-1-oxo-4-phenyl-2,8-diazaspiro[4.5]decan-8-yl)-1-oxobutan-2-yl)benzamide |
| **108** | 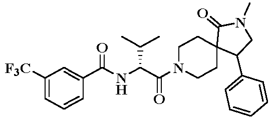 | N-((2R)-3-methyl-1-(2-methyl-1-oxo-4-phenyl-2,8-diazaspiro[4.5]decan-8-yl)-1-oxobutan-2-yl)-3-(trifluoromethyl)benzamide |
| **109** | 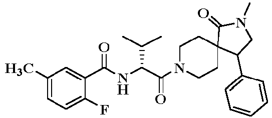 | 2-fluoro-5-methyl-N-((2R)-3-methyl-1-(2-methyl-1-oxo-4-phenyl-2,8-diazaspiro[4.5]decan-8-yl)-1-oxobutan-2-yl)benzamide |
| **110** | 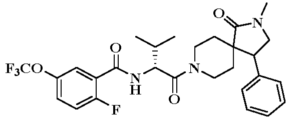 | 2-fluoro-N-((2R)-3-methyl-1-(2-methyl-1-oxo-4-phenyl-2,8-diazaspiro[4.5]decan-8-yl)-1-oxobutan-2-yl)-5-(trifluoromethoxy)benzamide |
| **111** | 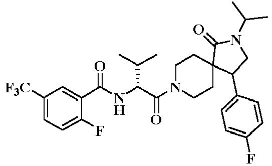 | 2-fluoro-N-((2R)-1-(4-(4-fluorophenyl)-2-isopropyl-1-oxo-2,8-diazaspiro[4.5]decan-8-yl)-3-methyl-1-oxobutan-2-yl)-5-(trifluoromethyl)benzamide |
| **112** | 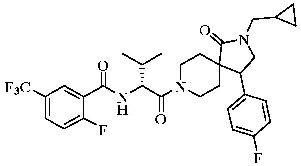 | N-((2R)-1-(2-(cyclopropylmethyl)-4-(4-fluorophenyl)-1-oxo-2,8-diazaspiro[4.5]decan-8-yl)-3-methyl-1-oxobutan-2-yl)-2-fluoro-5-(trifluoromethyl)benzamide |
| **113** | 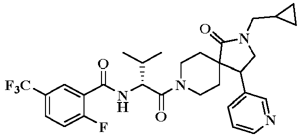 | N-((2R)-1-(2-(cyclopropylmethyl)-1-oxo-4-(pyridin-3-yl)-2,8-diazaspiro[4.5]decan-8-yl)-3-methyl-1-oxobutan-2-yl)-2-fluoro-5-(trifluoromethyl)benzamide |
| **114** | 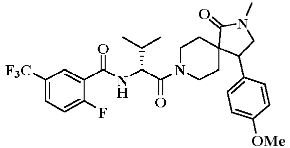 | 2-fluoro-N-((2R)-1-(4-(4-methoxyphenyl)-2-methyl-1-oxo-2,8-diazaspiro[4.5]decan-8-yl)-3-methyl-1-oxobutan-2-yl)-5-(trifluoromethyl)benzamide |
| **115** | 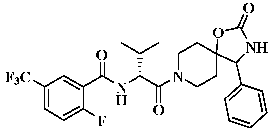 | 2-fluoro-N-((2R)-3-methyl-1-oxo-1-(2-oxo-4-phenyl-1-oxa-3,8-diazaspiro[4.5]decan-8-yl)butan-2-yl)-5-(trifluoromethyl)benzamide |
| **116** | 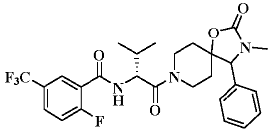 | 2-fluoro-N-((2R)-3-methyl-1-(3-methyl-2-oxo-4-phenyl-1-oxa-3,8-diazaspiro[4.5]decan-8-yl)-1-oxobutan-2-yl)-5-(trifluoromethyl)benzamide |
| **117** | 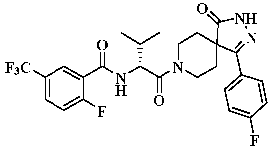 | (R)-2-fluoro-N-(1-(1-(4-fluorophenyl)-4-oxo-2,3,8-triazaspiro[4.5]dec-1-en-8-yl)-3-methyl-1-oxobutan-2-yl)-5-(trifluoromethyl)benzamide |
| **118** | 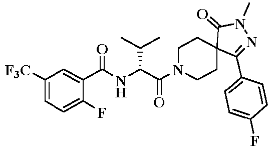 | (R)-2-fluoro-N-(1-(1-(4-fluorophenyl)-3-methyl-4-oxo-2,3,8-triazaspiro[4.5]dec-1-en-8-yl)-3-methyl-1-oxobutan-2-yl)-5-(trifluoromethyl)benzamide |
| **119** | 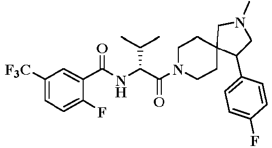 | 2-fluoro-N-((2R)-1-(4-(4-fluorophenyl)-2-methyl-2,8-diazaspiro[4.5]decan-8-yl)-3-methyl-1-oxobutan-2-yl)-5-(trifluoromethyl)benzamide |
| **120** | 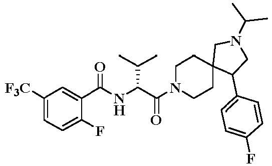 | 2-fluoro-N-((2R)-1-(4-(4-fluorophenyl)-2-isopropyl-2,8-diazaspiro[4.5]decan-8-yl)-3-methyl-1-oxobutan-2-yl)-5-(trifluoromethyl)benzamide |
| **121** | 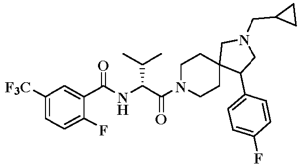 | N-((2R)-1-(2-(cyclopropylmethyl)-4-(4-fluorophenyl)-2,8-diazaspiro[4.5]decan-8-yl)-3-methyl-1-oxobutan-2-yl)-2-fluoro-5-(trifluoromethyl)benzamide |
| **122** | 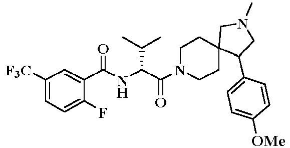 | 2-fluoro-N-((2R)-1-(4-(4-methoxyphenyl)-2-methyl-2,8-diazaspiro[4.5]decan-8-yl)-3-methyl-1-oxobutan-2-yl)-5-(trifluoromethyl)benzamide |
| **123** | 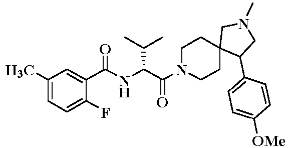 | 2-fluoro-N-((2R)-1-(4-(4-methoxyphenyl)-2-methyl-2,8-diazaspiro[4.5]decan-8-yl)-3-methyl-1-oxobutan-2-yl)-5-methylbenzamide |
| **124** | 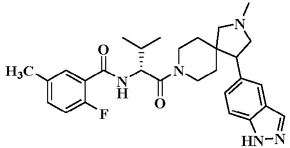 | N-((2R)-1-(4-(1H-indazol-5-yl)-2-methyl-2,8-diazaspiro[4.5]decan-8-yl)-3-methyl-1-oxobutan-2-yl)-2-fluoro-5-methylbenzamide |
| **125** | 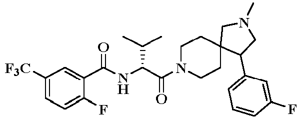 | 2-fluoro-N-((2R)-1-(4-(3-fluorophenyl)-2-methyl-2,8-diazaspiro[4.5]decan-8-yl)-3-methyl-1-oxobutan-2-yl)-5-(trifluoromethyl)benzamide |
| **126** | 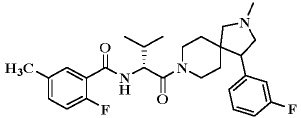 | 2-fluoro-N-((2R)-1-(4-(3-fluorophenyl)-2-methyl-2,8-diazaspiro[4.5]decan-8-yl)-3-methyl-1-oxobutan-2-yl)-5-methylbenzamide |
| **127** | 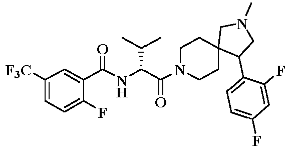 | N-((2R)-1-(4-(2,4-difluorophenyl)-2-methyl-2,8-diazaspiro-[4.5]decan-8-yl)-3-methyl-1-oxobutan-2-yl)-2-fluoro-5-(trifluoromethyl)benzamide |
| **128** | 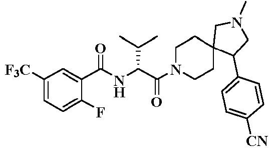 | N-((2R)-1-(4-(4-cyanophenyl)-2-methyl-2,8-diazaspiro-[4.5]decan-8-yl)-3-methyl-1-oxobutan-2-yl)-2-fluoro-5-(trifluoromethyl)benzamide |
| **129** | 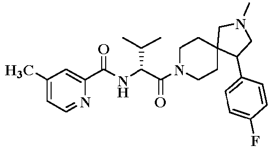 | N-((2R)-1-(4-(4-fluorophenyl)-2-methyl-2,8-diazaspiro-[4.5]decan-8-yl)-3-methyl-1-oxobutan-2-yl)-4-methylpicolinamide |
| **130** | 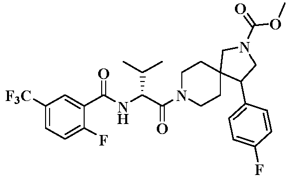 | methyl 8-((2-fluoro-5-(trifluoromethyl)benzoyl)-D-valyl)-4-(4-fluorophenyl)-2,8-diazaspiro[4.5]decane-2-carboxylate |
| **131** | 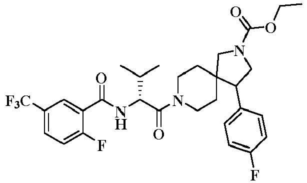 | ethyl 8-((2-fluoro-5-(trifluoromethyl)benzoyl)-D-valyl)-4-(4-fluorophenyl)-2,8-diazaspiro[4.5]decane-2-carboxylate |
| **132** | 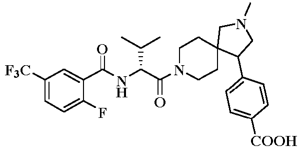 | 4-(8-((2-fluoro-5-(trifluoromethyl)benzoyl)-D-valyl)-2-methyl-2,8-diazaspiro[4.5]decan-4-yl)benzoic acid |
| **133** | 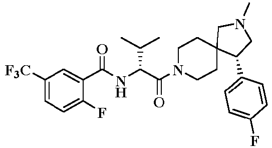 | 2-fluoro-N-((R)-1-((R)-4-(4-fluorophenyl)-2-methyl-2,8-diazaspiro[4.5]decan-8-yl)-3-methyl-1-oxobutan-2-yl)-5-(trifluoromethyl)benzamide |
| **134** | 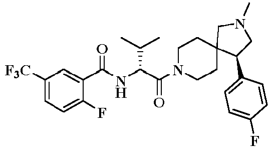 | 2-fluoro-N-((R)-1-((S)-4-(4-fluorophenyl)-2-methyl-2, 8-diazaspiro[4.5]decan-8-yl)-3-methyl-1-oxobutan-2-yl)-5-(trifluoromethyl)benzamide |
| **135** | 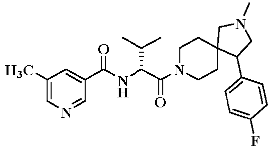 | N-((2R)-1-(4-(4-fluorophenyl)-2-methyl-2,8-diazaspiro-[4.5]decan-8-yl)-3-methyl-1-oxobutan-2-yl)-5-methylnicotinamide |
| **136** | 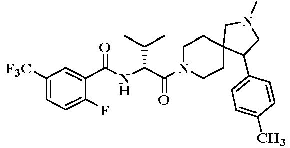 | 2-fluoro-N-((2R)-3-methyl-1-(2-methyl-4-(p-tolyl)-2,8-diazaspiro[4.5]decan-8-yl)-1-oxobutan-2-yl)-5-(trifluoromethyl)benzamide |
| **137** | 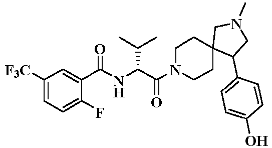 | 2-fluoro-N-((2R)-1-(4-(4-hydroxyphenyl)-2-methyl-2,8-diazaspiro[4.5]decan-8-yl)-3-methyl-1-oxobutan-2-yl)-5-(trifluoromethyl)benzamide |
| **138** | 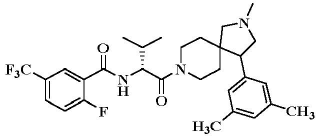 | N-((2R)-1-(4-(3,5-dimethylphenyl)-2-methyl-2,8-diazaspiro[4.5]decan-8-yl)-3-methyl-1-oxobutan-2-yl)-2-fluoro-5-(trifluoromethyl)benzamide |
| **139** | 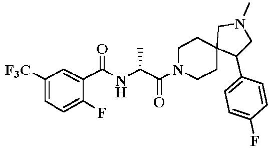 | 2-fluoro-N-((2R)-1-(4-(4-fluorophenyl)-2-methyl-2,8-diazaspiro[4.5]decan-8-yl)-1-oxopropan-2-yl)-5-(trifluoromethyl)benzamide |
| **140** | 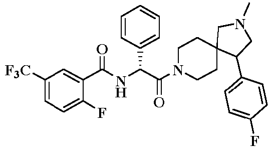 | 2-fluoro-N-((1R)-2-(4-(4-fluorophenyl)-2-methyl-2,8-diazaspiro[4.5]decan-8-yl)-2-oxo-1-phenylethyl)-5-(trifluoromethyl)benzamide |
| **141** | 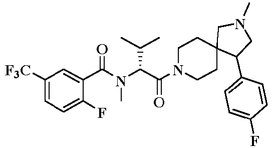 | 2-fluoro-N-((2R)-1-(4-(4-fluorophenyl)-2-methyl-2,8-diazaspiro[4.5]decan-8-yl)-3-methyl-1-oxobutan-2-yl)-N-methyl-5-(trifluoromethyl)benzamide |
| **142** | 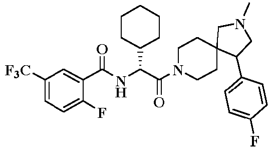 | N-((1R)-1-cyclohexyl-2-(4-(4-fluorophenyl)-2-methyl-2,8-diazaspiro[4.5]decan-8-yl)-2-oxoethyl)-2-fluoro-5-(trifluoromethyl)benzamide |
| **143** | 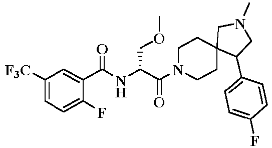 | 2-fluoro-N-((2R)-1-(4-(4-fluorophenyl)-2-methyl-2,8-diazaspiro[4.5]decan-8-yl)-3-methoxy-1-oxopropan-2-yl)-5-(trifluoromethyl)benzamide |
| **144** | 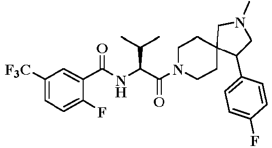 | 2-fluoro-N-((2S)-1-(4-(4-fluorophenyl)-2-methyl-2,8-diazaspiro[4.5]decan-8-yl)-3-methyl-1-oxobutan-2-yl)-5-(trifluoromethyl)benzamide |
| **145** | 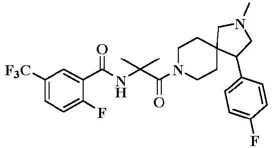 | 2-fluoro-N-(1-(4-(4-fluorophenyl)-2-methyl-2,8-diazaspiro[4.5]decan-8-yl)-2-methyl-1-oxopropan-2-yl)-5-(trifluoromethyl)benzamide |
| **146** | 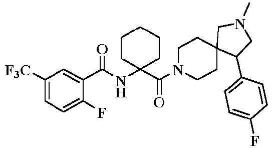 | 2-fluoro-N-(1-(4-(4-fluorophenyl)-2-methyl-2,8-diazaspiro [4.5]decane-8-carbonyl)cyclohexyl)-5-(trifluoromethyl)benzamide |
| **147** | 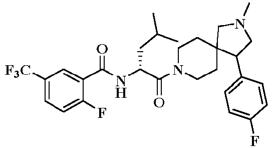 | 2-fluoro-N-((2R)-1-(4-(4-fluorophenyl)-2-methyl-2,8-diazaspiro[4.5]decan-8-yl)-4-methyl-1-oxopentan-2-yl)-5-(trifluoromethyl)benzamide |
| **148** | 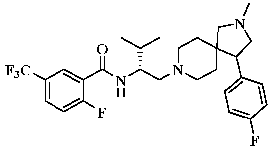 | 2-fluoro-N-((2R)-1-(4-(4-fluorophenyl)-2-methyl-2,8-diazaspiro[4.5]decan-8-yl)-3-methylbutan-2-yl)-5-(trifluoromethyl)benzamide |
| **149** | 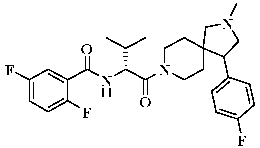 | 2,5-difluoro-N-((2R)-1-(4-(4-fluorophenyl)-2-methyl-2,8-diazaspiro[4.5]decan-8-yl)-3-methyl-1-oxobutan-2-yl)benzamide |
| **150** | 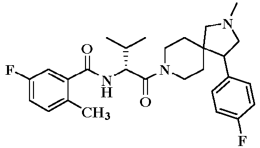 | 5-fluoro-N-((2R)-1-(4-(4-fluorophenyl)-2-methyl-2,8-diazaspiro[4.5]decan-8-yl)-3-methyl-1-oxobutan-2-yl)-2-methylbenzamide |
| **151** | 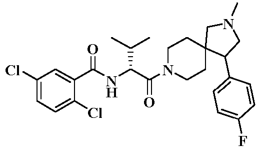 | 2,5-dichloro-N-((2R)-1-(4-(4-fluorophenyl)-2-methyl-2,8-diazaspiro[4.5]decan-8-yl)-3-methyl-1-oxobutan-2-yl)benzamide |
| **152** | 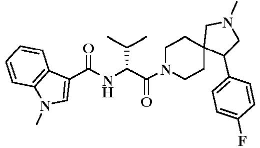 | N-((2R)-1-(4-(4-fluorophenyl)-2-methyl-2,8-diazaspiro[4.5]decan-8-yl)-3-methyl-1-oxobutan-2-yl)-1-methyl-1H-indole-3-carboxamide |
| **153** | 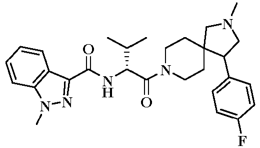 | N-((2R)-1-(4-(4-fluorophenyl)-2-methyl-2,8-diazaspiro[4.5]decan-8-yl)-3-methyl-1-oxobutan-2-yl)-1-methyl-1H-indazole-3-carboxamide |
| **154** | 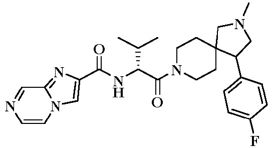 | N-((2R)-1-(4-(4-fluorophenyl)-2-methyl-2,8-diazaspiro[4.5]decan-8-yl)-3-methyl-1-oxobutan-2-yl)imidazo[1,2-a]pyrazine-2-carboxamide |
| **155** | 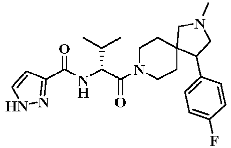 | N-((2R)-1-(4-(4-fluorophenyl)-2-methyl-2,8-diazaspiro[4.5]decan-8-yl)-3-methyl-1-oxobutan-2-yl)-1H-pyrazole-3-carboxamide |
| **156** | 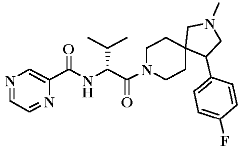 | N-((2R)-1-(4-(4-fluorophenyl)-2-methyl-2,8-diazaspiro[4.5]decan-8-yl)-3-methyl-1-oxobutan-2-yl)pyrazine-2-carboxamide |
| **157** | 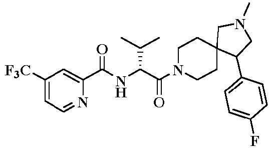 | N-((2R)-1-(4-(4-fluorophenyl)-2-methyl-2,8-diazaspiro[4.5]decan-8-yl)-3-methyl-1-oxobutan-2-yl)-4-(trifluoromethyl)picolinamide |
| **158** | 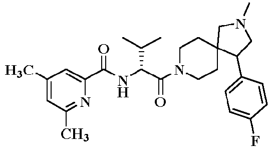 | N-((2R)-1-(4-(4-fluorophenyl)-2-methyl-2,8-diazaspiro[4.5]decan-8-yl)-3-methyl-1-oxobutan-2-yl)-4,6-dimethylpicolinamide |
| **159** | 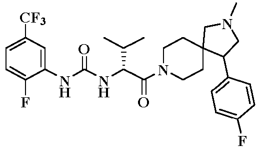 | 1-(2-fluoro-5-(trifluoromethyl)phenyl)-3-((2R)-1-(4-(4-fluorophenyl)-2-methyl-2,8-diazaspiro[4.5]decan-8-yl)-3-methyl-1-oxobutan-2-yl)urea |
| **160** | 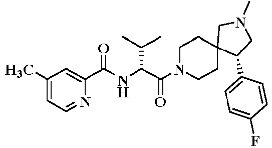 | N-((R)-1-((R)-4-(4-fluorophenyl)-2-methyl-2,8-diazaspiro[4.5]decan-8-yl)-3-methyl-1-oxobutan-2-yl)-4-methylpicolinamide |
| **161** | 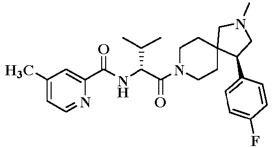 | N-((R)-1-((S)-4-(4-fluorophenyl)-2-methyl-2,8-diazaspiro[4.5]decan-8-yl)-3-methyl-1-oxobutan-2-yl)-4-methylpicolinamide |
| **162** | 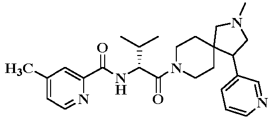 | 4-methyl-N-((2R)-3-methyl-1-(2-methyl-4-(pyridin-3-yl)-2,8-diazaspiro[4.5]decan-8-yl)-1-oxobutan-2-yl)picolinamide |
| **163** | 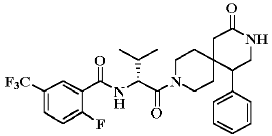 | 2-fluoro-N-((2R)-3-methyl-1-oxo-1-(10-oxo-7-phenyl-3,9-diazaspiro[5.5]undecan-3-yl)butan-2-yl)-5-(trifluoromethyl)benzamide |
| **164** | 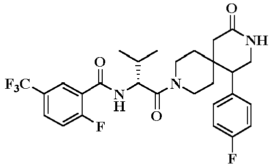 | 2-fluoro-N-((2R)-1-(7-(4-fluorophenyl)-10-oxo-3,9-diazaspiro[5.5]undecan-3-yl)-3-methyl-1-oxobutan-2-yl)-5-(trifluoromethyl)benzamide |
| **165** | 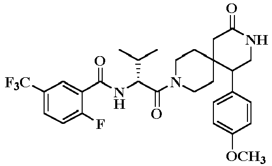 | 2-fluoro-N-((2R)-1-(7-(4-methoaηphenyl)-10-oxo-3,9-diazaspiro[5.5]undecan-3-yl)-3-methyl-1-oxobutan-2-yl)-5-(trifluoromethyl)benzamide |
| **166** | 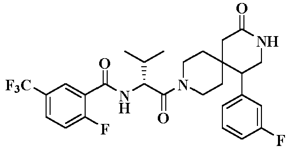 | 2-fluoro-N-((2R)-1-(7-(3-fluorophenyl)-10-oxo-3,9-diazaspiro[5.5]undecan-3-yl)-3-methyl-1-oxobutan-2-yl)-5-(trifluoromethyl)benzamide |
| **167** | 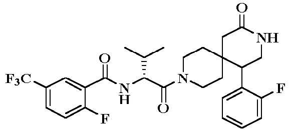 | 2-fluoro-N-((2R)-1-(7-(2-fluorophenyl)-10-oxo-3,9-diazaspiro[5.5]undecan-3-yl)-3-methyl-1-oxobutan-2-yl)-5-(trifluoromethyl)benzamide |
| **168** | 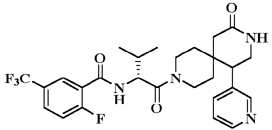 | 2-fluoro-N-((2R)-3-methyl-1-oxo-1-(10-oxo-7-(pyridin-3-yl)-3,9-diazaspiro[5.5]undecan-3-yl)butan-2-yl)-5-(trifluoromethyl)benzamide |
| **169** | 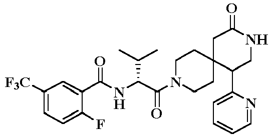 | 2-fluoro-N-((2R)-3-methyl-1-oxo-1-(10-oxo-7-(pyridin-2-yl)-3,9-diazaspiro[5.5]undecan-3-yl)butan-2-yl)-5-(trifluoromethyl)benzamide |
| **170** | 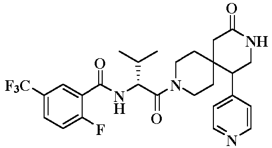 | 2-fluoro-N-((2R)-3-methyl-1-oxo-1-(10-oxo-7-(pyridin-4-yl)-3,9-diazaspiro[5.5]undecan-3-yl)butan-2-yl)-5-(trifluoromethyl)benzamide |
| **171** | 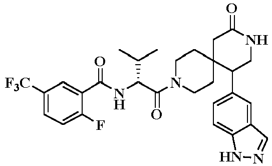 | N-((2R)-1-(7-(1H-indazol-5-yl)-10-oxo-3,9-diazaspiro[5.5]undecan-3-yl)-3-methyl-1-oxobutan-2-yl)-2-fluoro-5-(trifluoromethyl)benzamide |
| **172** | 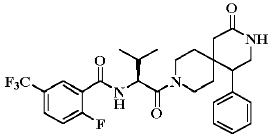 | 2-fluoro-N-((2S)-3-methyl-1-oxo-1-(10-oxo-7-phenyl-3,9-diazaspiro[5.5]undecan-3-yl)butan-2-yl)-5-(trifluoromethyl)benzamide |
| **173** | 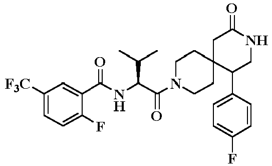 | 2-fluoro-N-((2S)-1-(7-(4-fluorophenyl)-10-oxo-3,9-diazaspiro[5.5]undecan-3-yl)-3-methyl-1-oxobutan-2-yl)-5-(trifluoromethyl)benzamide |
| **174** | 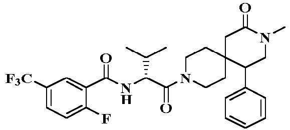 | 2-fluoro-N-((2R)-3-methyl-1-(9-methyl-10-oxo-7-phenyl-3,9-diazaspiro[5.5]undecan-3-yl)-1-oxobutan-2-yl)-5-(trifluoromethyl)benzamide |
| **175** | 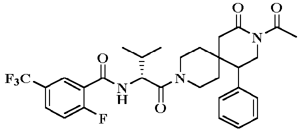 | N-((2R)-1-(9-acetyl-10-oxo-7-phenyl-3,9-diazaspiro[5.5]undecan-3-yl)-3-methyl-1-oxobutan-2-yl)-2-fluoro-5-(trifluoromethyl)benzamide |
| **176** | 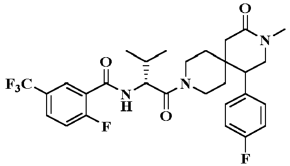 | 2-fluoro-N-((2R)-1-(7-(4-fluorophenyl)-9-methyl-10-oxo-3,9-diazaspiro[5.5]undecan-3-yl)-3-methyl-1-oxobutan-2-yl)-5-(trifluoromethyl)benzamide |
| **177** | 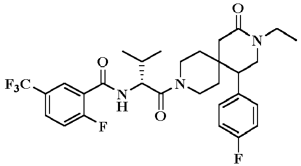 | N-((2R)-1-(9-ethyl-7-(4-fluorophenyl)-10-oxo-3,9-diazaspiro[5.5]undecan-3-yl)-3-methyl-1-oxobutan-2-yl)-2-fluoro-5-(trifluoromethyl)benzamide |
| **178** | 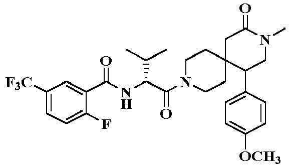 | 2-fluoro-N-((2R)-1-(7-(4-methoxyphenyl)-9-methyl-10-oxo-3,9-diazaspiro[5.5]undecan-3-yl)-3-methyl-1-oxobutan-2-yl)-5-(trifluoromethyl)benzamide |
| **179** | 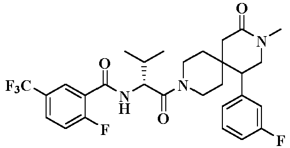 | 2-fluoro-N-((2R)-1-(7-(3-fluorophenyl)-9-methyl-10-oxo-3,9-diazaspiro[5.5]undecan-3-yl)-3-methyl-1-oxobutan-2-yl)-5-(trifluoromethyl)benzamide |
| **180** | 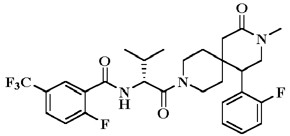 | 2-fluoro-N-((2R)-1-(7-(2-fluorophenyl)-9-methyl-10-oxo-3,9-diazaspiro[5.5]undecan-3-yl)-3-methyl-1-oxobutan-2-yl)-5-(trifluoromethyl)benzamide |
| **181** | 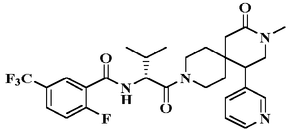 | 2-fluoro-N-((2R)-3-methyl-1-(9-methyl-10-oxo-7-(pyridin-3-yl)-3,9-diazaspiro[5.5]undecan-3-yl)-1-oxobutan-2-yl)-5-(trifluoromethyl)benzamide |
| **182** | 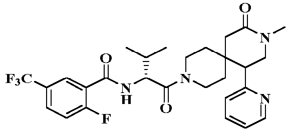 | 2-fluoro-N-((2R)-3-methyl-1-(9-methyl-10-oxo-7-(pyridin-2-yl)-3,9-diazaspiro[5.5]undecan-3-yl)-1-oxobutan-2-yl)-5-(trifluoromethyl)benzamide |
| **183** | 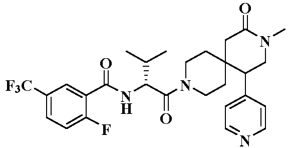 | 2-fluoro-N-((2R)-3-methyl-1-(9-methyl-10-oxo-7-(pyridin-4-yl)-3,9-diazaspiro[5.5]undecan-3-yl)-1-oxobutan-2-yl)-5-(trifluoromethyl)benzamide |
| **184** | 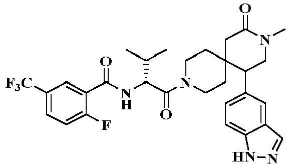 | N-((2R)-1-(7-(1H-indazol-5-yl)-9-methyl-10-oxo-3,9-diazaspiro[5.5]undecan-3-yl)-3-methyl-1-oxobutan-2-yl)-2-fluoro-5-(trifluoromethyl)benzamide |
| **185** | 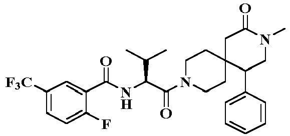 | 2-fluoro-N-((2S)-3-methyl-1-(9-methyl-10-oxo-7-phenyl-3,9-diazaspiro[5.5]undecan-3-yl)-1-oxobutan-2-yl)-5-(trifluoromethyl)benzamide |
| **186** | 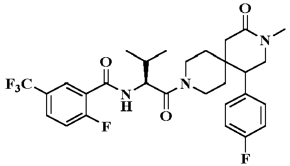 | 2-fluoro-N-((2S)-1-(7-(4-fluorophenyl)-9-methyl-10-oxo-3,9-diazaspiro[5.5]undecan-3-yl)-3-methyl-1-oxobutan-2-yl)-5-(trifluoromethyl)benzamide |
| **187** | 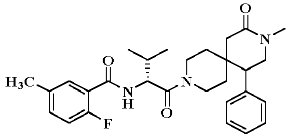 | 2-fluoro-5-methyl-N-((2R)-3-methyl-1-(9-methyl-10-oxo-7-phenyl-3,9-diazaspiro[5.5]undecan-3-yl)-1-oxobutan-2-yl)benzamide |
| **188** | 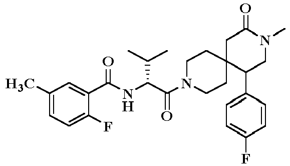 | 2-fluoro-N-((2R)-1-(7-(4-fluorophenyl)-9-methyl-10-oxo-3,9-diazaspiro[5.5]undecan-3-yl)-3-methyl-1-oxobutan-2-yl)-5-methylbenzamide |
| **189** | 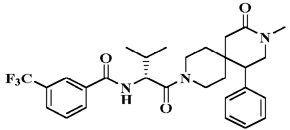 | N-((2R)-3-methyl-1-(9-methyl-10-oxo-7-phenyl-3,9-diazaspiro[5.5]undecan-3-yl)-1-oxobutan-2-yl)-3-(trifluoromethyl)benzamide |
| **190** | 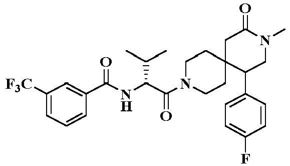 | N-((2R)-1-(7-(4-fluorophenyl)-9-methyl-10-oxo-3,9-diazaspiro[5.5]undecan-3-yl)-3-methyl-1-oxobutan-2-yl)-3-(trifluoromethyl)benzamide |
| **191** | 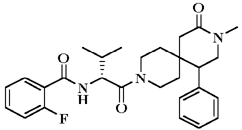 | 2-fluoro-N-((2R)-3-methyl-1-(9-methyl-10-oxo-7-phenyl-3,9-diazaspiro[5.5]undecan-3-yl)-1-oxobutan-2-yl)benzamide |
| **192** | 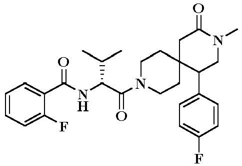 | 2-fluoro-N-((2R)-1-(7-(4-fluorophenyl)-9-methyl-10-oxo-3,9-diazaspiro[5.5]undecan-3-yl)-3-methyl-1-oxobutan-2-yl)benzamide |
| **193** | 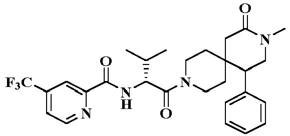 | N-((2R)-3-methyl-1-(9-methyl-10-oxo-7-phenyl-3,9-diazaspiro[5.5]undecan-3-yl)-1-oxobutan-2-yl)-4-(trifluoromethyl)picolinamide |
| **194** | 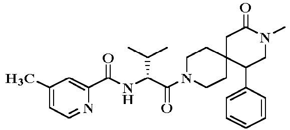 | N-((2R)-3-methyl-1-(9-methyl-10-oxo-7-phenyl-3,9-diazaspiro[5.5]undecan-3-yl)-1-oxobutan-2-yl)-4-(trifluoromethyl)picolinamide |
| **195** | 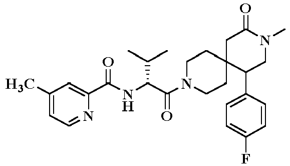 | N-((2R)-1-(7-(4-fluorophenyl)-9-methyl-10-oxo-3,9-diazaspiro[5.5]undecan-3-yl)-3-methyl-1-oxobutan-2-yl)-4-methylpicolinamide |
| **196** | 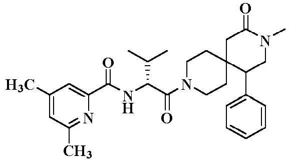 | 4,6-dimethyl-N-((2R)-3-methyl-1-(9-methyl-10-oxo-7-phenyl-3,9-diazaspiro[5.5]undecan-3-yl)-1-oxobutan-2-yl)picolinamide |
| **197** | 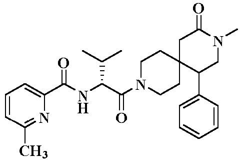 | 6-methyl-N-((2R)-3-methyl-1-(9-methyl-10-oxo-7-phenyl-3,9-diazaspiro[5.5]undecan-3-yl)-1-oxobutan-2-yl)picolinamide |
| **198** | 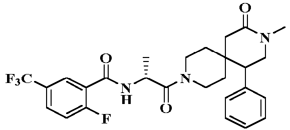 | 2-fluoro-N-((2R)-1-(9-methyl-10-oxo-7-phenyl-3,9-diazaspiro[5.5]undecan-3-yl)-1-oxopropan-2-yl)-5-(trifluoromethyl)benzamide |
| **199** | 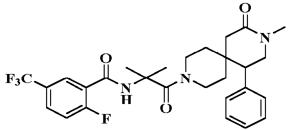 | 2-fluoro-N-(2-methyl-1-(9-methyl-10-oxo-7-phenyl-3,9-diazaspiro[5.5]undecan-3-yl)-1-oxopropan-2-yl)-5-(trifluoromethyl)benzamide |
| **200** | 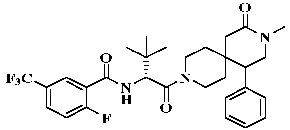 | N-((2R)-3,3-dimethyl-1-(9-methyl-10-oxo-7-phenyl-3,9-diazaspiro [5.5]undecan-3-yl)-1-oxobutan-2-yl)-2-fluoro-5-(trifluoromethyl)benzamide |
| **201** | 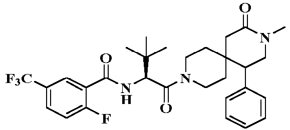 | N-((2S)-3,3-dimethyl-1-(9-methyl-10-oxo-7-phenyl-3,9-diazaspiro [5.5]undecan-3-yl)-1-oxobutan-2-yl)-2-fluoro-5-(trifluoromethyl)benzamide |
| **202** | 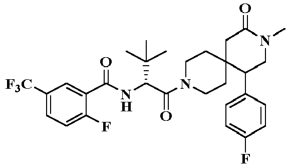 | 2-fluoro-N-((2R)-1-(7-(4-fluorophenyl)-9-methyl-10-oxo-3,9-diazaspiro[5.5]undecan-3-yl)-3,3-dimethyl-1-oxobutan-2-yl)-5-(trifluoromethyl)benzamide |
| **203** | 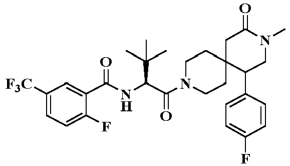 | 2-fluoro-N-((2S)-1-(7-(4-fluorophenyl)-9-methyl-10-oxo-3,9-diazaspiro[5.5]undecan-3-yl)-3,3-dimethyl-1-oxobutan-2-yl)-5-(trifluoromethyl)benzamide |
| **204** | 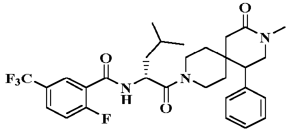 | 2-fluoro-N-((2R)-4-methyl-1-(9-methyl-10-oxo-7-phenyl-3,9-diazaspiro[5.5]undecan-3-yl)-1-oxopentan-2-yl)-5-(trifluoromethyl)benzamide |
| **205** | 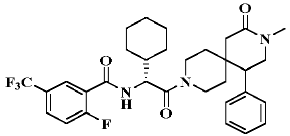 | N-((1R)-1-cyclohexyl-2-(9-methyl-10-oxo-7-phenyl-3,9-diazaspiro[5.5]undecan-3-yl)-2-oxoethyl)-2-fluoro-5-(trifluoromethyl)benzamide |
| **206** | 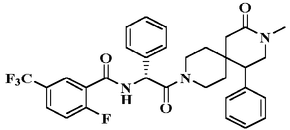 | 2-fluoro-N-((1R)-2-(9-methyl-10-oxo-7-phenyl-3,9-diazaspiro[5.5]undecan-3-yl)-2-oxo-1-phenylethyl)-5-(trifluoromethyl)benzamide |
| **207** | 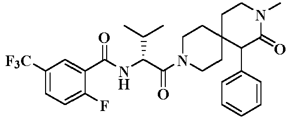 | 2-fluoro-N-((2R)-3-methyl-1-(9-methyl-8-oxo-7-phenyl-3,9-diazaspiro[5.5]undecan-3-yl)-1-oxobutan-2-yl)-5-(trifluoromethyl)benzamide |
| **208** | 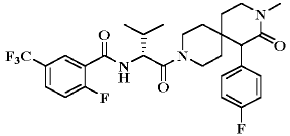 | 2-fluoro-N-((2R)-1-(7-(4-fluorophenyl)-9-methyl-8-oxo-3,9-diazaspiro[5.5]undecan-3-yl)-3-methyl-1-oxobutan-2-yl)-5-(trifluoromethyl)benzamide |
| **209** | 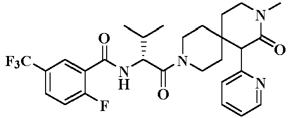 | 2-fluoro-N-((2R)-3-methyl-1-(9-methyl-8-oxo-7-(pyridin-2-yl)-3,9-diazaspiro[5.5]undecan-3-yl)-1-oxobutan-2-yl)-5-(trifluoromethyl)benzamide |
| **210** | 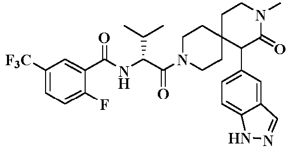 | N-((2R)-1-(7-(1H-indazol-5-yl)-9-methyl-8-oxo-3,9-diazaspiro[5.5]undecan-3-yl)-3-methyl-1-oxobutan-2-yl)-2-fluoro-5-(trifluoromethyl)benzamide |
| **211** | 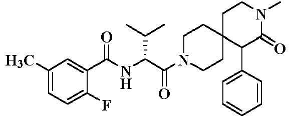 | 2-fluoro-5-methyl-N-((2R)-3-methyl-1-(9-methyl-8-oxo-7-phenyl-3,9-diazaspiro[5.5]undecan-3-yl)-1-oxobutan-2-yl)benzamide |
| **212** | 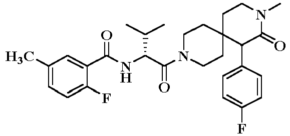 | 2-fluoro-N-((2R)-1-(7-(4-fluorophenyl)-9-methyl-8-oxo-3,9-diazaspiro[5.5]undecan-3-yl)-3-methyl-1-oxobutan-2-yl)-5-methylbenzamide |
| **213** | 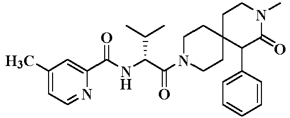 | 4-methyl-N-((2R)-3-methyl-1-(9-methyl-8-oxo-7-phenyl-3,9-diazaspiro[5.5]undecan-3-yl)-1-oxobutan-2-yl)picolinamide |
| **214** | 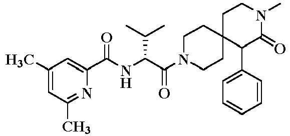 | 4,6-dimethyl-N-((2R)-3-methyl-1-(9-methyl-8-oxo-7-phenyl-3,9-diazaspiro[5.5]undecan-3-yl)-1-oxobutan-2-yl)picolinamide |
| **215** | 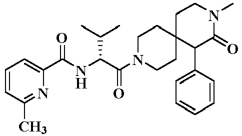 | 6-methyl-N-((2R)-3-methyl-1-(9-methyl-8-oxo-7-phenyl-3,9-diazaspiro[5.5]undecan-3-yl)-1-oxobutan-2-yl)picolinamide |
| **216** | 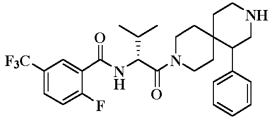 | 2-fluoro-N-((2R)-3-methyl-1-oxo-1-(7-phenyl-3,9-diazaspiro[5.5]undecan-3-yl)butan-2-yl)-5-(trifluoromethyl)benzamide |
| **217** | 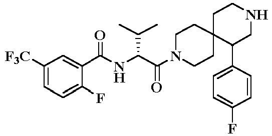 | 2-fluoro-N-((2R)-1-(7-(4-fluorophenyl)-3,9-diazaspiro[5.5]undecan-3-yl)-3-methyl-1-oxobutan-2-yl)-5-(trifluoromethyl)benzamide |
| **218** | 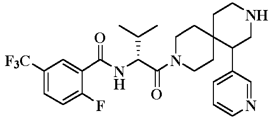 | 2-fluoro-N-((2R)-3-methyl-1-oxo-1-(7-(pyridin-3-yl)-3,9-diazaspiro[5.5]undecan-3-yl)butan-2-yl)-5-(trifluoromethyl)benzamide |
| **219** | 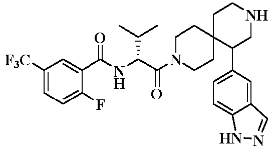 | N-((2R)-1-(7-(1H-indazol-5-yl)-3,9-diazaspiro[5.5]undecan-3-yl)-3-methyl-1-oxobutan-2-yl)-2-fluoro-5-(trifluoromethyl)benzamide |
| **220** | 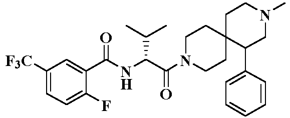 | 2-fluoro-N-((2R)-3-methyl-1-(9-methyl-7-phenyl-3,9-diazaspiro[5.5]undecan-3-yl)-1-oxobutan-2-yl)-5-(trifluoromethyl)benzamide |
| **221** | 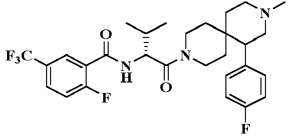 | 2-fluoro-N-((2R)-1-(7-(4-fluorophenyl)-9-methyl-3,9-diazaspiro [5.5]undecan-3-yl)-3-methyl-1-oxobutan-2-yl)-5-(trifluoromethyl)benzamide |
| **222** | 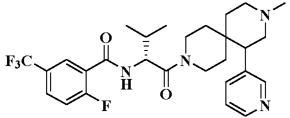 | 2-fluoro-N-((2R)-3-methyl-1-(9-methyl-7-(pyridin-3-yl)-3,9-diazaspiro[5.5]undecan-3-yl)-1-oxobutan-2-yl)-5-(trifluoromethyl)benzamide |
| **223** | 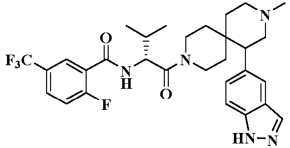 | N-((2R)-1-(7-(1H-indazol-5-yl)-9-methyl-3,9-diazaspiro[5.5]undecan-3-yl)-3-methyl-1-oxobutan-2-yl)-2-fluoro-5-(trifluoromethyl)benzamide |
| **224** | 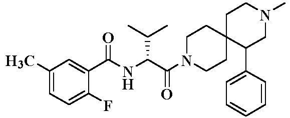 | 2-fluoro-5-methyl-N-((2R)-3-methyl-1-(9-methyl-7-phenyl-3,9-diazaspiro[5.5]undecan-3-yl)-1-oxobutan-2-yl)benzamide |
| **225** | 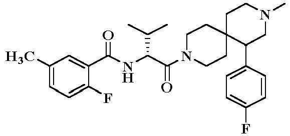 | 2-fluoro-N-((2R)-1-(7-(4-fluorophenyl)-9-methyl-3,9-diazaspiro[5.5]undecan-3-yl)-3-methyl-1-oxobutan-2-yl)-5-methylbenzamide |
| **226** | 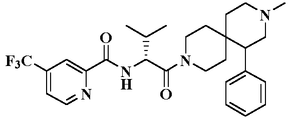 | N-((2R)-3-methyl-1-(9-methyl-7-phenyl-3,9-diazaspiro[5.5]undecan-3-yl)-1-oxobutan-2-yl)-4-(trifluoromethyl)picolinamide |
| **227** | 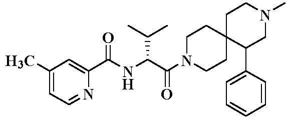 | 4-methyl-N-((2R)-3-methyl-1-(9-methyl-7-phenyl-3,9-diazaspiro[5.5]undecan-3-yl)-1-oxobutan-2-yl)picolinamide |
| **228** | 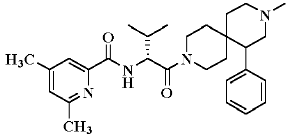 | 4,6-dimethyl-N-((2R)-3-methyl-1-(9-methyl-7-phenyl-3,9-diazaspiro[5.5]undecan-3-yl)-1-oxobutan-2-yl)picolinamide |
| **229** | 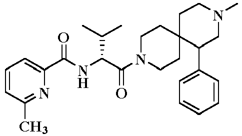 | 6-methyl-N-((2R)-3-methyl-1-(9-methyl-7-phenyl-3,9-diazaspiro[5.5]undecan-3-yl)-1-oxobutan-2-yl)picolinamide |
| **230** | 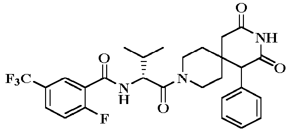 | N-((2R)-1-(8,10-dioxo-7-phenyl-3,9-diazaspiro[5.5]-undecan-3-yl)-3-methyl-1-oxobutan-2-yl)-2-fluoro-5-(trifluoromethyl)benzamide |
| **231** | 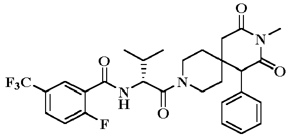 | 2-fluoro-N-((2R)-3-methyl-1-(9-methyl-8,10-dioxo-7-phenyl-3,9-diazaspiro[5.5]undecan-3-yl)-1-oxobutan-2-yl)-5-(trifluoromethyl)benzamide |
| **232** | 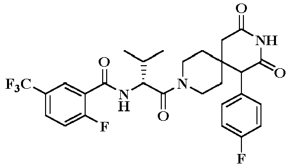 | 2-fluoro-N-((2R)-1-(7-(4-fluorophenyl)-8,10-dioxo-3,9-diazaspiro[5.5]undecan-3-yl)-3-methyl-1-oxobutan-2-yl)-5-(trifluoromethyl)benzamide |
| **233** | 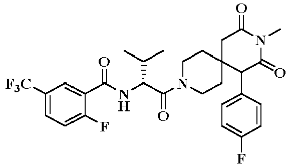 | 2-fluoro-N-((2R)-1-(7-(4-fluorophenyl)-9-methyl-8,10-dioxo-3,9-diazaspiro[5.5]undecan-3-yl)-3-methyl-1-oxobutan-2-yl)-5-(trifluoromethyl)benzamide |
| **234** | 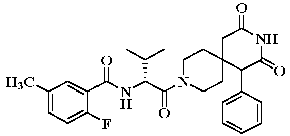 | N-((2R)-1-(8,10-dioxo-7-phenyl-3,9-diazaspiro[5.5]-undecan-3-yl)-3-methyl-1-oxobutan-2-yl)-2-fluoro-5-methylbenzamide |
| **235** | 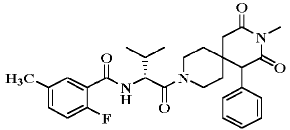 | 2-fluoro-5-methyl-N-((2R)-3-methyl-1-(9-methyl-8,10-dioxo-7-phenyl-3,9-diazaspiro[5.5]undecan-3-yl)-1-oxobutan-2-yl)benzamide |
| **236** | 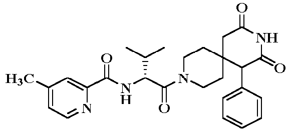 | N-((2R)-1-(8,10-dioxo-7-phenyl-3,9-diazaspiro[5.5]-undecan-3-yl)-3-methyl-1-oxobutan-2-yl)-4-methylpicolinamide |
| **237** | 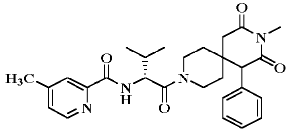 | 4-methyl-N-((2R)-3-methyl-1-(9-methyl-8,10-dioxo-7-phenyl-3,9-diazaspiro[5.5]undecan-3-yl)-1-oxobutan-2-yl)picolinamide |
| **238** | 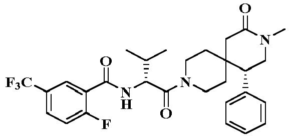 | 2-fluoro-N-((R)-3-methyl-1-((S)-9-methyl-10-oxo-7-phenyl-3,9-diazaspiro[5.5]undecan-3-yl)-1-oxobutan-2-yl)-5-(trifluoromethyl)benzamide |
| **239** | 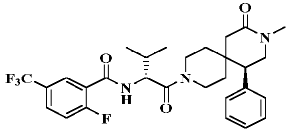 | 2-fluoro-N-((R)-3-methyl-1-((R)-9-methyl-10-oxo-7-phenyl-3,9-diazaspiro[5.5]undecan-3-yl)-1-oxobutan-2-yl)-5-(trifluoromethyl)benzamide |
| **240** | 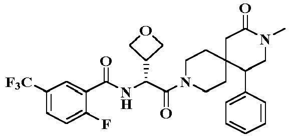 | 2-fluoro-N-((1R)-2-(9-methyl-10-oxo-7-phenyl-3,9-diazaspiro[5.5]undecan-3-yl)-1-(oxetan-3-yl)-2-oxoethyl)-5-(trifluoromethyl)benzamide |
| **241** | 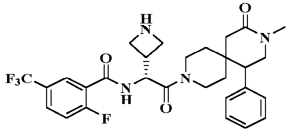 | N-((1R)-1-(azetidin-3-yl)-2-(9-methyl-10-oxo-7-phenyl-3,9-diazaspiro[5.5]undecan-3-yl)-2-oxoethyl)-2-fluoro-5-(trifluoromethyl)benzamide |
| **242** | 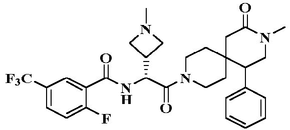 | 2-fluoro-N-((1R)-2-(9-methyl-10-oxo-7-phenyl-3,9-diazaspiro[5.5]undecan-3-yl)-1-(1-methylazetidin-3-yl)-2-oxoethyl)-5-(trifluoromethyl)benzamide |
| **243** | 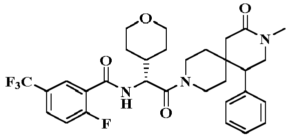 | 2-fluoro-N-((1R)-2-(9-methyl-10-oxo-7-phenyl-3,9-diazaspiro [5.5]undecan-3-yl)-2-oxo-1-(tetrahydro-2H-pyran-4-yl)ethyl)-5-(trifluoromethyl)benzamide |
| **244** | 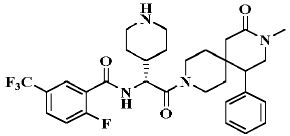 | 2-fluoro-N-((1R)-2-(9-methyl-10-oxo-7-phenyl-3,9-diazaspiro [5.5]undecan-3-yl)-2-oxo-1-(piperidin-4-yl)ethyl)-5-(trifluoromethyl)benzamide |
| **245** | 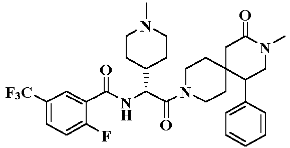 | 2-fluoro-N-((1R)-2-(9-methyl-10-oxo-7-phenyl-3,9-diazaspiro[5.5]undecan-3-yl)-1-(1-methylpiperidin-4-yl)-2-oxoethyl)-5-(trifluoromethyl)benzamide |

Following is a list of abbreviations used in the description of the preparation of the compounds of the present invention:
- ACN: : Acetonitrile
- BOC: : tert-Butyloxy carbonyl
- CDI: : Carbonyl diimidazole
- DCM: : Dichloro methane
- DIEA: : Diisopropyl ethyl amine
- DIPE: : Diisopropyl ether
- DIAD: : Diisopropylazodicarboxylate
- DMF: : N,N-Dimethyl formamide
- DMSO: : Dimethyl sulfoxide
- EtOH: : Ethanol
- EtOAc: : Ethyl acetate
- h: : hours
- HBTU: :*O*-(Benzotriazol-1-yl)-*N*,*N,N'*,*N*'-tetramethyluronium hexafluorophosphate
- HCl: : Hydrochloric acid
- HPLC: : High performance liquid chromatography
- IPA: : Isopropyl alcohol
- K₂CO₃: : Pottasium carbonate
- MeOH: : Methanol
- MeI: : Methyl iodide
- NaBH₄: : Sodium borohydride
- Na₂CO₃: : Sodium carbonate
- NaOH: : Sodium hydroxide
- Na₂SO₄: : Sodium sulfate
- NaHCO₃: : Sodium bicarbonate/sodium hydrogen carbonate
- NMP: : N-Methyl-2-pyrrolidone
- *t*-BuOK: : Potassium *tert*-butoxide
- TEA: : Triethylamine
- TFA: : Trifluoro acetic acid
- THF: : Tetrahydrofuran
- TLC: : Thin layer chromatography

The novel compounds of the present invention were prepared using the reactions and techniques described below, together with conventional techniques known to those skilled in the art of organic synthesis, or variations thereon as appreciated by those skilled in the art.

The reactions can be performed in solvents appropriate to the reagents and materials employed and are suitable for the transformations being effected. Preferred methods include, but not limited to those described below, where all symbols are as defined earlier unless and otherwise defined below.

The compounds of the formula (I) can be prepared as described in general **scheme-1** below along with suitable modifications/variations which are well within the scope of a person skilled in the art.

In a typical preparation. of a compound of formula (I), a compound of formula (II) and (III) were reacted under suitable amide coupling conditions. Suitable conditions include but are not limited to treating compounds of formula (II) and (III) with coupling reagents such as DCC or EDC in conjunction with DMAP, HOBt, HOAt, and the like. Morevere, HBTU, HATU in the presence of diisopropyl ethylamine (DIEA) can be used. Suitable solvents for use in the above process included, but were not limited to ethers such as THF, glyme, and the like DMF, DMSO, ACN, halogenated solvents such as CHCl₃ or DCM. If desired, mixtures of these sovents were used, however the preferred solvents were DCM and DMF. The above process was carried out at temperatures between about 0 °C and about 100 °C. Preferably, the reaction was carried out at RT. The above process was preferably carried out at or about atmospheric pressure although higher or lower pressures may be used if desired. The compound can be prepared by the conventional methods reported in literature or conventional techniques known to those skilled in the art of organic synthesis to furnish compounds of formula (I).

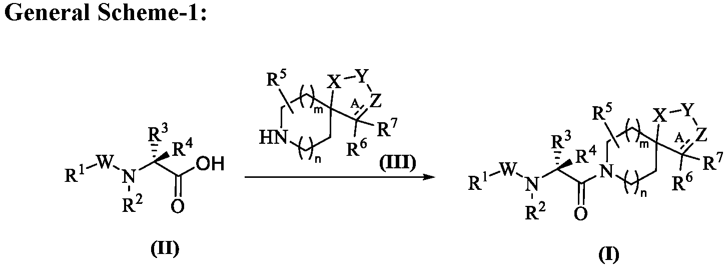

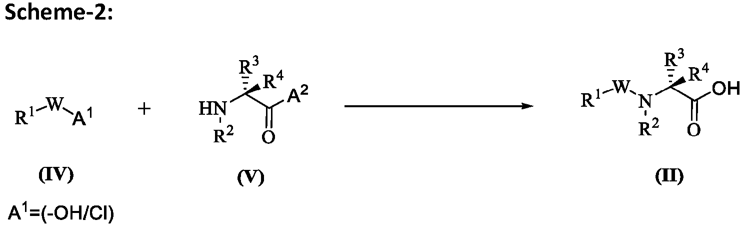

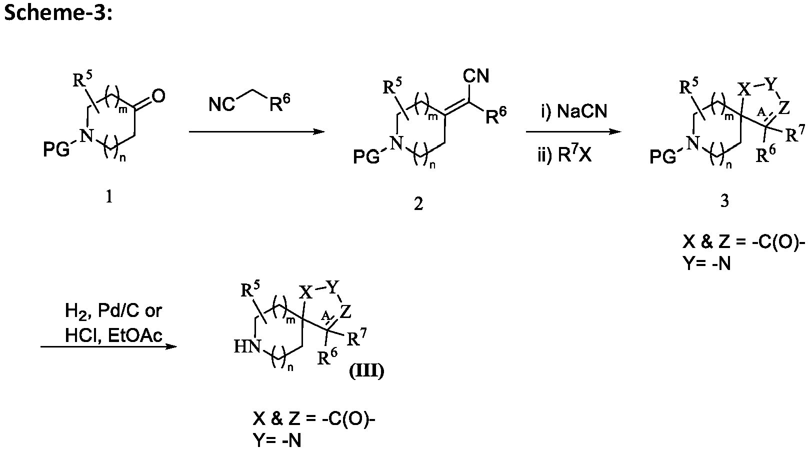

### General Methods

Melting points were recorded on a scientific melting point apparatus and are uncorrected. IR spectra were recorded as neat (for oils) or on KBr pellet (for solid) on FT-IR 8300 Shimadzu and are reported in wavenumbers v (cm⁻¹). NMR spectra were measured on a Varian Unity 400 (¹H at 400 MHz, ¹³C at 100 MHz), magnetic resonance spectrometer. Spectra were taken in the indicated solvent at ambient temperature. Chemical shifts (δ) are given in parts per million (ppm) with tetramethylsilane as an internal standard. Multiplicities are recorded as follows: s = singlet, d = doublet, t = triplet, q = quartet, br = broad. Coupling constants (*J* values) are given in Hz. Mass spectra are recorded on Perkin-Elmer Sciex API 3000. ESI-Q-TOF-MS measurements were performed with a micrOTOF-Q II (Bruker Daltonics) mass spectrometer. HPLC analysis were carried out at λmax 220 nm using column ODS C-18, 150 mm x 4.6 mm x 4 µm on AGILENT 1100 series. Reactions were monitored using thin layer silica gel chromatography (TLC) using 0.25 mm silica gel 60F plates from Merck. Plates were visualized by treatment with UV, acidic p-anisaldehyde stain, KMnO₄ stain with gentle heating. Products were purified by column chromatography using silica gel 100-200 mesh and the solvent systems indicated.

All reactions involving air or moisture sensitive compounds were performed under nitrogen atmosphere in flame dried glassware. Tetrahydrofuran (THF) and diethyl ether (Et₂O) were freshly distilled from sodium/ benzophenone under nitrogen atmosphere. Other solvents used for reactions were purified according to standard procedures. Starting reagents were purchased from commercial suppliers and used without further purification unless otherwise specified.

### Synthesis of Compound-1: 2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzamide

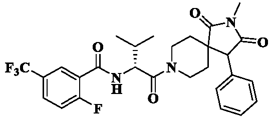

### Synthesis of compound-1 2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzamide was carried out as shown in Scheme-4 and the stepwise procedure is depicted below:

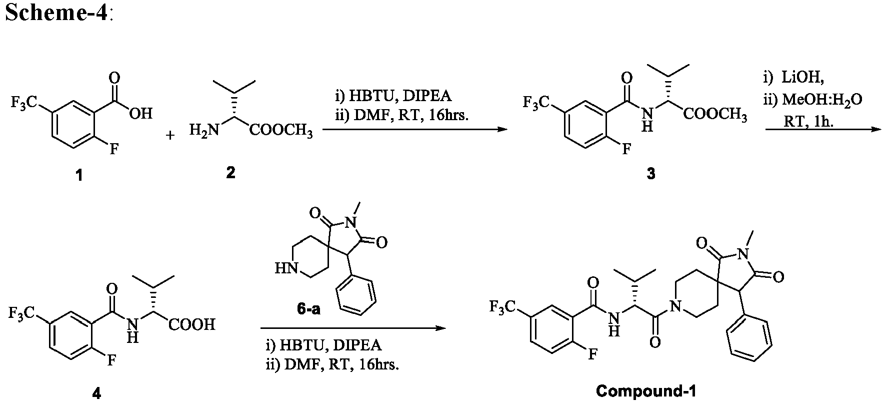

### Step-1: Preparation of methyl (2-fluoro-5-(trifluoromethyl)benzoyl)-D-valinate (3)

To a solution of 2-fluoro-5-(trifluoromethyl)benzoic acid (5.0 g, 24.03 mmol) in DMF (20 mL ) was added DIEA (12.59 mL, 72.1 mmol) at room temperature and stirred for 15 min. To the reaction mixture was added methyl D-valinate (1) (4.43 g, 26.4 mmol) and stirred for 16 h at RT. Reaction was poured into cold water and extracted with EtOAc (2 x 50 mL). Combined organic layer was washed with water (2 x 50 mL), sat. NaHCO₃ (1 x 50 mL) and brine (1 x 50 mL), dried over Na₂SO₄ and evaporated to dryness under reduced pressure. The residue was purified using a combiflash column chromatography (Hexane: EtOAC=1:5) to give methyl (2-fluoro-5-(trifluoromethyl)benzoyl)-D-valinate (3) as a solid (7.3 g, 95.0 % yield). **¹H NMR:** (CDCl₃, 400 MHz): δ 8.42 (m, 1H), 7.79-7.76 (m, 1H), 7.33-7.28 (m, 1H), 7.23-7.18 (m, 1H), 4.83-4.79 (m, 1H), 3.74 (s, 3H), 2.37-2.30 (m, 1H), 1.50-1.47 (m, 1H), 0.99 (d, 6H); ESI-MS: (m/z) 322.10 (M+H)⁺ (100 %).

### Step-2: 2-fluoro-5-(trifluoromethyl)benzoyl)-D-valine (4)

To a mixture of methyl methyl (2-fluoro-5-(trifluoromethyl)benzoyl)-D-valinate (3) (5.0 g, 15.56 mmol) in MeOH (30 mL) was added LiOH (1.96 g, 46.7 mmol) in H₂O (15 mL) at RT. Reaction mixture was stirred for 2 h at RT. Solvent was evaporated and diluted with water. Aqueous layer was acidified with 10% HCl solution and filtered to give (2-fluoro-5-(trifluoromethyl)benzoyl)-D-valine as a white solid (4) (4.51 g, 94.4 % yield). **¹H NMR:** (DMSO-d₆, 400 MHz): δ 12.79 (1H, s), 8.74-8.71 (d, 1H, J=8.40 Hz), 7.97-7.93 (m, 1H), 7.85-7.83 (m, 1H), 7.58-7.53 (t, 1H, J=9.2 Hz), 4.36-4.32 2.20-2.15 (m, 1H), 0.94 (d, 6H); ESI-MS: (m/z) 308.09 (M+H)⁺ (100 %).

### Step-3: 2-fluoro-N-((2R)-3-methyl-1-(2-methyl-1,3-dioxo-4-phenyl-2,8-diazaspiro[4.5] decan-8-yl)-1-oxobutan-2-yl)-5-(trifluoromethyl)benzamide (Compound-1)

To a solution of 2-fluoro-5-(trifluoromethyl)benzoyl)-D-valine (3) (0.34 g, 1.128 mmol) in DMF (3 mL) was added DIEA (0.53 mL, 3.08 mmol) at room temperature and stirred for 15 min. To the reaction mixture was added 2-methyl-4-phenyl-2,8-diazaspiro[4.5]decane-1,3-dione (6-a) (0.26 g, 1.026 mmol) and stirred for 16 h at RT. Reaction was poured into cold water and extracted with EtOAc (2 x 25 mL). Combined organic layer was washed with water (2 x 25 mL), sat. NaHCO₃ (1 x 25 mL) and brine (1 x 25 mL), dried over Na₂SO₄ and evaporated to dryness under reduced pressure. The residue was purified using a preparative HPLC to give the title compound 2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzamide (Compound-1) as a white solid. (0.35 g, 64.3% yield). **¹H NMR:** (CDCl₃, 400 MHz): δ 8.36-8.35 (d, 1H, J=8.56 Hz), 7.77-7.74 (m, 1H), 7.47-7.34 (m, 5H), 7.10-7.04 (m 2H), 5.05-4.97 (m, 1H), 4.23-4.17 (m, 1H), 3.92-3.82 (m, 2H), 3.44-3.31 (m, 3H), 3.15 (s, 3H), 2.12-2.07 (m, 2H), 2.05-1.93 (m, 2H), 1.61-1.56 (m, 2H), 1.30-1.27 (m,1H), 1.06-1.02 (m, 3H), 0.97 (d, 6H); ESI-MS: (m/z) 548.21 (M+H)⁺ (100 %).

### Synthesis of intermediate 6-a [2-methyl-4-phenyl-2,8-diazaspiro[4.5]decane-1,3-dione]

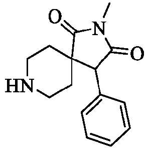

### Synthesis of intermediate-III (2-methyl-4-phenyl-2,8-diazaspiro[4.5]decane-1,3-dione) was carried out as shown in Scheme-5 and the stepwise procedure is depicted below:

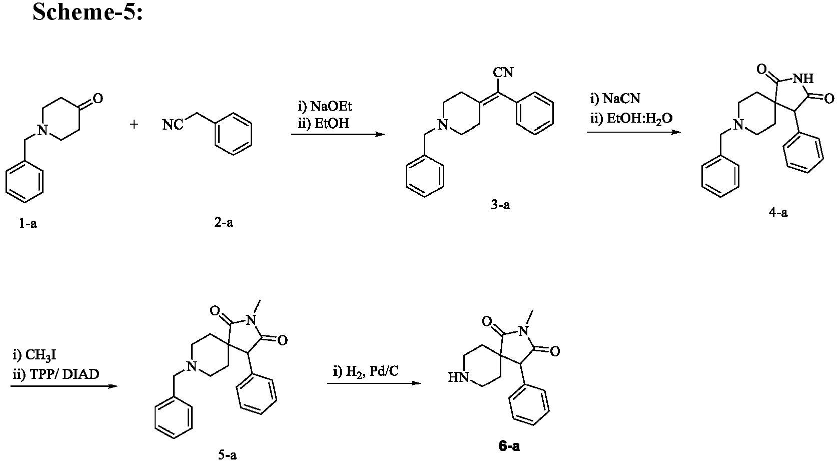

### Step-1: 2-(1-benzylpiperidin-4-ylidene)-2-phenylacetonitrile (3-a)

To a solution of sodium ethoxide (20 g, 88.8 mmol) in EtOH (45 mL) was added benzyl cyanide (5.04 g, 133.21 mmol) and N-benzyl piperidone (5.04 g, 133.21 mmol) at room temperature. The reaction mixture was stirred at 80-85°c for 3 h and then solvent was evaporated. The residue was poured into a mixture of ice (50 g) and conc. HCl (10 mL). The PH was then adjusted to 9 addition of solid NaOH (2 g) and extracted with EtOAc (3 x 25 mL). Organic phases were separated and dried over Na₂SO₄. The solvent was evaporated and crude product was purified by column chromatography using mobile phase (Hexane: EtOAc:TEA=80:20:1) gave 2-(1-benzylpiperidin-4-ylidene)-2-phenylacetonitrile (3-a) as yellow solid (6.40 g, 84.2% yield). **¹H NMR:** (CDCl₃, 400 MHz): δ 7.43-7.41 (m, 3H), 7.40-7.32 (m, 4H), 7.30-7.27 (m, 3H), 3.56 (s, 2H), 2.86-2.83 (m, 2H), 2.68-2.65 (m, 2H), 2.51-2.45 (m, 4H); ESI-MS: (m/z) 289.16 (M+H)⁺ (100 %).

### Step-2: 8-benzyl-4-phenyl-2,8-diazaspiro[4.5]decane-1,3-dione (4-a)

To a solution of 2-(1-benzylpiperidin-4-ylidene)-2-phenylacetonitrile (3-a) (6.35 g, 22.02 mmol) in EtOH (20 mL) was added NaCN (1.08 g, 22.02 mmol) in H₂O (3.8 mL). Reaction mixture was stirred for 16 h at 85-90 °C. Solvent was evaporated and residue was taken up in 2N HCl (14.2 mL) and Conc. HCl (2.4 mL) was added to adjust the PH 1-2. The reaction mixture was stirred for 16 h at reflux temperature. After cooling to room temperature solid Na₂CO₃ was added untill PH 8-9 and mixture was extracted with DCM (2 x 50 mL). Organic phase was seperated, dried over Na₂SO₄ and evaporated. The crude product was suspended in EtOAc (20 mL) and left at RT for overnight. The crystals were filtered off and dried to give 8-benzyl-4-phenyl-2,8-diazaspiro[4.5]decane-1,3-dione (4-a) as a solid (4.8 g, 67% yield). **¹H NMR:** (CDCl₃, 400 MHz): δ 7.45-7.35 (m, 4H), 7.34-7.32 (m, 1H), 7.28-7.23 (m, 2H), 7.15-7.13 (m, 2H), 4.17 (s, 1H), 3.56 (s, 2H), 2.89-2.83 (m, 1H), 2.55-2.51 (m, 2H), 2.17-2.12 (m, 2H), 1.86-1.82 (m, 3H), 1.38-1.33 (m, 1H); ESI-MS: (m/z) 335.17 (M+H)⁺ (100 %).

### Step-3: 8-benzyl-2-methyl-4-phenyl-2,8-diazaspiro[4.5]decane-1,3-dione (5-a)

To a solution of 8-benzyl-4-phenyl-2,8-diazaspiro[4.5]decane-1,3-dione (4-a) (1 g, 2.99 mmol) in THF (40 mL) was added triphenylphosphine (1.02 g, 3.89 mmol) MeOH (0.16, 3.89 mmol) and DIAD (0.79 g, 3.89 mmol) at RT. Reaction mixture was stirred for 16 h at RT. The solvent was evaporated and crude product was purified by column chromatography using mobile phase (DCM:MeOH=95:5) to give 8-benzyl-2-methyl-4-phenyl-2,8-diazaspiro[4.5]decane-1,3-dione (5-a). (0.66 g, 70% yield). **¹H NMR:** (CDCl₃, 400 MHz): δ 7.45-7.35 (m, 4H), 7.34-7.32 (m, 1H), 7.28-7.23 (m, 2H), 7.15-7.13 (m, 1H), 4.17 (s, 1H), 3.56 (s, 2H), 3.09 (s, 3H), 2.89-2.83 (m, 1H), 2.55-2.51 (m, 2H), 2.17-2.12 (m, 2H), 1.86-1.82 (m, 3H), 1.38-1.33 (m, 1H); **ESI-MS:** (m/z) 349.18 (M+H)⁺ (100 %).

### Step-4: 2-methyl-4-phenyl-2,8-diazaspiro[4.5]decane-1,3-dione (6-a)

To a solution of 8-benzyl-2-methyl-4-phenyl-2,8-diazaspiro[4.5]decane-1,3-dione **(5-a)** (0.36 g, 1.033 mmol) in MeOH (10 mL) was added 10% Pd/C (50%w/w) (0.036 g). Reaction mixture was stirred for 3 h at RT under H₂ atmosphere. The reaction mixture was filtered through celite and solvent was evaporated to give 2-methyl-4-phenyl-2,8-diazaspiro[4.5]decane-1,3-dione **(6-a).** (0.26 g, 67% yield). **¹H NMR:** (CDCl₃, 400 MHz): δ 7.39-7.28 (m, 5H), 7.11 (brs, 1H), 5.02-4.96 (m, 1H), 4.17 (s, 1H), 3.09 (s, 3H), 2.89-2.83 (m, 1H), 2.55-2.51 (m, 2H), 2.17-2.12 (m, 2H), 1.86-1.82 (m, 2H); **ESI-MS:** (m/z) 259.14 (M+H)⁺ (100 %).

Following specific novel compounds of general formula **(I)** of the present invention are prepared by using methods depicted in **general scheme-1**

### Compound-33: N-((2R)-1-(2-(2-aminoethyl)-1,3-dioxo-4-phenyl-2,8 diazaspiro[4.5]decan-8-yl)-3-methyl-1-oxobutan-2-yl)-2-fluoro-5-(trifluoromethyl)benzamide

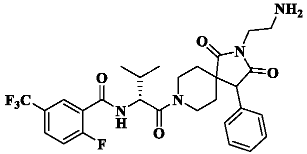

**¹H NMR:** (CDCl₃, 400 MHz): δ 8.37-8.34 (d, 1H, J=8.56 Hz), 7.77-7.74 (m, 1H), 7.47-7.34 (m, 5H), 7.10-7.04 (m 2H), 5.06-4.97 (m, 1H), 4.23-4.17 (m, 1H), 3.92-3.82 (m, 2H), 3.44-3.31 (m, 3H), 3.15 (s, 3H), 2.12-2.07 (m, 4H), 2.05-1.93 (m, 4H), 1.61-1.56 (m, 2H), 1.30-1.26 (m,1H), 1.06-1.02 (m, 3H), 0.97 (d, 6H); **ESI-MS:** (m/z) 577.24 (M+H)⁺ (100%).

### Compound-44: 2-fluoro-N-((2R)-3-methyl-1-(2-methyl-1-oxo-4-phenyl-2,8-diazaspiro-[4.5]decan-8-yl)-1-oxobutan-2-yl)-5-(trifluoromethyl)benzamide

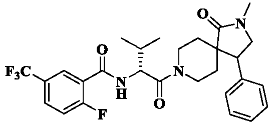

**¹H NMR:** (DMSO-d₆, 400 MHz): δ 8.68-8.62 (m, 1H), 7.93 (s, 1H), 7.83-7.78 (m, 1H), 7.55-7.53 (m, 1H), 7.28-7.21 (m, 5H), 4.74-4.70 (m, 1H), 3.72-3.69 (m, 2H), 3.67-3.62 (m, 2H), 3.51-3.48 (m, 2H), 3.38-3.36 (m,1H), 2.85 (s, 3H), 1.64-1.62 (m, 1H), 1.53-1.46 (m, 4H), 0.90 (s, 1H), 0.87 (d, 6H); **ESI-MS:** (m/z) 534.4 (M+H)⁺ (100%).

### Compound-45: 2-fluoro-N-((2R)-3-methyl-1-oxo-1-(1-oxo-4-phenyl-2,8-diazaspiro-[4.5]decan-8-yl)butan-2-yl)-5-(trifluoromethyl)benzamide

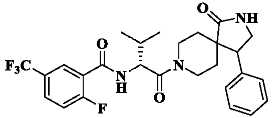

**¹H NMR:** (DMSO-d₆, 400 MHz): δ 8.67-8.62 (m, 1H), 7.93 (s, 1H), 7.83-7.78 (m, 1H), 7.55-7.53 (m, 1H), 7.28-7.21 (m, 5H), 4.74-4.70 (m, 1H), 3.72-3.69 (m, 2H), 3.67-3.62 (m, 2H), 3.51-3.48 (m, 2H), 3.38-3.36 (m,1H), 1.64-1.62 (m, 1H), 1.53-1.46 (m, 4H), 0.90 (s, 1H), 0.87 (d, 6H); **ESI-MS:** (m/z) 520.22 (M+H)⁺ (100%).

### Compound-56: 2-fluoro-N-((2R)-3-methyl-1-(2-methyl-1-oxo-4-(pyridin-3-yl)-2,8-diazaspiro[4.5]decan-8-yl)-1-oxobutan-2-yl)-5-(trifluoromethyl)benzamide

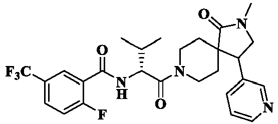

**¹H NMR:** (DMSO-d₆, 400 MHz): δ 8.69-8.67 (m, 1H), 8.51-8.49 (m, 1H), 8.46-8.42 (m, 1H), 7.93-7.82 (m, 1H), 7.66-7.62 (m, 1H), 7.56-7.53 (m, 1H), 7.39-7.29 (m, 2H), 4.86-4.74 (m, 1H), 3.85-3.79 (m, 2H), 3.73-3.70 (m, 2H), 3.69-3.62 (m, 2H), 3.55-3.52 (m, 1H), 3.41 (S, 3H), 2.03-1.89 (m, 1H), 1.76-1.72 (m, 1H), 1.65-1.54 (m, 3H), 0.92-0.81 (m, 6H); **ESI-MS:** (m/z) 535.23 (M+H)⁺ (100%).

### Compound-57: 2-fluoro-N-((2R)-3-methyl-1-oxo-1-(1-oxo-4-(pyridin-3-yl)-2,8-diazaspiro-[4.5]decan-8-yl)butan-2-yl)-5-(trifluoromethyl)benzamide

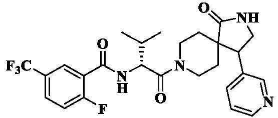

**¹H NMR:** (DMSO-d₆, 400 MHz): δ 8.67-8.66 (m, 1H), 8.53-8.50 (m, 1H), 8.47-8.43 (m, 1H), 7.93-7.82 (m, 1H), 7.66-7.62 (m, 1H), 7.56-7.53 (m, 1H), 7.39-7.29 (m, 2H), 4.86-4.74 (m, 1H), 3.85-3.79 (m, 2H), 3.74-3.70 (m, 2H), 3.69-3.62 (m, 2H), 3.55-3.52 (m, 1H), 2.03-1.89 (m, 1H), 1.76-1.72 (m, 1H), 1.65-1.54 (m, 3H), 0.92-0.81 (m, 6H); **ESI-MS:** (m/z) 521.21 (M+H)⁺ (100%).

### Compound-58: N-((2R)-1-(4-(1H-indazol-5-yl)-2-methyl-1-oxo-2,8-diazaspiro[4.5]decan-8-yl)-3-methyl-1-oxobutan-2-yl)-2-fluoro-5-(trifluoromethyl)benzamide

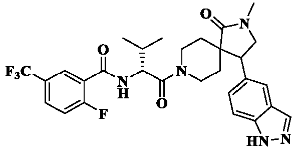

**¹H NMR:** (DMSO-d₆, 400 MHz): δ 8.65-8.61 (m, 1H), 8.51-8.49 (m, 1H), 8.46-8.42 (m, 1H), 7.93-7.82 (m, 2H), 7.66-7.62 (m, 1H), 7.56-7.53 (m, 1H), 7.39-7.29 (m, 2H), 4.86-4.74 (m, 1H), 3.85-3.79 (m, 2H), 3.73-3.70 (m, 2H), 3.69-3.62 (m, 2H), 3.55-3.52 (m, 1H), 3.41 (S, 3H), 2.03-1.89 (m, 1H), 1.76-1.72 (m, 1H), 1.66-1.56 (m, 3H), 0.94-0.83 (m, 6H); ESI-MS: (m/z) 574.40 (M+H)⁺ (100%).

### Compound-62: 2-fluoro-N-((2R)-3-methyl-1-(2-methyl-4-phenyl-2,8-diazaspiro[4.5]decan-8-yl)-1-oxobutan-2-yl)-5-(trifluoromethyl)benzamide

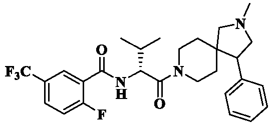

**¹H NMR:** (DMSO-d₆, 400 MHz): δ 8.35-8.34 (m, 1H), 7.76-7.73 (m, 1H), 7.33-7.30 (m, 1H), 7.28-7.25 (m, 2H), 7.23-7.16 (m,4H), 5.10-5.05 (m, 1H), 4.23-4.18 (m, 1H), 3.89-3.85 (m, 1H), 3.12-3.05 (m, 3H), 2.89-2.80 (m, 2H), 2.58-2.52 (m, 2H), 2.46 (s, 3H), 1.79-1.70 (m, 2H), 1.69-1.64 (m, 1H), 1.44-1.40 (m, 1H), 0.99-0.92 (m, 3H), 0.89-0,84 (m, 6H); ESI-MS: (m/z) 520.26 (M+H)⁺ (100%).

### Compound-64: 2-fluoro-N-((2R)-3-methyl-1-(2-methyl-4-(pyridin-3-yl)-2,8-diazaspiro-[4.5]decan-8-yl)-1-oxobutan-2-yl)-5-(trifluoromethyl)benzamide

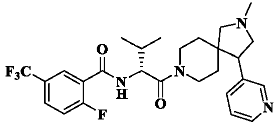

**¹H NMR:** (DMSO-d₆, 400 MHz): δ 8.66-8.64 (m, 1H), 8.46-8.44 (m, 1H), 8.38-833 (m, 1H), 7.76-7.73 (m, 1H), 7.33-7.30 (m, 1H), 7.28-7.25 (m, 1H), 7.23-7.16 (m,2H), 5.10-5.05 (m, 1H), 4.23-4.18 (m, 1H), 3.89-3.85 (m, 1H), 3.12-3.05 (m, 3H), 2.89-2.80 (m, 2H), 2.58-2.52 (m, 2H), 2.35 (s, 3H), 1.79-1.70 (m, 2H), 1.69-1.64 (m, 1H), 1.44-1.40 (m, 1H), 0.99-0.92 (m, 3H), 0.87-0,80 (m, 6H); **ESI-MS:** (m/z) 521.25 (M+H)⁺ (100%).

### Compound-65: N-((2R)-1-(4-(1H-indazol-5-yl)-2-methyl-2,8 diazaspiro[4.5]decan-8-yl)-3-methyl-1-oxobutan-2-yl)-2-fluoro-5-(trifluoromethyl)benzamide

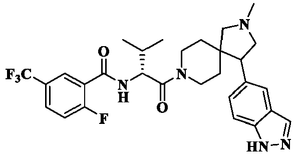

**¹H NMR:** (DMSO-d₆, 400 MHz): δ 12.97 (s, 1H), 8.17-8.16 (m, 1H), 7.96-7.93 (m, 1H), 7.33-7.62-7.59 (m, 1H), 7.51-7.47 (m, 2H), 7.28-7.23 (m, 4H), 4.73-4.71 (m, 1H), 4.23-4.18 (m, 1H), 3.89-3.85 (m, 1H), 3.12-3.05 (m, 3H), 2.89-2.80 (m, 2H), 2.58-2.52 (m, 2H), 3.09 (s, 3H), 1.79-1.70 (m, 2H), 1.69-1.64 (m, 1H), 1.44-1.40 (m, 1H), 0.99-0.92 (m, 3H), 0.85-0,80 (m, 6H); ESI-**MS:** (m/z) 560.53 (M+H)⁺ (100%).

### Compound-102: 2-fluoro-N-((2R)-1-(4-(4-fluorophenyl)-1-oxo-2,8-diazaspiro[4.5]decan-8-yl)-3-methyl-1-oxobutan-2-yl)-5-(trifluoromethyl)benzamide

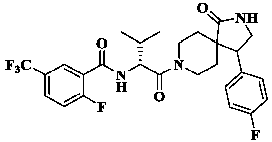

**¹H NMR:** (DMSO-d₆, 400 MHz): δ 8.35-8.33 (m, 1H), 7.76-7.73 (m, 1H), 7.30-7.25 (m, 1H), 7.24-20 (m, 1H), 7.19-7.14 (m, 2H), 7.06-7.00 (m, 2H), 5.86-5.84 (m, 1H), 5.10-5.05 (m, 1H), 4.12-4.09 (m, 1H), 3.83-3.75 (m, 3H), 3.64-3.55 (m, 2H), 2.02-1.94 (m, 2H), 1.71-1.67 (m, 3H), 1.04-0.99 (m, 3H), 0.97-0.89 (d, 6H); **ESI-MS:** (m/z) 538.30 (M+H)⁺ (100%).

### Compound-103: 2-fluoro-N-((2R)-1-(4-(4-fluorophenyl)-2-methyl-1-oxo-2,8-diazaspiro-[4.5]decan-8-yl)-3-methyl-1-oxobutan-2-yl)-5-(trifluoromethyl)benzamide

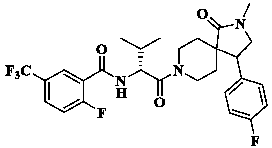

**¹H NMR:** (DMSO-d₆, 400 MHz): δ 8.35-8.33 (m, 1H), 7.76-7.73 (m, 1H), 7.53-7.51 (m, 1H), 7.28-7.25 (m, 1H), 7.10-6.97 (m, 4H), 5.05-5.00 (m, 1H), 4.12-3.96 (m, 1H), 3.76-3.71 (m, 3H), 3.66-3.558 (m, 2H), 3.24-3.21 (m, 2H), 2.99 (s, 3H), 2.09-1.95 (m, 2H), 1.67-1.63 (m, 2H), 1.04-0.89 (m, 6H); ESI-MS: (m/z) 552.32 (M+H)⁺ (100%).

### Compound-104: N-((2R)-1-(2-ethyl-4-(4-fluorophenyl)-1-oxo-2,8-diazaspiro[4.5]decan-8-yl)-3-methyl-1-oxobutan-2-yl)-2-fluoro-5-(trifluoromethyl)benzamide

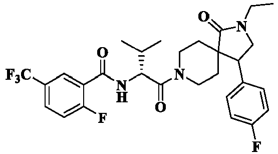

**¹H NMR:** (DMSO-d₆, 400 MHz): δ 8.35-8.33 (m, 1H), 7.76-7.73 (m, 1H), 7.53-7.51 (m, 1H), 7.28-7.25 (m, 1H), 7.10-6.97 (m, 4H), 5.05-5.00 (m, 1H), 4.12-3.96 (m, 1H), 3.76-3.71 (m, 3H), 3.66-3.558 (m, 2H), 3.46-3.42 (m, 2H), 3.24-3.21 (m, 2H), 2.09-1.95 (m, 2H), 1.67-1.63 (m, 2H), 1.24-1.14 (m, 3H), 1.04-0.89 (m, 6H); **ESI-MS:** (m/z) 566.30 (M+H)⁺ (100%).

### Compound-105: 2-fluoro-5-methoxy-N-((2R)-3-methyl-1-(2-methyl-1-oxo-4-phenyl-2,8-diazaspiro[4.5]decan-8-yl)-1-oxobutan-2-yl)benzamide

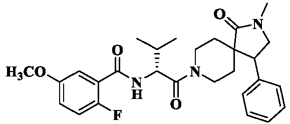

**¹H NMR:** (DMSO-d₆, 400 MHz): δ 8.32-8.29 (m, 1H), 7.76-7.73 (m, 1H), 7.51-7.49 (m, 1H), 7.40-7.24 (m, 6H), 5.32 (s, 1H), 5.02-5.00 (m, 2H), 4.32-4.29 (m, 2H), 3.84-3.81 (m, 2H), 3.77 (s, 3H), 3.50-3.44 (m, 1H), 3.39-3.37 (m, 1H), 2.98 (s, 3H), 2.96-2.94 (m, 1H), 1.96-1.91 (m, 1H), 1.68-1.62 (m, 2H), 1.40-1.37 9m, 2H), 1.02-0.97 (m, 3H), 0.91-0.85 (m, 4H); **ESI-MS:** (m/z) 496.30 (M+H)⁺ (100%).

### Compound-106: (R)-2-fluoro-N-(3-methyl-1-(2-methyl-1-oxo-4-phenyl-2,8-diazaspiro-[4.5]dec-3-en-8-yl)-1-oxobutan-2-yl)-5-(trifluoromethyl)benzamide

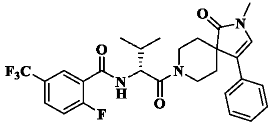

**¹H NMR:** (DMSO-d₆, 400 MHz): δ 8.38-8.36 (m, 1H), 7.79-7.76 (m, 1H), 7.58-7.54 (m, 1H), 7.36-7.32 (m, 2H), 7.28-7.23 (m, 3H), 6.60 (s, 1H), 5.15-5.11 (m, 1H), 4.53-4.50 (m, 1H), 4.21-4.17 (m, 1H), 3.93-3.90 (m, 1H), 3.71-3.68 (m, 1H), 3.15 (s, 3H), 2.16-2.11 (m, 2H), 1.69-1.62 (m, 2H), 0.99-0.90 (m, 3H), 0.89-0.84 (m, 3H); **ESI-MS:** (m/z) 532.21 (M+H)⁺ (100%).

### Compound-107: 2-fluoro-N-((2R)-3-methyl-1-(2-methyl-1-oxo-4-phenyl-2,8-diazaspiro-[4.5]decan-8-yl)-1-oxobutan-2-yl)benzamide

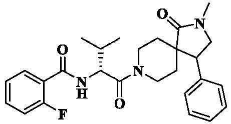

**¹H NMR:** (DMSO-d₆, 400 MHz): δ 8.67-8.62 (m, 1H), 7.94 (s, 1H), 7.82-7.78 (m, 2H), 7.55-7.53 (m, 1H), 7.28-7.21 (m, 5H), 4.74-4.70 (m, 1H), 3.72-3.69 (m, 2H), 3.67-3.62 (m, 2H), 3.51-3.48 (m, 2H), 3.38-3.36 (m,1H), 2.85 (s, 3H), 1.64-1.62 (m, 1H), 1.54-1.47 (m, 4H), 0.92 (s, 1H), 0.87 (d, 6H); **ESI-MS:** (m/z) 466.32 (M+H)⁺ (100%).

### Compound-108: N-((2R)-3-methyl-1-(2-methyl-1-oxo-4-phenyl-2,8-diazaspiro[4.5]decan-8-yl)-1-oxobutan-2-yl)-3-(trifluoromethyl)benzamide

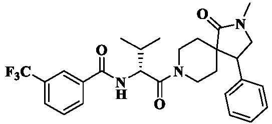

**¹H NMR:** (DMSO-d₆, 400 MHz): δ 8.86-8.84 (m, 1H), 8.22-8.16 (m, 2H), 7.82-7.78 (m, 2H), 7.55-7.53 (m, 1H), 7.28-7.21 (m, 4H), 4.74-4.70 (m, 1H), 3.72-3.69 (m, 2H), 3.67-3.62 (m, 2H), 3.51-3.48 (m, 2H), 3.38-3.36 (m,1H), 2.84 (s, 3H), 1.64-1.62 (m, 1H), 1.54-1.47 (m, 4H), 0.92 (s, 1H), 0.87 (d, 6H); **ESI-MS:** (m/z) 516.05 (M+H)⁺ (100%).

### Compound-109: 2-fluoro-5-methyl-N-((2R)-3-methyl-1-(2-methyl-1-oxo-4-phenyl-2,8-diazaspiro[4.5]decan-8-yl)-1-oxobutan-2-yl)benzamide

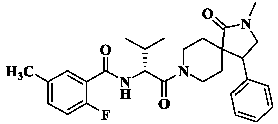

**¹H NMR:** (DMSO-d₆, 400 MHz): δ 7.55-7.53 (m, 1H), 7.37-7.21 (m, 6H), 7.13-7.10 (m, 1H), 4.95-4.91 (m, 1H), 4.88-4.84 (m, 1H), 3.87-3.81 (m, 2H), 3.69-3.62 (m, 3H), 3.33-3.31 (m, 1H), 2.39-2.34 (m, 3H), 1.81-1.79 (m, 2H), 1.76-1.72 (m, 2H), 1.01-0.98 (m, 3H), 0.95-0.89 (m, 5H); ESI-MS: (m/z) 479.59 (M+H)⁺ (100%).

### Compound-110: 2-fluoro-N-((2R)-3-methyl-1-(2-methyl-1-oxo-4-phenyl-2,8-diazaspiro-[4.5]decan-8-yl)-1-oxobutan-2-yl)-5-(trifluoromethoxy)benzamide

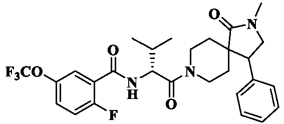

**¹H NMR:** (DMSO-d₆, 400 MHz): δ 7.91-7.89 (m, 1H), 7.55-7.53 (m, 1H), 7.36-7.30 (m, 3H), 7.20-7.15 (m, 3H), 4.74-4.70 (m, 1H), 3.72-3.69 (m, 2H), 3.67-3.62 (m, 2H), 3.51-3.48 (m, 2H), 3.38-3.36 (m,1H), 2.85 (s, 3H), 1.64-1.62 (m, 1H), 1.53-1.46 (m, 4H), 0.90 (s, 1H), 0.87 (d, 6H); ESI-MS: (m/z) 550.23 (M+H)⁺ (100%).

### Compound-111: 2-fluoro-N-((2R)-1-(4-(4-fluorophenyl)-2-isopropyl-1-oxo-2,8-diazaspiro-[4.5]decan-8-yl)-3-methyl-1-oxobutan-2-yl)-5-(trifluoromethyl)benzamide

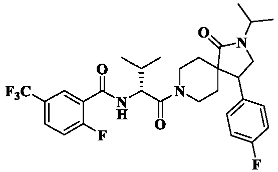

**¹H NMR:** (DMSO-d₆, 400 MHz): δ 8.35-8.33 (m, 1H), 7.76-7.73 (m, 1H), 7.53-7.51 (m, 1H), 7.28-7.25 (m, 1H), 7.10-6.97 (m, 4H), 5.05-5.00 (m, 1H), 4.12-3.96 (m, 1H), 3.76-3.71 (m, 3H), 3.66-3.558 (m, 2H), 3.24-3.21 (m, 3H), 2.09-1.95 (m, 2H), 1.67-1.63 (m, 2H), 1.04-0.89 (m, 6H); 0.86-0.82 (m, 6H); **ESI-MS:** (m/z) 580.26 (M+H)⁺ (100%).

### Compound-112: N-((2R)-1-(2-(cyclopropylmethyl)-4-(4-fluorophenyl)-1-oxo-2,8-diaza-spiro-[4.5]decan-8-yl)-3-methyl-1-oxobutan-2-yl)-2-fluoro-5-(trifluoromethyl)benzamide

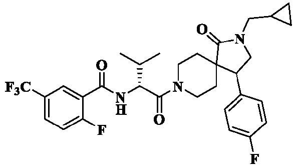

**¹H NMR:** (DMSO-d₆, 400 MHz): δ 8.36-8.34 (m, 1H), 7.75-7.74 (m, 1H), 7.51-7.41 (m, 1H), 7.28-7.25 (m, 1H), 7.19-7.12 (m, 2H), 7.06-0.99 (m, 2H), 5.05-5.00 (m, 1H), 3.97-3.78 (m, 5H), 3.31-3.27 (m, 2H), 3.26-3.21 (m, 3H), 2.12-2.05 (m, 2H), 1.67-1.65 (m, 1H), 1.05-0.98 (m, 4H), 0.95-0.87 (m, 4H), 0.61-0.58 (m, 2H), 0.31-0.28 (m, 2H); ESI-MS: (m/z) 592.26 (M+H)⁺ (100%).

### Compound-113: N-((2R)-1-(2-(cyclopropylmethyl)-1-oxo-4-(pyridin-3-yl)-2,8-diazaspiro-[4.5]decan-8-yl)-3-methyl-1-oxobutan-2-yl)-2-fluoro-5-(trifluoromethyl)benzamide

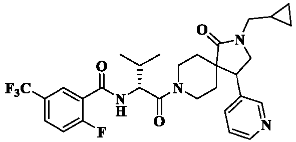

**¹H NMR:** (DMSO-d₆, 400 MHz): δ 8.36-8.34 (m, 1H), 7.68-7.60 (m, 1H), 7.51-7.41 (m, 1H), 7.25-7.22 (m, 1H), 7.16-7.12 (m, 1H), 7.06-0.99 (m, 2H), 5.05-5.00 (m, 1H), 3.97-3.78 (m, 5H), 3.31-3.27 (m, 2H), 3.26-3.21 (m, 3H), 2.12-2.05 (m, 2H), 1.67-1.65 (m, 1H), 1.05-0.98 (m, 4H), 0.95-0.87 (m, 4H), 0.61-0.58 (m, 2H), 0.31-0.28 (m, 2H); **ESI-MS:** (m/z) 575.26 (M+H)⁺ (100%).

### Compound-114: 2-fluoro-N-((2R)-1-(4-(4-methoxyphenyl)-2-methyl-1-oxo-2,8-diazaspiro-[4.5]decan-8-yl)-3-methyl-1-oxobutan-2-yl)-5-(trifluoromethyl)benzamide

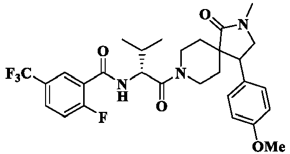

**¹H NMR:** (DMSO-d₆, 400 MHz): δ 8.35-8.33 (m, 1H), 7.76-7.73 (m, 1H), 7.53-7.51 (m, 1H), 7.28-7.25 (m, 1H), 7.10-6.97 (m, 4H), 5.05-5.00 (m, 1H), 4.12-3.96 (m, 1H), 3.76-3.71 (m, 3H), 3.66-3.558 (m, 2H), 3.24-3.21 (m, 2H), 3.21 (s, 3H), 2.99 (s, 3H), 2.09-1.95 (m, 2H), 1.67-1.63 (m, 2H), 1.04-0.89 (m, 6H); **ESI-MS:** (m/z) 564.25 (M+H)⁺ (100%).

### Compound-115: 2-fluoro-N-((2R)-3-methyl-1-oxo-1-(2-oxo-4-phenyl-1-oxa-3,8-diazaspiro-[4.5]decan-8-yl)butan-2-yl)-5-(trifluoromethyl)benzamide

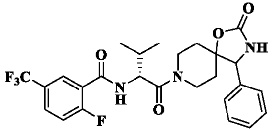

**¹H NMR:** (DMSO-d₆, 400 MHz): δ 8.68-8.62 (m, 1H), 7.93 (s, 1H), 7.83-7.78 (m, 1H), 7.55-7.53 (m, 1H), 7.28-7.21 (m, 5H), 4.74-4.70 (m, 1H), 3.72-3.69 (m, 2H), 3.67-3.62 (m, 2H), 3.51-3.48 (m, 2H), 3.38-3.36 (m,1H), 1.64-1.62 (m, 1H), 1.53-1.46 (m, 2H), 0.90 (s, 1H), 0.87 (d, 6H); ESI-MS: (m/z) 522.23 (M+H)⁺ (100%).

### Compound-118: (R)-2-fluoro-N-(1-(1-(4-fluorophenyl)-3-methyl-4-oxo-2,3,8-triazaspiro-[4.5]dec-1-en-8-yl)-3-methyl-1-oxobutan-2-yl)-5-(trifluoromethyl)benzamide

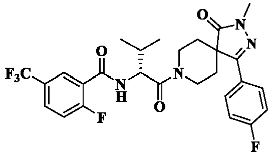

**¹H NMR:** (DMSO-d₆, 400 MHz): δ 9.04-9.02 (m, 1H), 7.96-7.94 (m, 1H), 7.88-7.87 (m, 1H), 7.77-7.71 (m, 2H), 7.57-7.53 (m, 1H), 7.47-7.46 (m, 1H), 7.36-7.32 (m, 2H), 4.82-4.77 (m, 1H), 4.40-4.38 (m, 1H), 4.18-4.14 (m, 1H), 2.68 (s, 3H), 2.19-2.10 (m, 3H), 2.08-2.04 (m, 3H), 1.01-0.94 (m, 6H) 1.04-0.89 (m, 6H); **ESI-MS:** (m/z) 533.22 (M+H)⁺ (100%).

### Compound-119: 2-fluoro-N-((2R)-1-(4-(4-fluorophenyl)-2-methyl-2,8-diazaspiro[4.5]decan-8-yl)-3-methyl-1-oxobutan-2-yl)-5-(trifluoromethyl)benzamide

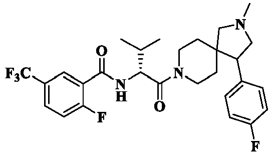

**¹H NMR:** (DMSO-d₆, 400 MHz): δ 8.56-8.54 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 2H), 1.26-1.20 (m, 2H), 1.10-0.89 (m, 6H); ESI-MS: (m/z) 538.32 (M+H)⁺ (100%).

### Compound-120: 2-fluoro-N-((2R)-1-(4-(4-fluorophenyl)-2-isopropyl-2,8-diazaspiro[4.5]-decan-8-yl)-3-methyl-1-oxobutan-2-yl)-5-(trifluoromethyl)benzamide

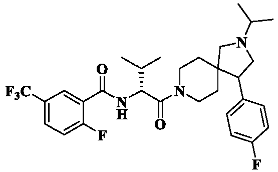

**¹H NMR:** (DMSO-d₆, 400 MHz): δ 8.52-8.50 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.03-1.96 (m, 2H), 1.26-1.20 (m, 2H), 1.10-0.89 (m, 12H); **ESI-MS:** (m/z) 566.28 (M+H)⁺ (100%).

### Compound-121: N-((2R)-1-(2-(cyclopropylmethyl)-4-(4-fluorophenyl)-2,8-diazaspiro[4.5]-decan-8-yl)-3-methyl-1-oxobutan-2-yl)-2-fluoro-5-(trifluoromethyl)benzamide

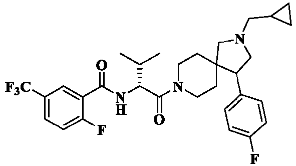

**¹H NMR:** (DMSO-d₆, 400 MHz): δ 8.36-8.34 (m, 1H), 7.75-7.74 (m, 1H), 7.51-7.41 (m, 1H), 7.28-7.25 (m, 1H), 7.19-7.12 (m, 2H), 7.06-0.99 (m, 2H), 5.05-5.00 (m, 1H), 3.97-3.78 (m, 5H), 3.31-3.27 (m, 2H), 3.26-3.21 (m, 3H), 2.12-2.05 (m, 4H), 1.67-1.65 (m, 1H), 1.05-0.98 (m, 4H), 0.95-0.87 (m, 4H), 0.61-0.58 (m, 2H), 0.31-0.28 (m, 2H); ESI-MS: (m/z) 578.28 (M+H)⁺ (100%).

### Compound-122: 2-fluoro-N-((2R)-1-(4-(4-methoxyphenyl)-2-methyl-2,8-diazaspiro[4.5]-decan-8-yl)-3-methyl-1-oxobutan-2-yl)-5-(trifluoromethyl)benzamide

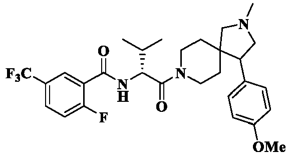

**¹H NMR:** (DMSO-d₆, 400 MHz): δ 8.35-8.33 (m, 1H), 7.76-7.73 (m, 1H), 7.53-7.51 (m, 1H), 7.28-7.25 (m, 1H), 7.10-6.97 (m, 4H), 5.05-5.00 (m, 1H), 4.12-3.96 (m, 1H), 3.76-3.71 (m, 3H), 3.66-3.558 (m, 2H), 3.24-3.21 (m, 2H), 3.21 (s, 3H), 2.99 (s, 3H), 2.09-1.95 (m, 4H), 1.67-1.63 (m, 2H), 1.04-0.89 (m, 6H); **ESI-MS:** (m/z) 560.27 (M+H)⁺ (100%).

### Compound-123: 2-fluoro-N-((2R)-1-(4-(4-methoxyphenyl)-2-methyl-2,8-diazaspiro-[4.5]decan-8-yl)-3-methyl-1-oxobutan-2-yl)-5-methylbenzamide

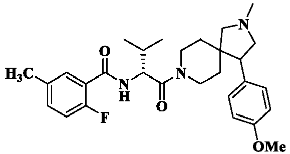

**¹H NMR:** (DMSO-d₆, 400 MHz): δ 8.19-8.16 (m, 1H), 7.76-7.73 (m, 1H), 7.48-7.46 (m, 1H), 7.28-7.25 (m, 1H), 7.10-6.97 (m, 4H), 5.05-5.00 (m, 1H), 4.12-3.96 (m, 1H), 3.76-3.71 (m, 3H), 3.66-3.558 (m, 2H), 3.24-3.21 (m, 2H), 3.21 (s, 3H), 2.56 (s, 3H), 2.33 (s, 3H), 2.09-1.95 (m, 4H), 1.67-1.63 (m, 2H), 0.86-0.70 (m, 6H); **ESI-MS:** (m/z) 496.29 (M+H)⁺ (100%).

### Compound-129: N-((2R)-1-(4-(4-fluorophenyl)-2-methyl-2,8-diazaspiro-[4.5]decan-8-yl)-3-methyl-1-oxobutan-2-yl)-4-methylpicolinamide

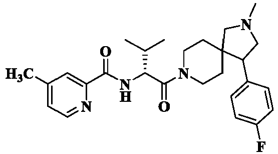

**¹H NMR:** (DMSO-d₆, 400 MHz): δ 8.68-8.64 (m, 1H), 8.43-8.40 (m,1H), 7.99-7.96 (m, 1H), 7.23-7.18 (m, 2H), 7.07-7.04 (m, 1H), 6.93-6.91 (m, 1H), 4.96-4.93 (m, 1H), 4.32-4.28 (m, 2H), 3.06-3.01 (m, 2H), 2.89-2.82 (m, 2H), 2.78-2.76 (m, 1H), 2.63-2.60 (m, 2H), 2.45-2.42 (m, 4H), 2.09-2.07 (m, 1H), 1.74-1.67 (m, 6H), 0.96-0.85 (m, 6H); **ESI-MS:** (m/z) 467.28 (M+H)⁺ (100%).

### Compound-130: methyl-8-((2-fluoro-5-(trifluoromethyl)benzoyl)-D-valyl)-4-(4-fluoro-phenyl)-2,8-diazaspiro[4.5]decane-2-carboxylate

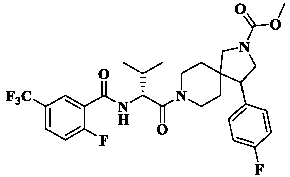

**¹H NMR:** (DMSO-d₆, 400 MHz): δ 8.67-8.65 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 2H), 0.96-0.89 (m, 2H), 0.79-0.66 (m, 6H); **ESI-MS:** (m/z) 582.24 (M+H)⁺ (100%).

### Compound-131: ethyl 8-((2-fluoro-5-(trifluoromethyl)benzoyl)-D-valyl)-4-(4-fluorophenyl)-2,8-diazaspiro[4.5]decane-2-carboxylate

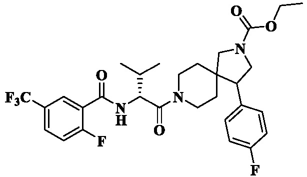

**¹H NMR:** (DMSO-d₆, 400 MHz): δ 8.67-8.65 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 4H), 3.09-2.94 (m, 4H), 2.72-2.65 (m, 4H), 2.51 (s, 3H), 2.03-1.96 (m, 2H), 0.96-0.89 (m, 5H), 0.79-0.66 (m, 6H); **ESI-MS:** (m/z) 596.25 (M+H)⁺ (100%).

### Compound-132: 4-(8-((2-fluoro-5-(trifluoromethyl)benzoyl)-D-valyl)-2-methyl-2,8-diazaspiro[4.5]decan-4-yl)benzoic acid

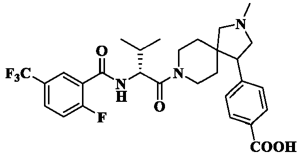

**¹H NMR:** (DMSO-d₆, 400 MHz): δ 12.005 (brs, 1H), 8.56-8.54 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 1H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 2H), 1.26-1.20 (m, 2H), 1.10-0.89 (m, 6H); **ESI-MS:** (m/z) 564.23 (M+H)⁺ (100%).

### Compound-133: 2-fluoro-N-((R)-1-((R)-4-(4-fluorophenyl)-2-methyl-2,8-diazaspiro[4.5]-decan-8-yl)-3-methyl-1-oxobutan-2-yl)-5-(trifluoromethyl)benzamide

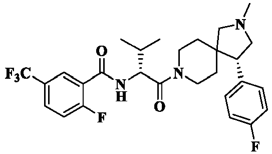

**¹H NMR:** (DMSO-d₆, 400 MHz): δ 8.56-8.54 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 2H), 1.26-1.20 (m, 2H), 1.10-0.89 (m, 6H); **ESI-MS:** (m/z) 538.25 (M+H)⁺ (100%).

### Compound-134: 2-fluoro-N-((R)-1-((S)-4-(4-fluorophenyl)-2-methyl-2,8-diazaspiro[4.5]-decan-8-yl)-3-methyl-1-oxobutan-2-yl)-5-(trifluoromethyl)benzamide

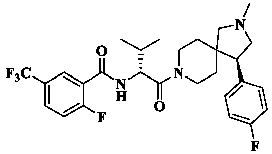

**¹H NMR:** (DMSO-d₆, 400 MHz): δ 8.56-8.54 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 2H), 1.26-1.20 (m, 2H), 1.10-0.89 (m, 6H); **ESI-MS:** (m/z) 538.25 (M+H)⁺ (100%).

### Compound-136: 2-fluoro-N-((2R)-3-methyl-1-(2-methyl-4-(p-tolyl)-2,8-diazaspiro[4.5]decan-8-yl)-1-oxobutan-2-yl)-5-(trifluoromethyl)benzamide

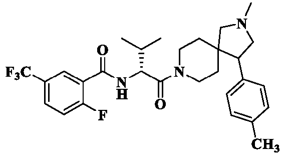

**¹H NMR:** (DMSO-d₆, 400 MHz): δ 8.48-8.46 (m, 1H), 7.88-7.86 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.30 (s, 3H), 2.03-1.96 (m, 2H), 1.26-1.20 (m, 2H), 1.10-0.89 (m, 6H); **ESI-MS:** (m/z) 534.27 (M+H)⁺ (100%).

### Compound-138: N-((2R)-1-(4-(3,5-dimethylphenyl)-2-methyl-2,8-diazaspiro[4.5]decan-8-yl)-3-methyl-1-oxobutan-2-yl)-2-fluoro-5-(trifluoromethyl)benzamide

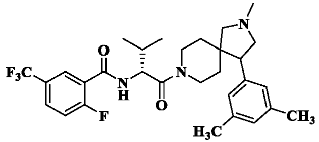

**¹H NMR:** (DMSO-d₆, 400 MHz): δ 8.48-8.46 (m, 1H), 7.88-7.86 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.18 (s, 6H), 2.03-1.96 (m, 2H), 1.26-1.20 (m, 2H), 1.10-0.89 (m, 6H); **ESI-MS:** (m/z) 548.29 (M+H)⁺ (100%).

### Compound-140: 2-fluoro-N-((1R)-2-(4-(4-fluorophenyl)-2-methyl-2,8-diazaspiro[4.5]decan-8-yl)-2-oxo-1-phenylethyl)-5-(trifluoromethyl)benzamide

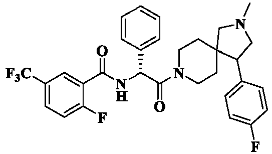

**¹H NMR:** (DMSO-d₆, 400 MHz): δ 8.51-8.49 (m, 1H), 8.33-8.31 (m, 1H), 7.71-7.69 (m, 1H), 7.45-7.01 (m, 8H), 6.99-6.95 (m, 2H), 5.88-5.86 (m, 1H), 4.23-4.21 (m, 1H), 3.53-3.50 (m, 1H), 3.10-3.05 (m, 1H), 2.77-2.70 (m, 4H), 2.49 (s, 3H), 1.78-1.71 (m, 3H), 1.27-1.25 (m, 2H); **ESI-MS:** (m/z) 572.23 (M+H)⁺ (100%).

### Compound-142: N-((1R)-1-cyclohexyl-2-(4-(4-fluorophenyl)-2-methyl-2,8-diazaspiro[4.5]-decan-8-yl)-2-oxoethyl)-2-fluoro-5-(trifluoromethyl)benzamide

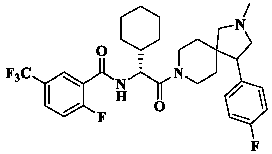

**¹H NMR:** (DMSO-d₆, 400 MHz): δ 8.60-8.58 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 4H), 1.26-1.20 (m, 4H), 1.10-0.89 (m, 4H); ESI-MS: (m/z) 578.28 (M+H)⁺ (100%).

### Compound-147: 2-fluoro-N-((2R)-1-(4-(4-fluorophenyl)-2-methyl-2,8-diazaspiro[4.5]decan-8-yl)-4-methyl-1-oxopentan-2-yl)-5-(trifluoromethyl)benzamide

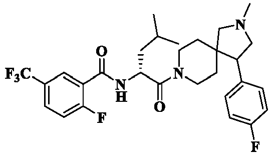

**¹H NMR:** (DMSO-d₆, 400 MHz): δ 8.48-8.46 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.53 (s, 3H), 2.03-1.96 (m, 4H), 1.26-1.20 (m, 2H), 1.10-0.92 (m, 6H); **ESI-MS:** (m/z) 552.26 (M+H)⁺ (100%).

### Compound-149: 2,5-difluoro-N-((2R)-1-(4-(4-fluorophenyl)-2-methyl-2,8-diazaspiro[4.5]-decan-8-yl)-3-methyl-1-oxobutan-2-yl)benzamide

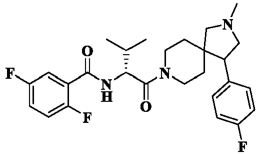

**¹H NMR:** (DMSO-d₆, 400 MHz): δ 8.56-8.54 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.51 (s, 3H), 2.03-1.96 (m, 2H), 1.26-1.20 (m, 2H), 1.10-0.89 (m, 6H); **ESI-MS:** (m/z) 488.20 (M+H)⁺ (100%).

### Compound-151: 2,5-dichloro-N-((2R)-1-(4-(4-fluorophenyl)-2-methyl-2,8-diazaspiro[4.5]-decan-8-yl)-3-methyl-1-oxobutan-2-yl)benzamide

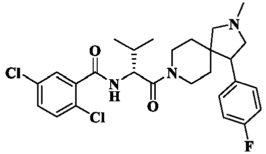

**¹H NMR:** (DMSO-d₆, 400 MHz): δ 8.53-8.51 (m, 1H), 7.94-7.92 (m, 1H), 7.82-7.80 (m, 1H), 7.52-7.50 (m, 1H), 7.29-7.26 (m, 1H), 7.20-7.18 (m, 1H), 7.05-7.01 (m, 2H), 4.72-4.70 (m, 1H), 4.68-4.65 (m, 2H), 3.09-2.94 (m, 4H), 2.72-2.65 (m, 4H), 2.54 (s, 3H), 2.03-1.96 (m, 2H), 1.26-1.20 (m, 2H), 1.10-0.89 (m, 6H); **ESI-MS:** (m/z) 520.19 (M+H)⁺ (100%).

### Compound-153: N-((2R)-1-(4-(4-fluorophenyl)-2-methyl-2,8-diazaspiro[4.5]decan-8-yl)-3-methyl-1-oxobutan-2-yl)-1-methyl-1H-indazole-3-carboxamide

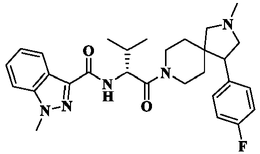

**¹H NMR:** (DMSO-d₆, 400 MHz): δ 8.26-8.24 (m, 1H), 7.84-7.82 (m, 1H), 7.76-7.74 (m, 1H), 7.52-7.50 (m, 2H), 7.29-7.26 (m, 1H), 7.20-7.18 (m, 1H), 7.05-7.01 (m, 2H), 4.72-4.70 (m, 1H), 4.68-4.65 (m, 2H), 4.14 (s, 3H), 3.09-2.94 (m, 4H), 2.72-2.65 (m, 4H), 2.51 (s, 3H), 2.03-1.96 (m, 2H), 1.26-1.20 (m, 2H), 1.10-0.89 (m, 6H); **ESI-MS:** (m/z) 506.04 (M+H)⁺ (100%).

### Compound-158: N-((2R)-1-(4-(4-fluorophenyl)-2-methyl-2,8-diazaspiro[4.5]decan-8-yl)-3-methyl-1-oxobutan-2-yl)-4,6-dimethylpicolinamide

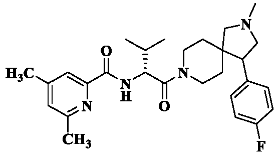

**¹H NMR:** (DMSO-d₆, 400 MHz): δ 8.68-8.64 (m, 1H), 8.43-8.40 (m, 1H), 7.99-7.96 (m, 1H), 7.23-7.18 (m, 2H), 7.07-7.04 (m, 1H), 6.93-6.91 (m, 1H), 4.96-4.93 (m, 1H), 4.32-4.28 (m, 2H), 3.06-3.01 (m, 2H), 2.89-2.82 (m, 2H), 2.78-2.76 (m, 1H), 2.63-2.60 (m, 2H), 2.56 (s, 3H), 2.45(s, 3H), 2.42 (s, 3H), 2.09-2.07 (m, 2H), 1.74-1.67 (m, 6H), 0.96-0.85 (m, 6H); ESI-MS: (m/z) 491.12 (M+H)⁺ (100%).

### Compound-162: 4-methyl-N-((2R)-3-methyl-1-(2-methyl-4-(pyridin-3-yl)-2,8-diazaspiro-[4.5]decan-8-yl)-1-oxobutan-2-yl)picolinamide

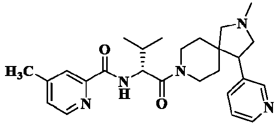

**¹H NMR:** (DMSO-d₆, 400 MHz): δ 8.64-8.62 (m, 1H), 8.43-8.40 (m,1H), 7.99-7.96 (m, 1H), 7.23-7.18 (m, 1H), 7.07-7.04 (m, 2H), 6.93-6.91 (m, 1H), 4.96-4.93 (m, 1H), 4.32-4.28 (m, 2H), 3.06-3.01 (m, 2H), 2.89-2.82 (m, 2H), 2.78-2.76 (m, 1H), 2.63-2.60 (m, 2H), 2.56 (s, 3H), 2.45(s, 3H), 2.09-2.07 (m, 2H), 1.74-1.67 (m, 6H), 0.96-0.85 (m, 6H); **ESI-MS:** (m/z) 450.26 (M+H)⁺ (100%).

### Compound-163: 2-fluoro-N-((2R)-3-methyl-1-oxo-1-(10-oxo-7-phenyl-3,9-diazaspiro[5.5]-undecan-3-yl)butan-2-yl)-5-(trifluoromethyl)benzamide

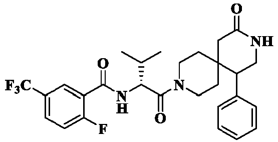

**¹H NMR:** (DMSO-d₆, 400 MHz): δ 8.66-8.64 (m, 1H), 7.94-7.92 (m, 1H), 7.83-7.81 (m, 1H), 7.55-7.50 (m, 2H), 7.27-7.17 (m, 4H), 4.74-4.70 (m, 2H), 4.14-4.10 (m, 2H), 3.52-3.50 (m, 2H), 3.35-3.33 (m, 2H), 3.03-2.96 (m, 2H), 2.24-2.20 (m, 2H), 1.46-1.41 (m, 2H), 1.34-1.30 (m, 2H), 0.88-0.73 (m, 6H); **ESI-MS:** (m/z) 534.23 (M+H)⁺ (100%).

### Compound-164: 2-fluoro-N-((2R)-1-(7-(4-fluorophenyl)-10-oxo-3,9-diazaspiro[5.5]-undecan-3-yl)-3-methyl-1-oxobutan-2-yl)-5-(trifluoromethyl)benzamide

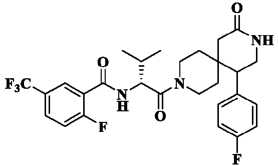

**¹H NMR:** (DMSO-d₆, 400 MHz): δ 8.67-8.64 (m, 1H), 7.94-7.92 (m, 1H), 7.83-7.81 (m, 1H), 7.55-7.50 (m, 1H), 7.27-7.17 (m, 4H), 4.74-4.70 (m, 2H), 4.14-4.10 (m, 2H), 3.52-3.50 (m, 2H), 3.35-3.33 (m, 2H), 3.03-2.96 (m, 2H), 2.24-2.20 (m, 2H), 1.46-1.41 (m, 2H), 1.34-1.30 (m, 2H), 0.88-0.73 (m, 6H); **ESI-MS:** (m/z) 552.22 (M+H)⁺ (100%).

### Compound-174: 2-fluoro-N-((2R)-3-methyl-1-(9-methyl-10-oxo-7-phenyl-3,9-diazaspiro-[5.5]undecan-3-yl)-1-oxobutan-2-yl)-5-(trifluoromethyl)benzamide

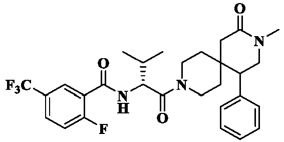

**¹H NMR:** (DMSO-d₆, 400 MHz): δ 8.64-8.62 (m, 1H), 7.93-7.91 (m, 1H), 7.83-7.81 (m, 1H), 7.55-7.53 (m, 1H), 7.33-7.14 (m, 5H), 4.71-4.70 (m, 1H), 4.12-4.08 (m, 1H), 3.85-3.83 (m, 1H), 3.65-3.63 (m, 1H), 3.46-3.44 (m, 1H), 3.33-3.25 (m, 1H), 3.08-3.06 (m, 1H), 2.89 (s, 3H), 2.85-2.83 (m, 1H), 2.27-2.25 (m, 1H), 1.91-1.88 (m, 1H), 1.44-1.40 (m, 2H), 1.33-1.30 (m, 2H), 1.25-1.23 (m, 1H), 0.88-0.82 (m, 6H); **ESI-MS:** (m/z) 548.25 (M+H)⁺ (100%).

### Compound-176: 2-fluoro-N-((2R)-1-(7-(4-fluorophenyl)-9-methyl-10-oxo-3,9-diazaspiro-[5.5]undecan-3-yl)-3-methyl-1-oxobutan-2-yl)-5-(trifluoromethyl)benzamide

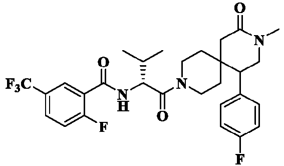

**¹H NMR:** (DMSO-d₆, 400 MHz): δ 8.67-8.65 (m, 1H), 7.93-7.92 (m, 1H), 7.83-7.81 (m, 1H), 7.55-7.53 (m, 1H), 7.33-7.14 (m, 4H), 4.71-4.70 (m, 1H), 4.12-4.08 (m, 1H), 3.85-3.83 (m, 1H), 3.65-3.63 (m, 1H), 3.46-3.44 (m, 1H), 3.33-3.25 (m, 1H), 3.08-3.06 (m, 1H), 2.88 (s, 3H), 2.85-2.83 (m, 1H), 2.27-2.25 (m, 1H), 1.91-1.88 (m, 1H), 1.44-1.40 (m, 2H), 1.33-1.30 (m, 2H), 1.25-1.23 (m, 1H), 0.88-0.82 (m, 6H); **ESI-MS:** (m/z) 566.24 (M+H)⁺ (100%).

### Compound-177: N-((2R)-1-(9-ethyl-7-(4-fluorophenyl)-10-oxo-3,9-diazaspiro[5.5]undecan-3-yl)-3-methyl-1-oxobutan-2-yl)-2-fluoro-5-(trifluoromethyl)benzamide

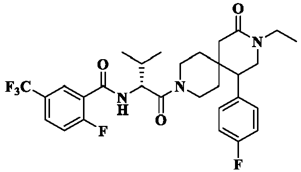

**¹H NMR:** (DMSO-d₆, 400 MHz): δ 8.67-8.65 (m, 1H), 7.93-7.92 (m, 1H), 7.83-7.81 (m, 1H), 7.55-7.53 (m, 1H), 7.33-7.14 (m, 4H), 4.71-4.70 (m, 1H), 4.12-4.08 (m, 1H), 3.85-3.83 (m, 3H), 3.65-3.63 (m, 1H), 3.46-3.44 (m, 1H), 3.33-3.25 (m, 1H), 3.08-3.06 (m, 1H), 2.85-2.83 (m, 1H), 2.27-2.25 (m, 1H), 1.91-1.88 (m, 1H), 1.44-1.40 (m, 2H), 1.33-1.30 (m, 2H), 1.25-1.23 (m, 1H), 1.09-1.05 (m, 3H), 0.88-0.82 (m, 6H); **ESI-MS:** (m/z) 580.26 (M+H)⁺ (100%).

### Compound-185: 2-fluoro-N-((2S)-3-methyl-1-(9-methyl-10-oxo-7-phenyl-3,9-diazaspiro-[5.5]undecan-3-yl)-1-oxobutan-2-yl)-5-(trifluoromethyl)benzamide

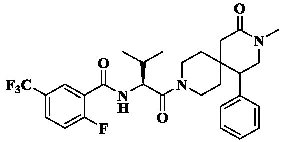

**¹H NMR:** (DMSO-d₆, 400 MHz): δ 8.64-8.62 (m, 1H), 7.93-7.91 (m, 1H), 7.83-7.81 (m, 1H), 7.55-7.53 (m, 1H), 7.33-7.14 (m, 5H), 4.71-4.70 (m, 1H), 4.12-4.08 (m, 1H), 3.85-3.83 (m, 1H), 3.65-3.63 (m, 1H), 3.46-3.44 (m, 1H), 3.33-3.25 (m, 1H), 3.08-3.06 (m, 1H), 2.89 (s, 3H), 2.85-2.83 (m, 1H), 2.27-2.25 (m, 1H), 1.91-1.88 (m, 1H), 1.44-1.40 (m, 2H), 1.33-1.30 (m, 2H), 1.25-1.23 (m, 1H), 0.88-0.82 (m, 6H); **ESI-MS:** (m/z) 548.25 (M+H)⁺ (100%).

### Compound-194: N-((2R)-3-methyl-1-(9-methyl-10-oxo-7-phenyl-3,9-diazaspiro[5.5]-undecan-3-yl)-1-oxobutan-2-yl)-4-(trifluoromethyl)picolinamide

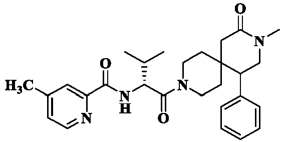

**¹H NMR:** (DMSO-d₆, 400 MHz): δ 8.51-8.49 (m, 1H), 7.90-7.87 (m, 1H), 7.85-7.83 (m, 1H), 7.46-7.44 (m, 1H), 7.33-7.14 (m, 5H), 4.71-4.70 (m, 1H), 4.12-4.08 (m, 1H), 3.85-3.83 (m, 1H), 3.65-3.63 (m, 1H), 3.46-3.44 (m, 1H), 3.33-3.25 (m, 1H), 3.08-3.06 (m, 1H), 2.89 (s, 3H), 2.85-2.83 (m, 1H), 2.50 (s, 3H), 2.27-2.25 (m, 1H), 1.91-1.88 (m, 1H), 1.44-1.40 (m, 2H), 1.33-1.30 (m, 2H), 1.25-1.23 (m, 1H), 0.88-0.82 (m, 6H); **ESI-MS:** (+ve mode) 477.28 (M+H)⁺ (100%).

### Compound-220: 2-fluoro-N-((2R)-3-methyl-1-(9-methyl-7-phenyl-3,9-diazaspiro[5.5]-undecan-3-yl)-1-oxobutan-2-yl)-5-(trifluoromethyl)benzamide

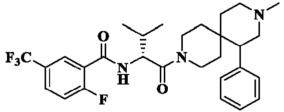

**¹H NMR:** (DMSO-d₆, 400 MHz): δ 8.63-8.61 (m, 1H), 7.92-7.90 (m, 1H), 7.81-7.80 (m, 1H), 7.75-7.74 (m, 1H), 7.29-7.13 (m, 5H), 4.67-4.60 (m, 1H), 4.08-4.04 (m, 1H), 3.83-3.81 (m, 1H), 3.24-3.21 (m, 1H), 2.77-2.65 (m, 3H), 2.27-2.16 (m, 5H), 1.86-1.84 (m, 1H), 1.76-1.72 (m, 2H), 1.38-1.24 (m, 3H), 0.85-0.83 (m, 3H), 0.58-0.53 (m, 3H); **ESI-MS:** (+ve mode) 534.27 (M+H)⁺ (100%).

### Compound-221: 2-fluoro-N-((2R)-1-(7-(4-fluorophenyl)-9-methyl-3,9-diazaspiro[5.5]-undecan-3-yl)-3-methyl-1-oxobutan-2-yl)-5-(trifluoromethyl)benzamide

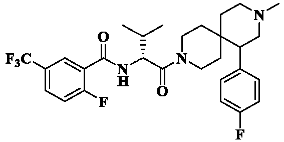

**¹H NMR:** (DMSO-d₆, 400 MHz): δ 8.62-8.60 (m, 1H), 7.93-7.92 (m, 1H), 7.81-7.80 (m, 1H), 7.75-7.74 (m, 1H), 7.29-7.13 (m, 4H), 4.67-4.60 (m, 1H), 4.08-4.04 (m, 1H), 3.83-3.81 (m, 1H), 3.24-3.21 (m, 1H), 2.77-2.65 (m, 3H), 2.27-2.16 (m, 5H), 1.86-1.84 (m, 1H), 1.76-1.72 (m, 2H), 1.38-1.24 (m, 3H), 0.85-0.83 (m, 3H), 0.58-0.55 (m, 3H); **ESI-MS:** (+ve mode) 552.26 (M+H)⁺ (100%).

In some embodiments, the present invention includes a pharmaceutical composition comprising the compound or salt of any one of the novel compounds of formula **(I),** formulated with or without one or more pharmaceutical carriers.

In some embodiments, the present invention includes a method for the treatment of at least one of cancer, chronic inflammation, neuropathic pain, fibrotic diseases mediated in part by ATX comprising administering to a subject in need thereof a therapeutically effective amount of a compound or salt of the novel compound of formula **(I).**

### Biological studies:

### ATX Inhibitory activity (IC₅₀ determination):

ATX Inhibitory activity was determined in a biochemical or whole blood assay as described previously (Bretschneider, T. et al., SLAS Discov 2017, 22, 425). The biochemical reaction consisted of 50 mM Tris (pH 8.0), 3 mM KCl, 1 mM CaCl₂, 1 mM Mg Cl₂, 0.14 mM NaCl and 0.1% bovine serum albumin, which was supplemented with 5 nM recombinant rat ATX, 5 µM 18:1 LPC and the test compound (0.1 - 10 µM). The reaction was stopped at 2 h by the addition of butanol, prior to the RapidFire-MS-based analysis.

The whole blood assay consisted of 45 µL µL heparinized mice/ rat whole blood and the test compound (0.12 - 100 µM). The reaction was stopped after 1 h at 37 °C by addition of 100 µL 40 mM disodium hydrogen phosphate buffer containing 30 mM citric acid (pH 4) and 1 µM 17:0 LPA (internal standard). Afterwards the samples were treated a described above and analysed by LC-MS/MS. The data was analyzed using graph pad prism (v 7.03) to arrive at the half-maximal inhibitory concentrations (IC₅₀) of the test compounds.

Mice serum ATX inhibitory activity (IC₅₀) for representative compounds are listed in the **Table 2.**

| **Table 2:** Mice serum ATX inhibitory activity (IC₅₀) of representative compounds | |
|---|---|
| Example No. | Mice serum IC₅₀ (nM) |
| **1** | 91 |
| **33** | 69.8 |
| **44** | 4 |
| **45** | 19.2 |
| **56** | 13.3 |
| **57** | 56.2 |
| **58** | 58.9 |
| **62** | 6.6 |
| **64** | 13.4 |
| **65** | 6.9 |
| **102** | 22.6 |
| **103** | 6.9 |
| **104** | 11.7 |
| **105** | 25.6 |
| **106** | 55.6 |
| **107** | 66.8 |
| **108** | 16.4 |
| **109** | 10.1 |
| **110** | 3.8 |
| **111** | 34.2 |
| **112** | 73.4 |
| **113** | 101.5 |
| **114** | 1.7 |
| **115** | 74.4 |
| **118** | 126.5 |
| **119** | 7.1 |
| **120** | 44.4 |
| **121** | 84.4 |
| **122** | 6.8 |
| **123** | 9.3 |
| **129** | 3.4 |
| **130** | 59.7 |
| **131** | 150.3 |
| **132** | 1.1 |
| **133** | 12.0 |
| **134** | 7.8 |
| **136** | 2.2 |
| **138** | 40.4 |
| **140** | 11.5 |
| **142** | 4.4 |
| **147** | 4.1 |
| **149** | 47.3 |
| **151** | 129.5 |
| **153** | 48.4 |
| **158** | 6.9 |
| **162** | 10.2 |
| **163** | 3.5 |
| **164** | 6.5 |
| **174** | 11.2 |
| **176** | 7.5 |
| **177** | 17.2 |
| **185** | 12.0 |
| **194** | 15.9 |
| **220** | 9.9 |
| **221** | 5.1 |
| **GLPG1690** | 508 |

In an embodiment, the present invention provides a pharmaceutical composition comprising a therapeutically effective amount of a compound of formula (I) and optionally one or more pharmaceutically acceptable excipients. The novel compounds of the present invention can be formulated into suitable pharmaceutically acceptable compositions by combining with suitable excipients by techniques and processes and concentrations as are well known.

The compounds of formula **(I),** or pharmaceutical compositions containing them are useful as a medicament for the inhibition of ATX activity and suitable for humans and other warm blooded animals, and may be administered either by oral, topical or parenteral administration.

The quantity of active component, that is, the novel compounds of formula (I) according to this invention, in the pharmaceutical composition and unit dosage form thereof may be varied or adjusted widely depending upon several factors such as the particular application method, the potency of the particular compound and the desired concentration.

The novel compounds of the present invention can be formulated into suitable pharmaceutically acceptable compositions by combining with suitable excipients by techniques and processes and concentrations as are well known. The pharmaceutical compositions further comprise an effective amount of an ATX inhibitor. The dosage of ATX inhibitors may vary within wide limits and should be adjusted, in each particular case, to the individual conditions.

In an embodiment, the compound of formula (I), may be used alone or in any combination with one or more therapeutic agents such as anti-inflammatory agents, antitumor agents, antifibrotic agents, autotaxin inhibitors, immunomodulators and cardiovascular agents and other therapeutic agents which are known to skilled medical practitioner. The selection of such therapeutic agents may be depend upon the type of disease and its severity, condition of patient being treated, and other medications being taken by the patients, etc.

In some embodiments, the present disclosure includes a pharmaceutical composition comprising the compound or salt of any one of the novel compounds of formula **(I),** formulated with or without one or more pharmaceutical carriers.

In some embodiments, the present disclosure includes compounds for use in treating of at least one of cancer, chronic inflammation, neuropathic pain, fibrotic diseases mediated in part by ATX comprising administering to a subject in need thereof a therapeutically effective amount of a compound or salt of the novel compound of Table I.

## Claims

1. Compound selected from:
2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzamide;
N-((2R)-1-(2-(2-aminoethyl)-1,3-dioxo-4-phenyl-2,8-diazaspiro[4.5]decan-8-yl)-3-methyl-1-oxobutan-2-yl)-2-fluoro-5-(trifluoromethyl)benzamide;
2-fluoro-N-((2R)-3-methyl-1-(2-methyl-1-oxo-4-phenyl-2,8-diazaspiro[4.5]decan-8-yl)-1-oxobutan-2-yl)-5-(trifluoromethyl)benzamide;
2-fluoro-N-((2R)-3-methyl-1-oxo-1-(1-oxo-4-phenyl-2,8-diazaspiro[4.5]decan-8-yl)butan-2-yl)-5-(trifluoromethyl)benzamide;
2-fluoro-N-((2R)-3-methyl-1-(2-methyl-1-oxo-4-(pyridin-3-yl)-2,8-diazaspiro[4.5]decan-8-yl)-1-oxobutan-2-yl)-5-(trifluoromethyl)benzamide;
2-fluoro-N-((2R)-3-methyl-1-oxo-1-(1-oxo-4-(pyridin-3-yl)-2,8-diazaspiro[4.5]decan-8-yl)butan-2-yl)-5-(trifluoromethyl)benzamide;
N-((2R)-1-(4-(1H-indazol-5-yl)-2-methyl-1-oxo-2,8-diazaspiro[4.5]decan-8-yl)-3-methyl-1-oxobutan-2-yl)-2-fluoro-5-(trifluoromethyl)benzamide;
2-fluoro-N-((2R)-3-methyl-1-(2-methyl-4-phenyl-2,8-diazaspiro[4.5]decan-8-yl)-1-oxobutan-2-yl)-5-(trifluoromethyl)benzamide;
2-fluoro-N-((2R)-3-methyl-1-(2-methyl-4-(pyridin-3-yl)-2,8-diazaspiro[4.5]decan-8-yl)-1-oxobutan-2-yl)-5-(trifluoromethyl)benzamide;
N-((2R)-1-(4-(1H-indazol-5-yl)-2-methyl-2,8-diazaspiro[4.5]decan-8-yl)-3-methyl-1-oxobutan-2-yl)-2-fluoro-5-(trifluoromethyl)benzamide;
2-fluoro-N-((2R)-1-(4-(4-fluorophenyl)-1-oxo-2,8-diazaspiro[4.5]decan-8-yl)-3-methyl-1-oxobutan-2-yl)-5-(trifluoromethyl)benzamide;
2-fluoro-N-((2R)-1-(4-(4-fluorophenyl)-2-methyl-1-oxo-2,8-diazaspiro[4.5]decan-8-yl)-3-methyl-1-oxobutan-2-yl)-5-(trifluoromethyl)benzamide;
N-((2R)-1-(2-ethyl-4-(4-fluorophenyl)-1-oxo-2,8-diazaspiro[4.5]decan-8-yl)-3-methyl-1-oxobutan-2-yl)-2-fluoro-5-(trifluoromethyl)benzamide;
2-fluoro-5-methoxy-N-((2R)-3-methyl-1-(2-methyl-1-oxo-4-phenyl-2,8-diazaspiro[4.5]decan-8-yl)-1-oxobutan-2-yl)benzamide;
(R)-2-fluoro-N-(3-methyl-1-(2-methyl-1-oxo-4-phenyl-2,8-diazaspiro[4.5]dec-3-en-8-yl)-1-oxobutan-2-yl)-5-(trifluoromethyl)benzamide;
2-fluoro-N-((2R)-3-methyl-1-(2-methyl-1-oxo-4-phenyl-2,8-diazaspiro[4.5]decan-8-yl)-1-oxobutan-2-yl)benzamide;
N-((2R)-3-methyl-1-(2-methyl-1-oxo-4-phenyl-2,8-diazaspiro[4.5]decan-8-yl)-1-oxobutan-2-yl)-3-(trifluoromethyl)benzamide;
2-fluoro-5-methyl-N-((2R)-3-methyl-1-(2-methyl-1-oxo-4-phenyl-2,8-diazaspiro[4.5]decan-8-yl)-1-oxobutan-2-yl)benzamide;
2-fluoro-N-((2R)-3-methyl-1-(2-methyl-1-oxo-4-phenyl-2,8-diazaspiro[4.5]decan-8-yl)-1-oxobutan-2-yl)-5-(trifluoromethoxy)benzamide;
2-fluoro-N-((2R)-1-(4-(4-fluorophenyl)-2-isopropyl-1-oxo-2,8-diazaspiro[4.5]decan-8-yl)-3-methyl-1-oxobutan-2-yl)-5-(trifluoromethyl)benzamide;
N-((2R)-1-(2-(cyclopropylmethyl)-4-(4-fluorophenyl)-1-oxo-2,8-diazaspiro[4.5]decan-8-yl)-3-methyl-1-oxobutan-2-yl)-2-fluoro-5-(trifluoromethyl)benzamide;
N-((2R)-1-(2-(cyclopropylmethyl)-1-oxo-4-(pyridin-3-yl)-2,8-diazaspiro[4.5]decan-8-yl)-3-methyl-1-oxobutan-2-yl)-2-fluoro-5-(trifluoromethyl)benzamide;
2-fluoro-N-((2R)-1-(4-(4-methoxyphenyl)-2-methyl-1-oxo-2,8-diazaspiro[4.5]decan-8-yl)-3-methyl-1-oxobutan-2-yl)-5-(trifluoromethyl)benzamide;
2-fluoro-N-((2R)-3-methyl-1-oxo-1-(2-oxo-4-phenyl-1-oxa-3,8-diazaspiro[4.5]decan-8-yl)butan-2-yl)-5-(trifluoromethyl)benzamide;
(R)-2-fluoro-N-(1-(1-(4-fluorophenyl)-3-methyl-4-oxo-2,3,8-triazaspiro[4.5]dec-1-en-8-yl)-3-methyl-1-oxobutan-2-yl)-5-(trifluoromethyl)benzamide;
2-fluoro-N-((2R)-1-(4-(4-fluorophenyl)-2-methyl-2,8-diazaspiro[4.5]decan-8-yl)-3-methyl-1-oxobutan-2-yl)-5-(trifluoromethyl)benzamide;
2-fluoro-N-((2R)-1-(4-(4-fluorophenyl)-2-isopropyl-2,8-diazaspiro[4.5]decan-8-yl)-3-methyl-1-oxobutan-2-yl)-5-(trifluoromethyl)benzamide;
N-((2R)-1-(2-(cyclopropylmethyl)-4-(4-fluorophenyl)-2,8-diazaspiro[4.5]decan-8-yl)-3-methyl-1-oxobutan-2-yl)-2-fluoro-5-(trifluoromethyl)benzamide;
2-fluoro-N-((2R)-1-(4-(4-methoxyphenyl)-2-methyl-2,8-diazaspiro[4.5]decan-8-yl)-3-methyl-1-oxobutan-2-yl)-5-(trifluoromethyl)benzamide;
2-fluoro-N-((2R)-1-(4-(4-methoxyphenyl)-2-methyl-2,8-diazaspiro[4.5]decan-8-yl)-3-methyl-1-oxobutan-2-yl)-5-methylbenzamide;
N-((2R)-1-(4-(4-fluorophenyl)-2-methyl-2,8-diazaspiro-[4.5]decan-8-yl)-3-methyl-1-oxobutan-2-yl)-4-methylpicolinamide;
methyl 8-((2-fluoro-5-(trifluoromethyl)benzoyl)-D-valyl)-4-(4-fluorophenyl)-2,8-diazaspiro[4.5]decane-2-carboxylate;
ethyl 8-((2-fluoro-5-(trifluoromethyl)benzoyl)-D-valyl)-4-(4-fluorophenyl)-2,8-diazaspiro[4.5]decane-2-carboxylate;
4-(8-((2-fluoro-5-(trifluoromethyl)benzoyl)-D-valyl)-2-methyl-2,8-diazaspiro[4.5]decan-4-yl)benzoic acid;
2-fluoro-N-((R)-1-((R)-4-(4-fluorophenyl)-2-methyl-2,8-diazaspiro[4.5]decan-8-yl)-3-methyl-1-oxobutan-2-yl)-5-(trifluoromethyl)benzamide;
2-fluoro-N-((R)-1-((S)-4-(4-fluorophenyl)-2-methyl-2,8-diazaspiro[4.5]decan-8-yl)-3-methyl-1-oxobutan-2-yl)-5-(trifluoromethyl)benzamide;
2-fluoro-N-((2R)-3-methyl-1-(2-methyl-4-(p-tolyl)-2,8-diazaspiro[4.5]decan-8-yl)-1-oxobutan-2-yl)-5-(trifluoromethyl)benzamide;
N-((2R)-1-(4-(3,5-dimethylphenyl)-2-methyl-2,8-diazaspiro[4.5]decan-8-yl)-3-methyl-1-oxobutan-2-yl)-2-fluoro-5-(trifluoromethyl)benzamide;
2-fluoro-N-((1R)-2-(4-(4-fluorophenyl)-2-methyl-2,8-diazaspiro[4.5]decan-8-yl)-2-oxo-1-phenylethyl)-5-(trifluoromethyl)benzamide;
N-((1R)-1-cyclohexyl-2-(4-(4-fluorophenyl)-2-methyl-2,8-diazaspiro[4.5]decan-8-yl)-2-oxoethyl)-2-fluoro-5-(trifluoromethyl)benzamide
2-fluoro-N-((2R)-1-(4-(4-fluorophenyl)-2-methyl-2,8-diazaspiro[4.5]decan-8-yl)-4-methyl-1-oxopentan-2-yl)-5-(trifluoromethyl)benzamide;
2,5-difluoro-N-((2R)-1-(4-(4-fluorophenyl)-2-methyl-2,8-diazaspiro[4.5]decan-8-yl)-3-methyl-1-oxobutan-2-yl)benzamide;
2,5-dichloro-N-((2R)-1-(4-(4-fluorophenyl)-2-methyl-2,8-diazaspiro[4.5]decan-8-yl)-3-methyl-1-oxobutan-2-yl)benzamide;
N-((2R)-1-(4-(4-fluorophenyl)-2-methyl-2,8-diazaspiro[4.5]decan-8-yl)-3-methyl-1-oxobutan-2-yl)-1-methyl-1H-indazole-3-carboxamide;
N-((2R)-1-(4-(4-fluorophenyl)-2-methyl-2,8-diazaspiro[4.5]decan-8-yl)-3-methyl-1-oxobutan-2-yl)-4,6-dimethylpicolinamide;
4-methyl-N-((2R)-3-methyl-1-(2-methyl-4-(pyridin-3-yl)-2,8-diazaspiro[4.5]decan-8-yl)-1-oxobutan-2-yl)picolinamide;
2-fluoro-N-((2R)-3-methyl-1-oxo-1-(10-oxo-7-phenyl-3,9-diazaspiro[5.5]undecan-3-yl)butan-2-yl)-5-(trifluoromethyl)benzamide;
2-fluoro-N-((2R)-1-(7-(4-fluorophenyl)-10-oxo-3,9-diazaspiro[5.5]undecan-3-yl)-3-methyl-1-oxobutan-2-yl)-5-(trifluoromethyl)benzamide;
2-fluoro-N-((2R)-3-methyl-1-(9-methyl-10-oxo-7-phenyl-3,9-diazaspiro[5.5]undecan-3-yl)-1-oxobutan-2-yl)-5-(trifluoromethyl)benzamide;
2-fluoro-N-((2R)-1-(7-(4-fluorophenyl)-9-methyl-10-oxo-3,9-diazaspiro[5.5]undecan-3-yl)-3-methyl-1-oxobutan-2-yl)-5-(trifluoromethyl)benzamide;
N-((2R)-1-(9-ethyl-7-(4-fluorophenyl)-10-oxo-3,9-diazaspiro[5.5]undecan-3-yl)-3-methyl-1-oxobutan-2-yl)-2-fluoro-5-(trifluoromethyl)benzamide;
2-fluoro-N-((2S)-3-methyl-1-(9-methyl-10-oxo-7-phenyl-3,9-diazaspiro[5.5]undecan-3-yl)-1-oxobutan-2-yl)-5-(trifluoromethyl)benzamide;
N-((2R)-3-methyl-1-(9-methyl-10-oxo-7-phenyl-3,9-diazaspiro[5.5]undecan-3-yl)-1-oxobutan-2-yl)-4-(trifluoromethyl)picolinamide;
2-fluoro-N-((2R)-3-methyl-1-(9-methyl-7-phenyl-3,9-diazaspiro[5.5]undecan-3-yl)-1-oxobutan-2-yl)-5-(trifluoromethyl)benzamide;
2-fluoro-N-((2R)-1-(7-(4-fluorophenyl)-9-methyl-3,9-diazaspiro[5.5]undecan-3-yl)-3-methyl-1-oxobutan-2-yl)-5-(trifluoromethyl)benzamide.

## Patentansprüche

1. Verbindung, ausgewählt aus:
2-fluoro-N-((2R)-3-methyl-1-(2-methyl-1,3-dioxo-4-phenyl-2,8-diazaspiro[4.5]decan-8-yl)-1-oxobutan-2-yl)-5-(trifluoromethyl)benzamid;
N-((2R)-1-(2-(2-aminoethyl)-1,3-dioxo-4-phenyl-2,8-diazaspiro[4.5]decan-8-yl)-3-methyl-1-oxobutan-2-yl)-2-fluoro-5-(trifluoromethyl)benzamid;
2-fluoro-N-((2R)-3-methyl-1-(2-methyl-1-oxo-4-phenyl-2,8-diazaspiro[4.5]decan-8-yl)-1-oxobutan-2-yl)-5-(trifluoromethyl)benzamid;
2-fluoro-N-((2R)-3-methyl-1-oxo-1-(1-oxo-4-phenyl-2,8-diazaspiro[4.5]decan-8-yl)butan-2-yl)-5-(trifluoromethyl)benzamid;
2-fluoro-N-((2R)-3-methyl-1-(2-methyl-1-oxo-4-(pyridin-3-yl)-2,8-diazaspiro[4.5]decan-8-yl)-1-oxobutan-2-yl)-5-(trifluoromethyl)benzamid;
2-fluoro-N-((2R)-3-methyl-1-oxo-1-(1-oxo-4-(pyridin-3-yl)-2,8-diazaspiro[4.5]decan-8-yl)butan-2-yl)-5-(trifluoromethyl)benzamid;
N-((2R)-1-(4-(1H-indazol-5-yl)-2-methyl-1-oxo-2,8-diazaspiro[4.5]decan-8-yl)-3-methyl-1-oxobutan-2-yl)-2-fluoro-5-(trifluoromethyl)benzamid;
2-fluoro-N-((2R)-3-methyl-1-(2-methyl-4-phenyl-2,8-diazaspiro[4.5]decan-8-yl)-1-oxobutan-2-yl)-5-(trifluoromethyl)benzamid;
2-fluoro-N-((2R)-3-methyl-1-(2-methyl-4-(pyridin-3-yl)-2,8-diazaspiro[4.5]decan-8-yl)-1-oxobutan-2-yl)-5-(trifluoromethyl)benzamid;
N-((2R)-1-(4-(1H-indazol-5-yl)-2-methyl-2,8-diazaspiro[4.5]decan-8-yl)-3-methyl-1-oxobutan-2-yl)-2-fluoro-5-(trifluoromethyl)benzamid;
2-fluoro-N-((2R)-1-(4-(4-fluorophenyl)-1-oxo-2,8-diazaspiro[4.5]decan-8-yl)-3-methyl-1-oxobutan-2-yl)-5-(trifluoromethyl)benzamid;
2-fluoro-N-((2R)-1-(4-(4-fluorophenyl)-2-methyl-1-oxo-2,8-diazaspiro[4.5]decan-8-yl)-3-methyl-1-oxobutan-2-yl)-5-(trifluoromethyl)benzamid;
N-((2R)-1-(2-ethyl-4-(4-fluorophenyl)-1-oxo-2,8-diazaspiro[4.5]decan-8-yl)-3-methyl-1-oxobutan-2-yl)-2-fluoro-5-(trifluoromethyl)benzamide;
2-fluoro-5-methoxy-N-((2R)-3-methyl-1-(2-methyl-1-oxo-4-phenyl-2,8-diazaspiro[4.5]decan-8-yl)-1-oxobutan-2-yl)benzamid;
(R)-2-fluoro-N-(3-methyl-1-(2-methyl-1-oxo-4-phenyl-2,8-diazaspiro[4.5]dec-3-en-8-yl)-1-oxobutan-2-yl)-5-(trifluoromethyl)benzamid;
2-fluoro-N-((2R)-3-methyl-1-(2-methyl-1-oxo-4-phenyl-2,8-diazaspiro[4.5]decan-8-yl)-1-oxobutan-2-yl)benzamid;
N-((2R)-3-methyl-1-(2-methyl-1-oxo-4-phenyl-2,8-diazaspiro[4.5]decan-8-yl)-1-oxobutan-2-yl)-3-(trifluoromethyl)benzamid;
2-fluoro-5-methyl-N-((2R)-3-methyl-1-(2-methyl-1-oxo-4-phenyl-2,8-diazaspiro[4.5]decan-8-yl)-1-oxobutan-2-yl)benzamid;
2-fluoro-N-((2R)-3-methyl-1-(2-methyl-1-oxo-4-phenyl-2,8-diazaspiro[4.5]decan-8-yl)-1-oxobutan-2-yl)-5-(trifluoromethoxy)benzamid;
2-fluoro-N-((2R)-1-(4-(4-fluorophenyl)-2-isopropyl-1-oxo-2,8-diazaspiro[4.5]decan-8-yl)-3-methyl-1-oxobutan-2-yl)-5-(trifluoromethyl)benzamid;
N-((2R)-1-(2-(cyclopropylmethyl)-4-(4-fluorophenyl)-1-oxo-2,8-diazaspiro[4.5]decan-8-yl)-3-methyl-1-oxobutan-2-yl)-2-fluoro-5-(trifluoromethyl)benzamid;
N-((2R)-1-(2-(cyclopropylmethyl)-1-oxo-4-(pyridin-3-yl)-2,8-diazaspiro[4.5]decan-8-yl)-3-methyl-1-oxobutan-2-yl)-2-fluoro-5-(trifluoromethyl)benzamid;
2-fluoro-N-((2R)-1-(4-(4-methoxyphenyl)-2-methyl-1-oxo-2,8-diazaspiro[4.5]decan-8-yl)-3-methyl-1-oxobutan-2-yl)-5-(trifluoromethyl)benzamid;
2-fluoro-N-((2R)-3-methyl-1-oxo-1-(2-oxo-4-phenyl-1-oxa-3,8-diazaspiro[4.5]decan-8-yl)butan-2-yl)-5-(trifluoromethyl)benzamid;
(R)-2-fluoro-N-(1-(1-(4-fluorophenyl)-3-methyl-4-oxo-2,3,8-triazaspiro[4.5]dec-1-en-8-yl)-3-methyl-1-oxobutan-2-yl)-5-(trifluoromethyl)benzamid;
2-fluoro-N-((2R)-1-(4-(4-fluorophenyl)-2-methyl-2,8-diazaspiro[4.5]decan-8-yl)-3-methyl-1-oxobutan-2-yl)-5-(trifluoromethyl)benzamid;
2-fluoro-N-((2R)-1-(4-(4-fluorophenyl)-2-isopropyl-2,8-diazaspiro[4.5]decan-8-yl)-3-methyl-1-oxobutan-2-yl)-5-(trifluoromethyl)benzamid;
N-((2R)-1-(2-(cyclopropylmethyl)-4-(4-fluorophenyl)-2,8-diazaspiro[4.5]decan-8-yl)-3-methyl-1-oxobutan-2-yl)-2-fluoro-5-(trifluoromethyl)benzamid;
2-fluoro-N-((2R)-1-(4-(4-methoxyphenyl)-2-methyl-2,8-diazaspiro[4.5]decan-8-yl)-3-methyl-1-oxobutan-2-yl)-5-(trifluoromethyl)benzamid;
2-fluoro-N-((2R)-1-(4-(4-methoxyphenyl)-2-methyl-2,8-diazaspiro[4.5]decan-8-yl)-3-methyl-1-oxobutan-2-yl)-5-methylbenzamid;
N-((2R)-1-(4-(4-fluorophenyl)-2-methyl-2,8-diazaspiro-[4.5]decan-8-yl)-3-methyl-1-oxobutan-2-yl)-4-methylpicolinamid;
Methyl 8-((2-fluoro-5-(trifluoromethyl)benzoyl)-D-valyl)-4-(4-fluorophenyl)-2,8-diazaspiro[4.5]decane-2-carboxylat;
Ethyl 8-((2-fluoro-5-(trifluoromethyl)benzoyl)-D-valyl)-4-(4-fluorophenyl)-2,8-diazaspiro[4.5]decane-2-carboxylat;
4-(8-((2-fluoro-5-(trifluoromethyl)benzoyl)-D-valyl)-2-methyl-2,8-diazaspiro[4.5]decan-4-yl)benzoesäure;
2-fluoro-N-((R)-1-((R)-4-(4-fluorophenyl)-2-methyl-2,8-diazaspiro[4.5]decan-8-yl)-3-methyl-1-oxobutan-2-yl)-5-(trifluoromethyl)benzamid;
2-fluoro-N-((R)-1-((S)-4-(4-fluorophenyl)-2-methyl-2,8-diazaspiro[4.5]decan-8-yl)-3-methyl-1-oxobutan-2-yl)-5-(trifluoromethyl)benzamid;
2-fluoro-N-((2R)-3-methyl-1-(2-methyl-4-(p-tolyl)-2,8-diazaspiro[4.5]decan-8-yl)-1-oxobutan-2-yl)-5-(trifluoromethyl)benzamid;
N-((2R)-1-(4-(3,5-dimethylphenyl)-2-methyl-2,8-diazaspiro[4.5]decan-8-yl)-3-methyl-1-oxobutan-2-yl)-2-fluoro-5-(trifluoromethyl)benzamid;
2-fluoro-N-((1R)-2-(4-(4-fluorophenyl)-2-methyl-2,8-diazaspiro[4.5]decan-8-yl)-2-oxo-1-phenylethyl)-5-(trifluoromethyl)benzamid;
N-((1R)-1-cyclohexyl-2-(4-(4-fluorophenyl)-2-methyl-2,8-diazaspiro[4.5]decan-8-yl)-2-oxoethyl)-2-fluoro-5-(trifluoromethyl)benzamid
2-fluoro-N-((2R)-1-(4-(4-fluorophenyl)-2-methyl-2,8-diazaspiro[4.5]decan-8-yl)-4-methyl-1-oxopentan-2-yl)-5-(trifluoromethyl)benzamid;
2.5-difluoro-N-((2R)-1-(4-(4-fluorophenyl)-2-methyl-2,8-diazaspiro[4.5]decan-8-yl)-3-methyl-1-oxobutan-2-yl)benzamid;
2.5-dichloro-N-((2R)-1-(4-(4-fluorophenyl)-2-methyl-2,8-diazaspiro[4.5]decan-8-yl)-3-methyl-1-oxobutan-2-yl)benzamid;
N-((2R)-1-(4-(4-fluorophenyl)-2-methyl-2,8-diazaspiro[4.5]decan-8-yl)-3-methyl-1-oxobutan-2-yl)-1-methyl-1H-indazole-3-carboxamid;
N-((2R)-1-(4-(4-fluorophenyl)-2-methyl-2,8-diazaspiro[4.5]decan-8-yl)-3-methyl-1-oxobutan-2-yl)-4,6-dimethylpicolinamid;
4-methyl-N-((2R)-3-methyl-1-(2-methyl-4-(pyridin-3-yl)-2,8-diazaspiro[4.5]decan-8-yl)-1-oxobutan-2-yl)picolinamid;
2-fluoro-N-((2R)-3-methyl-1-oxo-1-(10-oxo-7-phenyl-3,9-diazaspiro[5.5]undecan-3-yl)butan-2-yl)-5-(trifluoromethyl)benzamid;
2-fluoro-N-((2R)-1-(7-(4-fluorophenyl)-10-oxo-3,9-diazaspiro[5.5]undecan-3-yl)-3-methyl-1-oxobutan-2-yl)-5-(trifluoromethyl)benzamid;
2-fluoro-N-((2R)-3-methyl-1-(9-methyl-10-oxo-7-phenyl-3,9-diazaspiro[5.5]undecan-3-yl)-1-oxobutan-2-yl)-5-(trifluoromethyl)benzamid;
2-fluoro-N-((2R)-1-(7-(4-fluorophenyl)-9-methyl-10-oxo-3,9-diazaspiro[5.5]undecan-3-yl)-3-methyl-1-oxobutan-2-yl)-5-(trifluoromethyl)benzamid;
N-((2R)-1-(9-ethyl-7-(4-fluorophenyl)-10-oxo-3,9-diazaspiro[5.5]undecan-3-yl)-3-methyl-1-oxobutan-2-yl)-2-fluoro-5-(trifluoromethyl)benzamid;
2-fluoro-N-((2S)-3-methyl-1-(9-methyl-10-oxo-7-phenyl-3,9-diazaspiro[5.5]undecan-3-yl)-1-oxobutan-2-yl)-5-(trifluoromethyl)benzamid;
N-((2R)-3-methyl-1-(9-methyl-10-oxo-7-phenyl-3,9-diazaspiro[5.5]undecan-3-yl)-1-oxobutan-2-yl)-4-(trifluoromethyl)picolinamid;
2-fluoro-N-((2R)-3-methyl-1-(9-methyl-7-phenyl-3,9-diazaspiro[5.5]undecan-3-yl)-1-oxobutan-2-yl)-5-(trifluoromethyl)benzamid;
2-fluoro-N-((2R)-1-(7-(4-fluorophenyl)-9-methyl-3,9-diazaspiro[5.5]undecan-3-yl)-3-methyl-1-oxobutan-2-yl)-5-(trifluoromethyl)benzamid.

## Revendications

1. Composé choisi parmi :
2-fluoro-N-((2R)-3-méthyl-1-(2-méthyl-1,3-dioxo-4-phényl-2,8-diazaspiro[4.5]décan-8-yl)-1-oxobutan-2-yl)-5-(trifluorométhyl)benzamide ;
N-((2R)-1-(2-(2-aminoéthyl)-1,3-dioxo-4-phényl-2,8-diazaspiro[4.5]décan-8-yl)-3-méthyl-1-oxobutan-2-yl)-2-fluoro-5-(trifluorométhyl)benzamide ;
2-fluoro-N-((2R)-3-méthyl-1-(2-méthyl-1-oxo-4-phényl-2,8-diazaspiro[4.5]décan-8-yl)-1-oxobutan-2-yl)-5-(trifluorométhyl)benzamide ;
2-fluoro-N-((2R)-3-méthyl-1-oxo-1-(1-oxo-4-phényl-2,8-diazaspiro[4.5]décan-8-yl)butan-2-yl)-5-(trifluorométhyl)benzamide ;
2-fluoro-N-((2R)-3-méthyl-1-(2-méthyl-1-oxo-4-(pyridin-3-yl)-2,8-diazaspiro[4.5]décan-8-yl)-1-oxobutan-2-yl)-5-(trifluorométhyl)benzamide ;
2-fluoro-N-((2R)-3-méthyl-1-oxo-1-(1-oxo-4-(pyridin-3-yl)-2,8-diazaspiro[4.5]décan-8-yl)butan-2-yl)-5-(trifluorométhyl)benzamide ;
N-((2R)-1-(4-(1 H-indazol-5-yl)-2-méthyl-1-oxo-2,8-diazaspiro[4.5]décan-8-yl)-3-méthyl-1-oxobutan-2-yl)-2-fluoro-5-(trifluorométhyl)benzamide ;
2-fluoro-N-((2R)-3-méthyl-1-(2-méthyl-4-phényl-2,8-diazaspiro[4.5]décan-8-yl)-1-oxobutan-2-yl)-5-(trifluorométhyl)benzamide ;
2-fluoro-N-((2R)-3-méthyl-1-(2-méthyl-4-(pyridin-3-yl)-2,8-diazaspiro[4.5]décan-8-yl)-1-oxobutan-2-yl)-5-(trifluorométhyl)benzamide ;
N-((2R)-1-(4-(1H-indazol-5-yl)-2-méthyl-2,8-diazaspiro[4.5]décan-8-yl)-3-méthyl-1-oxobutan-2-yl)-2-fluoro-5-(trifluorométhyl)benzamide ;
2-fluoro-N-((2R)-1-(4-(4-fluorophényl)-1-oxo-2,8-diazaspiro[4.5]décan-8-yl)-3-méthyl-1-oxobutan-2-yl)-5-(trifluorométhyl)benzamide ;
2-fluoro-N-((2R)-1-(4-(4-fluorophényl)-2-méthyl-1-oxo-2,8-diazaspiro[4.5]décan-8-yl)-3-méthyl-1-oxobutan-2-yl)-5-(trifluorométhyl)benzamide ;
N-((2R)-1-(2-éthyl-4-(4-fluorophényl)-1-oxo-2,8-diazaspiro[4.5]décan-8-yl)-3-méthyl-1-oxobutan-2-yl)-2-fluoro-5-(trifluorométhyl)benzamide ;
2-fluoro-5-méthoxy-N-((2R)-3-méthyl-1-(2-méthyl-1-oxo-4-phényl-2,8-diazaspiro[4.5]décan-8-yl)-1-oxobutan-2-yl)benzamide ;
(R)-2-fluoro-N-(3-méthyl-1-(2-méthyl-1-oxo-4-phényl-2,8-diazaspiro[4.5]dec-3-en-8-yl)-1-oxobutan-2-yl)-5-(trifluorométhyl)benzamide ;
2-fluoro-N-((2R)-3-méthyl-1-(2-méthyl-1-oxo-4-phényl-2,8-diazaspiro[4.5]décan-8-yl)-1-oxobutan-2-yl)benzamide ;
N-((2R)-3-méthyl-1-(2-méthyl-1-oxo-4-phényl-2,8-diazaspiro[4.5]décan-8-yl)-1-oxobutan-2-yl)-3-(trifluorométhyl)benzamide ;
2-fluoro-5-méthyl-N-((2R)-3-méthyl-1-(2-méthyl-1-oxo-4-phényl-2,8-diazaspiro[4.5]décan-8-yl)-1-oxobutan-2-yl)benzamide ;
2-fluoro-N-((2R)-3-méthyl-1-(2-méthyl-1-oxo-4-phényl-2,8-diazaspiro[4.5]décan-8-yl)-1-oxobutan-2-yl)-5-(trifluorométhoxy)benzamide ;
2-fluoro-N-((2R)-1-(4-(4-fluorophényl)-2-isopropyl-1-oxo-2,8-diazaspiro[4.5]décan-8-yl)-3-méthyl-1-oxobutan-2-yl)-5-(trifluorométhyl)benzamide ;
N-((2R)-1-(2-(cyclopropylméthyl)-4-(4-fluorophényl)-1-oxo-2,8-diazaspiro[4.5]décan-8-yl)-3-méthyl-1-oxobutan-2-yl)-2-fluoro-5-(trifluorométhyl)benzamide ;
N-((2R)-1-(2-(cyclopropylméthyl)-1-oxo-4-(pyridin-3-yl)-2,8-diazaspiro[4.5]décan-8-yl)-3-méthyl-1-oxobutan-2-yl)-2-fluoro-5-(trifluorométhyl)benzamide ;
2-fluoro-N-((2R)-1-(4-(4-méthoxyphényl)-2-méthyl-1-oxo-2,8-diazaspiro[4.5]décan-8-yl)-3-méthyl-1-oxobutan-2-yl)-5-(trifluorométhyl)benzamide ;
2-fluoro-N-((2R)-3-méthyl-1-oxo-1-(2-oxo-4-phényl-1-oxa-3,8-diazaspiro[4.5]décan-8-yl)butan-2-yl)-5-(trifluorométhyl)benzamide ;
(R)-2-fluoro-N-(1-(1-(4-fluorophényl)-3-méthyl-4-oxo-2,3,8-triazaspiro[4.5]dec-1-en- 8-yl)-
3-méthyl-1-oxobutan-2-yl)-5-(trifluorométhyl)benzamide ; 2-fluoro-N-((2R)-1-(4-(4-fluorophényl)-2-méthyl-2,8-diazaspiro[4.5]décan-8-yl)-3-méthyl-1-oxobutan-2-yl)-5-(trifluorométhyl)benzamide ;
2-fluoro-N-((2R)-1-(4-(4-fluorophényl)-2-isopropyl-2,8-diazaspiro[4.5]décan-8-yl)-3-méthyl-1-oxobutan-2-yl)-5-(trifluorométhyl)benzamide ;
N-((2R)-1-(2-(cyclopropylméthyl)-4-(4-fluorophényl)-2,8-diazaspiro[4.5]décan-8-yl)-3-méthyl-1-oxobutan-2-yl)-2-fluoro-5-(trifluorométhyl)benzamide ;
2-fluoro-N-((2R)-1-(4-(4-méthoxyphényl)-2-méthyl-2,8-diazaspiro[4.5]décan-8-yl)-3-méthyl-1-oxobutan-2-yl)-5-(trifluorométhyl)benzamide ;
2-fluoro-N-((2R)-1-(4-(4-méthoxyphényl)-2-méthyl-2,8-diazaspiro[4.5]décan-8-yl)-3-méthyl-1-oxobutan-2-yl)-5-méthylbenzamide ;
N-((2R)-1-(4-(4-fluorophényl)-2-méthyl-2,8-diazaspiro-[4.5]décan-8-yl)-3-méthyl-1-oxobutan-2-yl)-4-méthylpicolinamide ;
méthyl 8-((2-fluoro-5-(trifluorométhyl)benzoyl)-D-valyl)-4-(4-fluorophényl)-2,8-diazaspiro[4.5]décane-2-carboxylate ;
éthyl 8-((2-fluoro-5-(trifluorométhyl)benzoyl)-D-valyl)-4-(4-fluorophényl)-2,8-diazaspiro[4.5]décane-2-carboxylate ;
acide 4-(8-((2-fluoro-5-(trifluorométhyl)benzoyl)-D-valyl)-2-méthyl-2,8-diazaspiro[4.5]décan-4-yl)benzoïque ;
2-fluoro-N-((R)-1-((R)-4-(4-fluorophényl)-2-méthyl-2,8-diazaspiro[4.5]décan-8-yl)-3-méthyl-1-oxobutan-2-yl)-5-(trifluorométhyl)benzamide ;
2-fluoro-N-((R)-1-((S)-4-(4-fluorophényl)-2-méthyl-2,8-diazaspiro[4.5]décan-8-yl)-3-méthyl-1-oxobutan-2-yl)-5-(trifluorométhyl)benzamide ;
2-fluoro-N-((2R)-3-méthyl-1-(2-méthyl-4-(p-tolyl)-2,8-diazaspiro[4.5]décan-8-yl)-1-oxobutan-2-yl)-5-(trifluorométhyl)benzamide ;
N-((2R)-1-(4-(3,5-diméthylphényl)-2-méthyl-2,8-diazaspiro[4.5]décan-8-yl)-3-méthyl-1-oxobutan-2-yl)-2-fluoro-5-(trifluorométhyl)benzamide ;
2-fluoro-N-((1R)-2-(4-(4-fluorophényl)-2-méthyl-2,8-diazaspiro[4.5]décan-8-yl)-2-oxo-1-phényléthyl)-5-(trifluorométhyl)benzamide ;
N-((1R)-1-cyclohexyl-2-(4-(4-fluorophényl)-2-méthyl-2,8-diazaspiro[4.5]décan-8-yl)-2-oxoéthyl)-2-fluoro-5-(trifluorométhyl)benzamide ;
2-fluoro-N-((2R)-1-(4-(4-fluorophényl)-2-méthyl-2,8-diazaspiro[4.5]décan-8-yl)-4-méthyl-1-oxopentan-2-yl)-5-(trifluorométhyl)benzamide ;
2,5-difluoro-N-((2R)-1-(4-(4-fluorophényl)-2-méthyl-2,8-diazaspiro[4.5]décan-8-yl)-3-méthyl-1-oxobutan-2-yl)benzamide ;
2,5-dichloro-N-((2R)-1-(4-(4-fluorophényl)-2-méthyl-2,8-diazaspiro[4.5]décan-8-yl)-3-méthyl-1-oxobutan-2-yl)benzamide ;
N-((2R)-1-(4-(4-fluorophényl)-2-méthyl-2,8-diazaspiro[4.5]décan-8-yl)-3-méthyl-1-oxobutan-2-yl)-1-méthyl-1H-indazole-3-carboxamide ;
N-((2R)-1-(4-(4-fluorophényl)-2-méthyl-2,8-diazaspiro[4.5]décan-8-yl)-3-méthyl-1-oxobutan-2-yl)-4,6-diméthylpicolinamide ;
4-méthyl-N-((2R)-3-méthyl-1-(2-méthyl-4-(pyridin-3-yl)-2,8-diazaspiro[4.5]décan-8-yl)-1-oxobutan-2-yl)picolinamide ;
2-fluoro-N-((2R)-3-méthyl-1-oxo-1-(10-oxo-7-phényl-3,9-diazaspiro[5.5]undécan-3-yl)butan-2-yl)-5-(trifluorométhyl)benzamide ;
2-fluoro-N-((2R)-1-(7-(4-fluorophényl)-10-oxo-3,9-diazaspiro[5.5]undécan-3-yl)-3-méthyl-1-oxobutan-2-yl)-5-(trifluorométhyl)benzamide ;
2-fluoro-N-((2R)-3-méthyl-1-(9-méthyl-10-oxo-7-phényl-3,9-diazaspiro[5.5]undécan-3-yl)-1-oxobutan-2-yl)-5-(trifluorométhyl)benzamide ;
2-fluoro-N-((2R)-1-(7-(4-fluorophényl)-9-méthyl-10-oxo-3,9-diazaspiro[5.5]undécan-3-yl)-3-méthyl-1-oxobutan-2-yl)-5-(trifluorométhyl)benzamide ;
N-((2R)-1-(9-éthyl-7-(4-fluorophényl)-10-oxo-3,9-diazaspiro[5.5]undécan-3-yl)-3-méthyl-1-oxobutan-2-yl)-2-fluoro-5-(trifluorométhyl)benzamide ;
2-fluoro-N-((2S)-3-méthyl-1-(9-méthyl-10-oxo-7-phényl-3,9-diazaspiro[5.5]undécan-3-yl)-1-oxobutan-2-yl)-5-(trifluorométhyl)benzamide ;
N-((2R)-3-méthyl-1-(9-méthyl-10-oxo-7-phényl-3,9-diazaspiro[5.5]undécan-3-yl)-1-oxobutan-2-yl)-4-(trifluorométhyl)picolinamide ;
2-fluoro-N-((2R)-3-méthyl-1-(9-méthyl-7-phényl-3,9-diazaspiro[5.5]undécan-3-yl)-1-oxobutan-2-yl)-5-(trifluorométhyl)benzamide ;
2-fluoro-N-((2R)-1-(7-(4-fluorophényl)-9-méthyl-3,9-diazaspiro[5.5]undécan-3-yl)-3-méthyl-1-oxobutan-2-yl)-5-(trifluorométhyl)benzamide.
